# EUROPEAN PATENT SPECIFICATION

(11) **EP 3 397 639 B1**
(45) Date of publication and mention of the grant of the patent: **23.02.2022**
(21) Application number: 16826561.9
(22) Date of filing: 22.12.2016
(51) Int. Cl.: C07D 471/16, A61K 31/5025, A61P 31/18

(54) **FUSED TRICYCLIC HETEROCYCLIC COMPOUNDS AS HIV INTEGRASE INHIBITORS**
KONDENSIERTE TRICYCLISCHE HETEROCYCLISCHE VERBINDUNGEN ALS HIV-INTEGRASE-HEMMER
COMPOSÉS HÉTÉROCYCLIQUES TRICYCLIQUES CONDENSÉS À TITRE D'INHIBITEURS DE L'INTÉGRASE DU VIH

(30) Priority: 31.12.2015 WO PCT/CN2015/100106
(43) Date of publication of application: 07.11.2018
(73) Proprietor: Merck Sharp & Dohme Corp., Rahway, NJ 07065-0907 (US)
(72) Inventor: YU, Tao, Kenilworth New Jersey 07033 (US); WADDELL, Sherman, T., Westfield New Jersey 07090 (US); GRAHAM, Thomas, H., Boston Massachusetts 02115-5727 (US); ZHANG, Yonglian, Kenilworth New Jersey 07033 (US); MCCAULEY, John, A., West Point Pennsylvania 19486 (US); STAMFORD, Andrew, W., Chatham Township New Jersey 07928 (US); CAI, Jiaqiang, Shanghai 200131 (CN); QI, Zhiqi, Shanghai 200131 (CN)
(74) Representative: Jaap, David Robert
(86) International application number: PCT/US2016/068173
(87) International publication number: WO 2017/116928

(56) References cited:
- WO-A1-2014/183532

## Description

### FIELD OF THE INVENTION

The present invention relates to Fused Tricyclic Heterocyclic Compounds, compositions comprising at least one Fused Tricyclic Heterocyclic Compound, and the Fused Tricyclic Heterocyclic Compounds for use in treating or preventing HIV infection in a subject.

### BACKGROUND OF THE INVENTION

A retrovirus designated human immunodeficiency virus (HIV), particularly the strains known as HIV type-1 (HIV-1) virus and type-2 (HIV-2) virus, is the etiological agent of the complex disease that includes progressive destruction of the immune system (acquired immune deficiency syndrome; AIDS) and degeneration of the central and peripheral nervous system. A common feature of retrovirus replication is the insertion by virally-encoded integrase of +proviral DNA into the host cell genome, a required step in HIV replication in human T-lymphoid and monocytoid cells. Integration is believed to be mediated by integrase in three steps: assembly of a stable nucleoprotein complex with viral DNA sequences; cleavage of two nucleotides from the 3' termini of the linear proviral DNA; covalent joining of the recessed 3' OH termini of the proviral DNA at a staggered cut made at the host target site. The fourth step in the process, repair synthesis of the resultant gap, may be accomplished by cellular enzymes.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, integrase and an HIV protease [Toh, H. et al., EMBO J. 4, 1267 (1985); Power, M.D. et al., Science, 231, 1567 (1986); Pearl, L.H. et al., Nature, 329, 351 (1987)]. All three enzymes have been shown to be essential for the replication of HIV.

It is known that some antiviral compounds which act as inhibitors of HIV replication are effective agents in the treatment of AIDS and similar diseases, including reverse transcriptase inhibitors such as azidothymidine (AZT) and efavirenz and protease inhibitors such as indinavir and nelfinavir. The compounds of this invention are inhibitors of HIV integrase and inhibitors of HIV replication.

The following references are of interest as background:
International Publication Nos. WO11/045330 and WO11/121105 disclose macrocyclic compounds having HIV integrase inhibitory activity.
Kinzel et al., Tet. Letters 2007, 48(37): pp. 6552-6555 discloses the synthesis of tetrahydropyridopyrimidones as a scaffold for HIV-1 integrase inhibitors.
Ferrara et al., Tet. Letters 2007, 48(37), pp. 8379-8382 discloses the synthesis of a hexahydropyrimido[1,2-a]azepine-2-carboxamide derivative useful as an HIV integrase inhibitor.
Muraglia et al., J.Med. Chem. 2008, 51: 861-874 discloses the design and synthesis of bicyclic pyrimidinones as potent and orally bioavailable HIV-1 integrase inhibitors.
US2004/229909 discloses certain compounds having integrase inhibitory activity.
US7232819 and US2007/0083045 disclose certain 5,6-dihydroxypyrimidine-4-carboxamides as HIV integrase inhibitors.
US7169780, US7217713, and US 2007/0123524 disclose certain N-substituted 5-hydroxy-6-oxo-1,6-dihydropyrimidine-4-carboxamides as HIV integrase inhibitors.
US7279487 discloses certain hydroxynaphthyridinone carboxamides that are useful as HIV integrase inhibitors.
US7135467 and US7037908 disclose certain pyrimidine carboxamides that are useful as HIV integrase inhibitors.
US7211572 discloses certain nitrogenous condensed ring compounds that are HIV integrase inhibitors.
US7414045 discloses certain tetrahydro-4H-pyrido[1,2-a]pyrimidine carboxamides, hexahydropyrimido[1,2-a]azepine carboxamides, and related compounds that are useful as HIV integrase inhibitors.
WO2006/103399 discloses certain tetrahydro-4H-pyrimidooxazepine carboaxmides, tetrahydropyrazinopyrimidine carboxamides, hexahydropyrimidodiazepine carboxamides, and related compounds that are useful as HIV integrase inhibitors.
US2007/0142635 discloses processes for preparing hexahydropyrimido[1,2-a]azepine-2-carboxylates and related compounds.
US2007/0149556 discloses certain hydroxypyrimidinone derivatives having HIV integrase inhibitory activity.
Various pyrimidinone compounds useful as HIV integrase inhibitors are also disclosed in US7115601, US7157447, US7173022, US7176196, US7192948, US7273859, and US7419969.
US2007/0111984 discloses a series of bicyclic pyrimidinone compounds useful as HIV integrase inhibitors.
US2006/0276466, US2007/0049606, US2007/0111985, US2007/0112190, US2007/0281917, US2008/0004265 each disclose a series of bicyclic pyrimidinone compounds useful as HIV integrase inhibitors.

### SUMMARY OF THE INVENTION

In one aspect, the present invention provides Compounds of Formula (I): 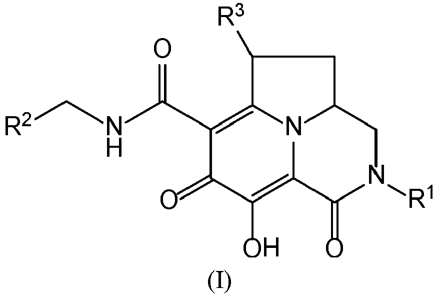 and pharmaceutically acceptable salts thereof, wherein:
R¹ is C₁-C₆ alkyl or -(C₁-C₆ alkenyl)-O-(C₁-C₆ alkyl);
R² is phenyl, which is optionally substituted with up to 3 ring substituent groups, each independently selected from halo;
R³ is -N(R⁴)₂;
each occurrence of R⁴ is independently selected from H, C₁-C₆ alkyl, C₁-C₆ haloalkyl, C₃-C₇ cycloalkyl, -CH₂-(C₃-C₇ cycloalkyl), phenyl, benzyl, -C(O)-C(O)-N(R⁵)₂, -S(O)₂-C₁-C₆ alkyl, -S(O)₂-phenyl, -(C₁-C₆ alkenyl)-O-(C₁-C₆ alkyl) and -C(O)-C₁-C₆ alkyl, where the phenyl moiety of a -S(O)₂-phenyl group can be optionally substituted with a C₁-C₆ alkyl group; or both R⁴ groups, and the common nitrogen atom to which they are attached, join to form an azetidinyl, piperidinyl, or pyrrolidinyl group; and
each occurrence of R⁵ is independently selected from H and C₁-C₆ alkyl.

The Compounds of Formula (I) (also referred to herein as the "Fused Tricyclic Heterocyclic Compounds") and pharmaceutically acceptable salts thereof may be useful, for example, for inhibiting HIV viral replication or replicon activity, or for treating or preventing HIV infection in a subject. Without being bound by any specific theory, it is believed that the Fused Tricyclic Heterocyclic Compounds inhibit HIV viral replication by inhibiting HIV Integrase.

Accordingly, the present invention provides compounds for use in methods for treating or preventing HIV infection in a subject, comprising administering to the subject an effective amount of at least one Fused Tricyclic Heterocyclic Compound.

The details of the invention are set forth in the accompanying detailed description below.

Although any methods and materials similar to those described herein may be used in the practice or testing of the present invention, illustrative methods and materials are now described. Other embodiments, aspects and features of the present invention are either further described in or will be apparent from the ensuing description, examples and appended claims.

### DETAILED DESCRIPTION OF THE INVENTION

The present invention relates to Fused Tricyclic Heterocyclic Compounds, compositions comprising at least one Fused Tricyclic Heterocyclic Compound, and compounds for use in methods of using the Fused Tricyclic Heterocyclic Compounds for inhibiting HIV integrase, inhibiting HIV viral replication or for treating or preventing HIV infection in a subject.

### Definitions and Abbreviations

The terms used herein have their ordinary meaning and the meaning of such terms is independent at each occurrence thereof. That notwithstanding and except where stated otherwise, the following definitions apply throughout the specification and claims. Chemical names, common names, and chemical structures may be used interchangeably to describe the same structure. These definitions apply regardless of whether a term is used by itself or in combination with other terms, unless otherwise indicated. Hence, the definition of "alkyl" applies to "alkyl" as well as the "alkyl" portions of "hydroxyalkyl," "haloalkyl," "-O-alkyl," etc...

As used herein, and throughout this disclosure, the following terms, unless otherwise indicated, shall be understood to have the following meanings:
A "subject" is a human or non-human mammal. In one embodiment, a subject is a human. In another embodiment, a subject is a primate. In another embodiment, a subject is a monkey. In another embodiment, a subject is a chimpanzee. In still another embodiment, a subject is a rhesus monkey.

The term "effective amount" as used herein, refers to an amount of Fused Tricyclic Heterocyclic Compound and/or an additional therapeutic agent, or a composition thereof that is effective in inhibiting HIV replication and in producing the desired therapeutic, ameliorative, inhibitory or preventative effect when administered to a subject suffering from HIV infection or AIDS. In the combination therapies of the present invention, an effective amount can refer to each individual agent or to the combination as a whole, wherein the amounts of all agents administered are together effective, but wherein the component agent of the combination may not be present individually in an effective amount.

The term "preventing," as used herein with respect to an HIV viral infection or AIDS, refers to reducing the likelihood or severity of HIV infection or AIDS.

The term "alkyl," as used herein, refers to an aliphatic hydrocarbon group having one of its hydrogen atoms replaced with a bond. An alkyl group may be straight or branched and contain from about 1 to about 20 carbon atoms. In one embodiment, an alkyl group contains from about 1 to about 12 carbon atoms. In different embodiments, an alkyl group contains from 1 to 6 carbon atoms (C₁-C₆ alkyl) or from about 1 to about 4 carbon atoms (C₁-C₄ alkyl). Non-limiting examples of alkyl groups include methyl, ethyl, n-propyl, isopropyl, n-butyl, sec-butyl, isobutyl, tert-butyl, n-pentyl, neopentyl, isopentyl, n-hexyl, isohexyl and neohexyl. An alkyl group may be unsubstituted or substituted by one or more substituents which may be the same or different, each substituent being independently selected from the group consisting of halo, alkenyl, alkynyl, aryl, cycloalkyl, cyano, hydroxy, -O-alkyl, -O-aryl, -alkylene-O-alkyl, alkylthio, -NH₂, -NH(alkyl), -N(alkyl)₂, -NH(cycloalkyl), -O-C(O)-alkyl, -O-C(O)-aryl, -O-C(O)-cycloalkyl, -C(O)OH and -C(O)O-alkyl. In one embodiment, an alkyl group is linear. In another embodiment, an alkyl group is branched. Unless otherwise indicated, an alkyl group is unsubstituted.

The term "alkenyl," as used herein, refers to an aliphatic hydrocarbon group containing at least one carbon-carbon double bond and having one of its hydrogen atoms replaced with a bond. An alkenyl group may be straight or branched and contain from about 2 to about 15 carbon atoms. In one embodiment, an alkenyl group contains from about 2 to about 12 carbon atoms. In another embodiment, an alkenyl group contains from about 2 to about 6 carbon atoms. Non-limiting examples of alkenyl groups include ethenyl, propenyl, n-butenyl, 3-methylbut-2-enyl, n-pentenyl, octenyl and decenyl. An alkenyl group may be unsubstituted or substituted by one or more substituents which may be the same or different, each substituent being independently selected from the group consisting of halo, alkenyl, alkynyl, aryl, cycloalkyl, cyano, hydroxy, -O-alkyl, -O-aryl, -alkylene-O-alkyl, alkylthio, -NH₂, -NH(alkyl), -N(alkyl)₂, -NH(cycloalkyl), -O-C(O)-alkyl, -O-C(O)-aryl, -O-C(O)-cycloalkyl, -C(O)OH and -C(O)O-alkyl. The term "C₂-C₆ alkenyl" refers to an alkenyl group having from 2 to 6 carbon atoms. Unless otherwise indicated, an alkenyl group is unsubstituted.

The term "alkynyl," as used herein, refers to an aliphatic hydrocarbon group containing at least one carbon-carbon triple bond and having one of its hydrogen atoms replaced with a bond. An alkynyl group may be straight or branched and contain from about 2 to about 15 carbon atoms. In one embodiment, an alkynyl group contains from about 2 to about 12 carbon atoms. In another embodiment, an alkynyl group contains from about 2 to about 6 carbon atoms. Non-limiting examples of alkynyl groups include ethynyl, propynyl, 2-butynyl and 3-methylbutynyl. An alkynyl group may be unsubstituted or substituted by one or more substituents which may be the same or different, each substituent being independently selected from the group consisting of halo, alkenyl, alkynyl, aryl, cycloalkyl, cyano, hydroxy, -O-alkyl, -O-aryl, -alkylene-O-alkyl, alkylthio, -NH₂, -NH(alkyl), -N(alkyl)₂, -NH(cycloalkyl), -O-C(O)-alkyl, -O-C(O)-aryl, -O-C(O)-cycloalkyl, -C(O)OH and -C(O)O-alkyl. The term "C₂-C₆ alkynyl" refers to an alkynyl group having from 2 to 6 carbon atoms. Unless otherwise indicated, an alkynyl group is unsubstituted.

The term "alkylene," as used herein, refers to an alkyl group, as defined above, wherein one of the alkyl group's hydrogen atoms has been replaced with a bond. Non-limiting examples of alkylene groups include -CH₂-, -CH₂CH₂-, -CH₂CH₂CH₂-, -CH₂CH₂CH₂CH₂-, - CH(CH₃)CH₂CH₂-, -CH(CH₃)- and -CH₂CH(CH₃)CH₂-. In one embodiment, an alkylene group has from 1 to about 6 carbon atoms. In another embodiment, an alkylene group has from about 3 to about 5 carbon atoms. In another embodiment, an alkylene group is branched. In another embodiment, an alkylene group is linear. In one embodiment, an alkylene group is -CH₂-. The term "C₁-C₆ alkylene" refers to an alkylene group having from 1 to 6 carbon atoms. The term "C₂-C₄ alkylene" refers to an alkylene group having from 2 to 4 carbon atoms.

The term "alkenylene," as used herein, refers to an alkenyl group, as defined above, wherein one of the alkenyl group's hydrogen atoms has been replaced with a bond. Non-limiting examples of alkenylene groups include -CH=CH-, -CH=CHCH₂-, -CH₂CH=CH-, -CH₂CH=CHCH₂-, -CH=CHCH₂CH₂-, -CH₂CH₂CH=CH- and -CH(CH₃)CH=CH-. In one embodiment, an alkenylene group has from 2 to about 6 carbon atoms. In another embodiment, an alkenylene group has from about 3 to about 5 carbon atoms. In another embodiment, an alkenylene group is branched. In another embodiment, an alkenylene group is linear. The term "C₂-C₆ alkylene" refers to an alkenylene group having from 2 to 6 carbon atoms. The term "C₃-C₅ alkenylene" refers to an alkenylene group having from 3 to 5 carbon atoms.

The term "aryl," as used herein, refers to an aromatic monocyclic or multicyclic ring system comprising from about 6 to about 14 carbon atoms. In one embodiment, an aryl group contains from about 6 to about 10 carbon atoms. An aryl group may be optionally substituted with one or more "ring system substituents" which may be the same or different, and are as defined herein below. In one embodiment, an aryl group may be optionally fused to a cycloalkyl or cycloalkanoyl group. Non-limiting examples of aryl groups include phenyl and naphthyl. In one embodiment, an aryl group is phenyl. Unless otherwise indicated, an aryl group is unsubstituted.

The term "arylene," as used herein, refers to a bivalent group derived from an aryl group, as defined above, by removal of a hydrogen atom from a ring carbon of an aryl group. An arylene group may be derived from a monocyclic or multicyclic ring system comprising from about 6 to about 14 carbon atoms. In one embodiment, an arylene group contains from about 6 to about 10 carbon atoms. In another embodiment, an arylene group is a naphthylene group. In another embodiment, an arylene group is a phenylene group. An arylene group may be optionally substituted with one or more "ring system substituents" which may be the same or different, and are as defined herein below. An arylene group is divalent and either available bond on an arylene group can connect to either group flanking the arylene group. For example, the group "A-arylene-B," wherein the arylene group is: 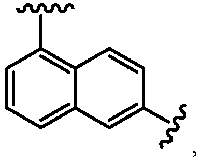 is understood to represent both:

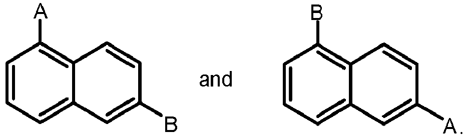

In one embodiment, an arylene group may be optionally fused to a cycloalkyl or cycloalkanoyl group. Non-limiting examples of arylene groups include phenylene and naphthalene. In one embodiment, an arylene group is unsubstituted. In another embodiment, an arylene group is:

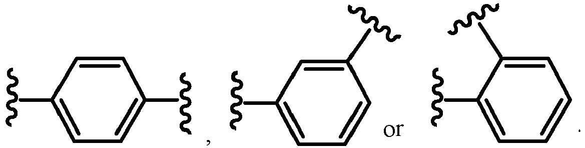

Unless otherwise indicated, an arylene group is unsubstituted.

The term "cycloalkyl," as used herein, refers to a saturated or unsaturated non-aromatic mono- or multicyclic ring system comprising from about 3 to about 10 ring carbon atoms. In one embodiment, a cycloalkyl contains from about 5 to about 10 ring carbon atoms. In another embodiment, a cycloalkyl contains from about 3 to about 7 ring atoms. In another embodiment, a cycloalkyl contains from about 5 to about 6 ring atoms. The term "cycloalkyl" also encompasses a cycloalkyl group, as defined above, which is fused to an aryl (e.g., benzene) or heteroaryl ring. Non-limiting examples of monocyclic cycloalkyls include cyclopropyl, cyclobutyl, cyclopentyl, cyclohexyl, cycloheptyl and cyclooctyl. Non-limiting examples of multicyclic cycloalkyls include 1-decalinyl, norbornyl and adamantyl. A cycloalkyl group may be optionally substituted with one or more "ring system substituents" which may be the same or different, and are as defined herein below. In one embodiment, a cycloalkyl group is unsubstituted. The term "3 to 7-membered cycloalkyl" refers to a cycloalkyl group having from 3 to 7 ring carbon atoms. Unless otherwise indicated, a cycloalkyl group is unsubstituted. A ring carbon atom of a cycloalkyl group may be functionalized as a carbonyl group. An illustrative example of such a cycloalkyl group (also referred to herein as a "cycloalkanoyl" group) includes, but is not limited to, cyclobutanoyl:

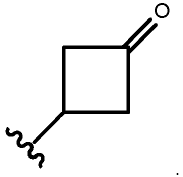

The term "halo," as used herein, means -F, -Cl, -Br or -I.

The term "haloalkyl," as used herein, refers to an alkyl group as defined above, wherein one or more of the alkyl group's hydrogen atoms has been replaced with a halogen. In one embodiment, a haloalkyl group has from 1 to 6 carbon atoms. In another embodiment, a haloalkyl group is substituted with from 1 to 3 F atoms. Non-limiting examples of haloalkyl groups include -CH₂F, -CHF₂, -CF₃, -CH₂Cl and -CCl₃. The term "C₁-C₆ haloalkyl" refers to a haloalkyl group having from 1 to 6 carbon atoms.

The term "hydroxyalkyl," as used herein, refers to an alkyl group as defined above, wherein one or more of the alkyl group's hydrogen atoms have been replaced with an -OH group. In one embodiment, a hydroxyalkyl group has from 1 to 6 carbon atoms. Non-limiting examples of hydroxyalkyl groups include -CH₂OH, -CH₂CH₂OH, -CH₂CH₂CH₂OH and -CH₂CH(OH)CH₃. The term "C₁-C₆ hydroxyalkyl" refers to a hydroxyalkyl group having from 1 to 6 carbon atoms.

The term "heteroaryl," as used herein, refers to an aromatic monocyclic or multicyclic ring system comprising about 5 to about 14 ring atoms, wherein from 1 to 4 of the ring atoms is independently O, N or S and the remaining ring atoms are carbon atoms. In one embodiment, a heteroaryl group has 5 to 10 ring atoms. In another embodiment, a heteroaryl group is monocyclic and has 5 or 6 ring atoms. In another embodiment, a heteroaryl group is bicyclic. In another embodiment, a heteroaryl group is bicyclic and has 9 or 10 ring atoms. A heteroaryl group may be optionally substituted by one or more "ring system substituents" which may be the same or different, and are as defined herein below. A heteroaryl group is joined via a ring carbon atom, and any nitrogen atom of a heteroaryl may be optionally oxidized to the corresponding N-oxide. The term "heteroaryl" also encompasses a heteroaryl group, as defined above, which is fused to a benzene ring. Non-limiting examples of heteroaryls include pyridyl, pyrazinyl, furanyl, thienyl, pyrimidinyl, pyridone (including N-substituted pyridones), isoxazolyl, isothiazolyl, oxazolyl, oxadiazolyl, thiazolyl, pyrazolyl, furazanyl, pyrrolyl, triazolyl, 1,2,4-thiadiazolyl, pyrazinyl, pyridazinyl, quinoxalinyl, phthalazinyl, oxindolyl, imidazo[1,2-a]pyridinyl, imidazo[2,1-b]thiazolyl, benzofurazanyl, indolyl, azaindolyl, benzimidazolyl, benzothienyl, quinolinyl, imidazolyl, benzimidazolyl, thienopyridyl, quinazolinyl, thienopyrimidyl, pyrrolopyridyl, imidazopyridyl, isoquinolinyl, benzoazaindolyl, 1,2,4-triazinyl, benzothiazolyl and the like, and all isomeric forms thereof. The term "heteroaryl" also refers to partially saturated heteroaryl moieties such as, for example, tetrahydroisoquinolyl, tetrahydroquinolyl and the like. In one embodiment, a heteroaryl group is a 5-membered heteroaryl. In another embodiment, a heteroaryl group is a 6-membered monocyclic heteroaryl. In another embodiment, a heteroaryl group comprises a 5- to 6-membered monocyclic heteroaryl group fused to a benzene ring. Unless otherwise indicated, a heteroaryl group is unsubstituted.

The term "heterocycloalkyl," as used herein, refers to a non-aromatic saturated monocyclic or multicyclic ring system comprising 3 to about 11 ring atoms, wherein from 1 to 4 of the ring atoms are independently O, S, N or Si, and the remainder of the ring atoms are carbon atoms. A heterocycloalkyl group may be joined via a ring carbon, ring silicon atom or ring nitrogen atom. In one embodiment, a heterocycloalkyl group is monocyclic and has from about 3 to about 7 ring atoms. In another embodiment, a heterocycloalkyl group is monocyclic has from about 5 to about 8 ring atoms. In another embodiment, a heterocycloalkyl group is bicyclic and has from about 8 to about 11 ring atoms. In still another embodiment, a heterocycloalkyl group is monocyclic and has 5 or 6 ring atoms. In one embodiment, a heterocycloalkyl group is monocyclic. In another embodiment, a heterocycloalkyl group is bicyclic. There are no adjacent oxygen and/or sulfur atoms present in the ring system. Any -NH group in a heterocycloalkyl ring may exist protected such as, for example, as an -N(BOC), -N(Cbz), -N(Tos) group and the like; such protected heterocycloalkyl groups are considered part of this invention. The term "heterocycloalkyl" also encompasses a heterocycloalkyl group, as defined above, which is fused to an aryl (e.g., benzene) or heteroaryl ring. A heterocycloalkyl group may be optionally substituted by one or more "ring system substituents" which may be the same or different, and are as defined herein below. The nitrogen or sulfur atom of the heterocycloalkyl may be optionally oxidized to the corresponding N-oxide, S-oxide or S,S-dioxide. Non-limiting examples of monocyclic heterocycloalkyl rings include oxetanyl, piperidyl, pyrrolidinyl, pyrrolidinyl, morpholinyl, thiomorpholinyl, thiazolidinyl, 1,4-dioxanyl, tetrahydrofuranyl, tetrahydrothiophenyl, delta-lactam, delta-lactone and the like, and all isomers thereof.

A ring carbon atom of a heterocycloalkyl group may be functionalized as a carbonyl group. An illustrative example of such a heterocycloalkyl group is:

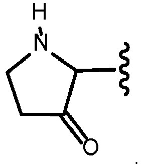

In one embodiment, a heterocycloalkyl group is a 5-membered monocyclic heterocycloalkyl. In another embodiment, a heterocycloalkyl group is a 6-membered monocyclic heterocycloalkyl. The term "4 to 7-membered monocyclic heterocycloalkyl" refers to a monocyclic heterocycloalkyl group having from 4 to 7 ring atoms. The term "5 to 8-membered monocyclic heterocycloalkyl" refers to a monocyclic heterocycloalkyl group having from 5 to 8 ring atoms. The term "8 to 11-membered bicyclic heterocycloalkyl" refers to a bicyclic heterocycloalkyl group having from 8 to 11 ring atoms. Unless otherwise indicated, a heterocycloalkyl group is unsubstituted.

The term "heterocycloalkenyl," as used herein, refers to a heterocycloalkyl group, as defined above, which is non-aromatic and contains at least one endocyclic double bond between two adjacent ring atoms. A heterocycloalkenyl group may be joined via a ring carbon, ring silicon atom or ring nitrogen atom. In one embodiment, a heterocycloalkenyl group is monocyclic and has from about 3 to about 7 ring atoms. In another embodiment, a heterocycloalkenyl group is monocyclic has from about 5 to about 8 ring atoms. In another embodiment, a heterocycloalkenyl group is bicyclic and has from about 8 to about 11 ring atoms. In still another embodiment, a heterocycloalkenyl group is monocyclic and has 5 or 6 ring atoms. In one embodiment, a heterocycloalkenyl group is monocyclic. In another embodiment, a heterocycloalkenyl group is bicyclic. There are no adjacent oxygen and/or sulfur atoms present in the ring system. Any -NH group in a heterocycloalkenyl ring may be substituted or may exist protected such as, for example, as an -N(BOC), -N(Cbz), -N(Tos) group and the like; such protected heterocycloalkenyl groups are considered part of this invention. The term "heterocycloalkenyl" also encompasses a heterocycloalkenyl group, as defined above, which is fused to an aryl (e.g., benzene) or heteroaryl ring. A heterocycloalkenyl group may be optionally substituted by one or more "ring system substituents" which may be the same or different, and are as defined herein below. The nitrogen or sulfur atom of the heterocycloalkenyl may be optionally oxidized to the corresponding N-oxide, S-oxide or S,S-dioxide.

A ring carbon atom of a heterocycloalkenyl group may be functionalized as a carbonyl group. An illustrative example of such a heterocycloalkenyl group is:

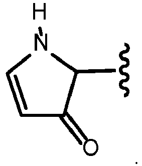

In one embodiment, a heterocycloalkenyl group is a 5-membered monocyclic heterocycloalkenyl. In another embodiment, a heterocycloalkenyl group is a 6-membered monocyclic heterocycloalkenyl. The term "4 to 7-membered monocyclic heterocycloalkenyl" refers to a monocyclic heterocycloalkenyl group having from 4 to 7 ring atoms. The term "5 to 8-membered monocyclic heterocycloalkenyl" refers to a monocyclic heterocycloalkenyl group having from 5 to 8 ring atoms. The term "8 to 11-membered bicyclic heterocycloalkenyl" refers to a bicyclic heterocycloalkenyl group having from 8 to 11 ring atoms. Unless otherwise indicated, a heterocycloalkenyl group is unsubstituted.

The term "ring system substituent," as used herein, refers to a substituent group attached to an aromatic or non-aromatic ring system which, for example, replaces an available hydrogen on the ring system. Ring system substituents may be the same or different, each being independently selected from the group consisting of alkyl, alkenyl, alkynyl, aryl, heteroaryl, -alkylene-aryl, -arylene-alkyl, -alkylene-heteroaryl, -alkenylene-heteroaryl, -alkynylene-heteroaryl, -OH, hydroxyalkyl, haloalkyl, -O-alkyl, -O-haloalkyl, -alkylene-O-alkyl, -O-aryl, -O-alkylene-aryl, acyl, -C(O)-aryl, halo, -NO₂, -CN, -SF₅, -C(O)OH, -C(O)O-alkyl, -C(O)O-aryl, -C(O)O-alkylene-aryl, -S(O)-alkyl, -S(O)₂-alkyl, -S(O)-aryl, -S(O)₂-aryl, -S(O)-heteroaryl, -S(O)₂-heteroaryl, -S-alkyl, -S-aryl, -S-heteroaryl, -S-alkylene-aryl, -S-alkylene-heteroaryl, -S(O)₂-alkylene-aryl, -S(O)₂-alkylene-heteroaryl, -Si(alkyl)₂, -Si(aryl)₂, -Si(heteroaryl)₂, -Si(alkyl)(aryl), -Si(alkyl)(cycloalkyl), - Si(alkyl)(heteroaryl), cycloalkyl, heterocycloalkyl, -O-C(O)-alkyl, -O-C(O)-aryl, -O-C(O)-cycloalkyl, -C(=N-CN)-NH₂, -C(=NH)-NH₂, -C(=NH)-NH(alkyl), -N(Y₁)(Y₂), -alkylene-N(Y₁)(Y₂), -C(O)N(Y₁)(Y₂) and -S(O)₂N(Y₁)(Y₂), wherein Y₁ and Y₂ may be the same or different and are independently selected from the group consisting of hydrogen, alkyl, aryl, cycloalkyl, and -alkylene-aryl. "Ring system substituent" may also mean a single moiety which simultaneously replaces two available hydrogens on two adjacent carbon atoms (one H on each carbon) on a ring system. Examples of such moiety are methylenedioxy, ethylenedioxy, -C(CH₃)₂- and the like which form moieties such as, for example:

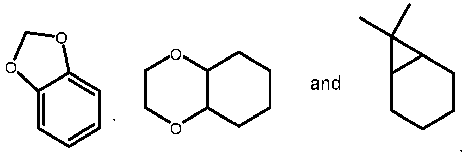

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" is meant a compound that is sufficiently robust to survive isolation to a useful degree of purity from a reaction mixture, and formulation into an efficacious therapeutic agent.

The term "in substantially purified form," as used herein, refers to the physical state of a compound after the compound is isolated from a synthetic process (e.g., from a reaction mixture), a natural source, or a combination thereof. The term "in substantially purified form," also refers to the physical state of a compound after the compound is obtained from a purification process or processes described herein or well-known to the skilled artisan (e.g., chromatography, recrystallization and the like), in sufficient purity to be characterizable by standard analytical techniques described herein or well-known to the skilled artisan.

It should also be noted that any carbon as well as heteroatom with unsatisfied valences in the text, schemes, examples and tables herein is assumed to have the sufficient number of hydrogen atom(s) to satisfy the valences.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in Organic Synthesis (1991), Wiley, New York.

When any substituent or variable (*e.g.,* R¹, R⁴, m, etc.) occurs more than one time in any constituent or in Formula (I), its definition on each occurrence is independent of its definition at every other occurrence, unless otherwise indicated.

As used herein, the term "composition" is intended to encompass a product comprising the specified ingredients in the specified amounts, as well as any product which results from combination of the specified ingredients in the specified amounts.

Pharmaceutically acceptable esters of the present compounds include the following groups: (1) carboxylic acid esters obtained by esterification of the hydroxy group of a hydroxyl compound, in which the non-carbonyl moiety of the carboxylic acid portion of the ester grouping is selected from straight or branched chain alkyl (e.g., methyl, ethyl, n-propyl, isopropyl, t-butyl, sec-butyl or n-butyl), alkoxyalkyl (*e.g.,* methoxymethyl), aralkyl (*e.g.,* benzyl), aryloxyalkyl (for example, phenoxymethyl), aryl (e.g., phenyl optionally substituted with, for example, halogen, C₁₋₄alkyl, -O-(C₁₋₄alkyl) or amino); (2) sulfonate esters, such as alkyl- or aralkylsulfonyl (for example, methanesulfonyl); (3) amino acid esters, including those corresponding to both natural and non-natural amino acids (e.g., L-valyl or L-isoleucyl); (4) phosphonate esters and (5) mono-, di- or triphosphate esters. The phosphate esters may be further esterified by, for example, a C₁₋₂₀ alcohol or reactive derivative thereof, or by a 2,3-di (C₆₋₂₄)acyl glycerol.

One or more compounds of the invention may exist in unsolvated as well as solvated forms with pharmaceutically acceptable solvents such as water, ethanol, and the like, and it is intended that the invention embrace both solvated and unsolvated forms. "Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of solvates include ethanolates, methanolates, and the like. A "hydrate" is a solvate wherein the solvent molecule is water.

One or more compounds of the invention may optionally be converted to a solvate. Preparation of solvates is generally known. Thus, for example, M. Caira et al, J. Pharmaceutical Sci., 93(3), 601-611 (2004) describe the preparation of the solvates of the antifungal fluconazole in ethyl acetate as well as from water. Similar preparations of solvates, hemisolvates, hydrates and the like are described by E. C. van Tonder et al, AAPS PharmSciTechours., 5(1), article 12 (2004); and A. L. Bingham et al, Chem. Commun., 603-604 (2001). A typical, non-limiting, process involves dissolving the inventive compound in desired amounts of the desired solvent (organic or water or mixtures thereof) at a higher than room temperature, and cooling the solution at a rate sufficient to form crystals which are then isolated by standard methods. Analytical techniques such as, for example IR spectroscopy, show the presence of the solvent (or water) in the crystals as a solvate (or hydrate).

The Fused Tricyclic Heterocyclic Compounds can form salts which are also within the scope of this invention. Reference to a Fused Tricyclic Heterocyclic Compound herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a Fused Tricyclic Heterocyclic Compound contains both a basic moiety, such as, but not limited to a pyridine or imidazole, and an acidic moiety, such as, but not limited to a carboxylic acid, zwitterions ("inner salts") may be formed and are included within the term "salt(s)" as used herein. In one embodiment, the salt is a pharmaceutically acceptable (*i.e.,* non-toxic, physiologically acceptable) salt. In another embodiment, the salt is other than a pharmaceutically acceptable salt. Salts of the Compounds of Formula (I) may be formed, for example, by reacting a Fused Tricyclic Heterocyclic Compound with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates) and the like. Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their web

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamine, t-butyl amine, choline, and salts with amino acids such as arginine, lysine and the like. Basic nitrogen-containing groups may be quarternized with agents such as lower alkyl halides (e.g., methyl, ethyl, and butyl chlorides, bromides and iodides), dialkyl sulfates (e.g., dimethyl, diethyl, and dibutyl sulfates), long chain halides (e.g., decyl, lauryl, and stearyl chlorides, bromides and iodides), arylalkyl halides (e.g., benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Diastereomeric mixtures may be separated into their individual diastereomers on the basis of their physical chemical differences by methods well-known to those skilled in the art, such as, for example, by chromatography and/or fractional crystallization. Enantiomers may be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereomers to the corresponding pure enantiomers. Sterochemically pure compounds may also be prepared by using chiral starting materials or by employing salt resolution techniques. Also, some of the Fused Tricyclic Heterocyclic Compounds may be atropisomers (e.g., substituted biaryls) and are considered as part of this invention. Enantiomers can also be directly separated using chiral chromatographic techniques.

It is also possible that the Fused Tricyclic Heterocyclic Compounds may exist in different tautomeric forms, and all such forms are embraced within the scope of the invention. For example, all keto-enol and imine-enamine forms of the compounds are included in the invention.

All stereoisomers (for example, geometric isomers, optical isomers and the like) of the present compounds (including those of the salts, solvates, hydrates, esters and prodrugs of the compounds as well as the salts, solvates and esters of the prodrugs), such as those which may exist due to asymmetric carbons on various substituents, including enantiomeric forms (which may exist even in the absence of asymmetric carbons), rotameric forms, atropisomers, and diastereomeric forms, are contemplated within the scope of this invention. If a Fused Tricyclic Heterocyclic Compound incorporates a double bond or a fused ring, both the cis- and transforms, as well as mixtures, are embraced within the scope of the invention.

Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations. The use of the terms "salt", "solvate", "ester", is intended to apply equally to the salt, solvate, ester and prodrug of enantiomers, stereoisomers, rotamers, tautomers, racemates of the inventive compounds.

### General List of Abbreviations

- ACN =: acetonitrile
- br =: broad
- m-CPBA =: *meta*-chloroperbenzoic acid
- d =: doublet
- DCM =: dichloromethane
- DIEA =: *N,N-*diisopropylethyl amine
- DMF =: dimethylformamide
- DMSO =: dimethyl sulfoxide
- EDC =: 1-ethyl-3-(3-dimethylaminopropyl)carbodiimide
- ESI =: electrospray ionization
- EtOAc =: ethyl acetate
- EtOH =: ethanol
- HOBt =: 1-hydroxy 1H-benzotriazole
- HPLC =: high-performance liquid chromatography
- LCMS =: liquid chromatography / mass sepectrometry
- LiHMDS =: lithium bis(trimethylsilyl)amide
- m =: multiplet
- MeOH =: methanol
- MS =: mass spectroscopy
- Ms =: methanesulfonyl
- NIS =: *N*-iodosuccinimide
- NMR =: nuclear magnetic resonance spectroscopy
- Ph =: phenyl
- s =: singlet
- SFC =: supercritical fluid chromatography
- t =: triplet
- TBAF =: tetra(n-butyl)ammonium fluoride
- TBS =: tert-butyl dimethyl silyl
- TEA =: triethylamine
- TFA =: trifluoroacetic acid
- THF =: tetrahydrofuran
- TLC =: thin-layer chromatography
- Ts =: *para*-toluenesulfonyl
- wt% =: weight percent

### The Compounds of Formula (I)

The present invention provides Fused Tricyclic Heterocyclic Compounds of Formula (I): 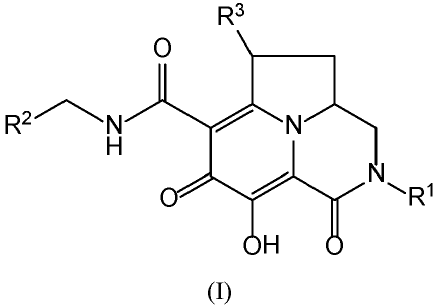 and pharmaceutically acceptable salts thereof, wherein R¹, R² and R³ are defined above for the Compounds of Formula (I).

In one embodiment, the compound of formula (I) has the formula (Ia), (Ib), (Ic) or (Id):

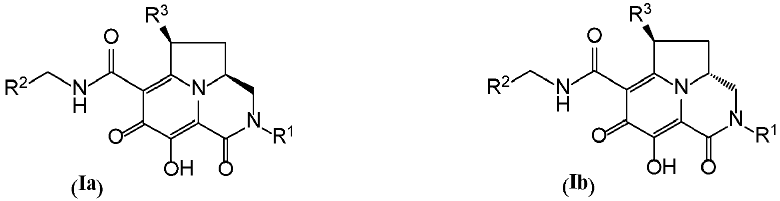

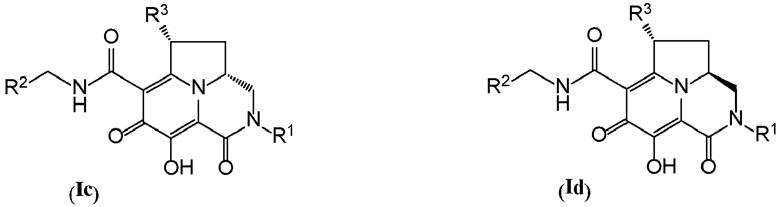

In one embodiment, for the compounds of formulas (I), (Ia), (Ib), (Ic) and (Id):
R¹ is C₁-C₆ alkyl;
R² is phenyl, which is optionally substituted with up to 3 ring substituent groups, each independently selected from halo;
R³ is -N(R⁴)₂;
each occurrence of R⁴ is independently selected from H, C₁-C₆ alkyl, C₁-C₆ haloalkyl, C₃-C₇ cycloalkyl, phenyl, -C(O)-C(O)-N(R⁵)₂, -S(O)₂-C₁-C₆ alkyl and -C(O)-C₁-C₆ alkyl; or both R⁴ groups, and the common nitrogen atom to which they are attached, join to form an azetidinyl, piperidinyl, or pyrrolidinyl group; and
each occurrence of R⁵ is independently selected from H and C₁-C₆ alkyl.

In one embodiment, for the compounds of formulas (I), (Ia), (Ib), (Ic) and (Id), R¹ is selected from methyl, ethyl, n-propyl and -CH₂CH₂OCH₃.

In another embodiment, for the compounds of formulas (I), (Ia), (Ib), (Ic) and (Id), R¹ is C₁-C₆ alkyl.

In another embodiment, for the compounds of formulas (I), (Ia), (Ib), (Ic) and (Id), R¹ is ethyl.

In one embodiment, for the compounds of formulas (I), (Ia), (Ib), (Ic) and (Id), R² is selected from:

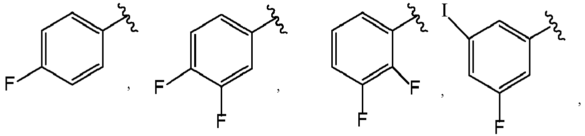

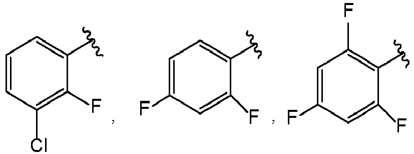

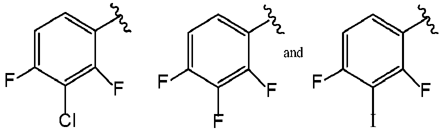

In still another embodiment, for the compounds of formulas (I), (Ia), (Ib), (Ic) and (Id), R² is:

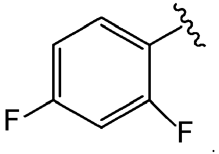

In one embodiment, for the compounds of formulas (I), (Ia), (Ib), (Ic) and (Id), R³ is selected from -NH₂, -NHCH₃, -NHCH₂CH₃, -NHCH₂CH₂CH₃, -NHCH₂-cyclopropyl, -NH-benzyl, -NHCH₂CH₂OCH₃, -NHCH₂CH₂CF₃, -NHCD₂CD₃, pyrrolidinyl, -N(CH₃)₂, azetidinyl, -N(CH₂)₃Cl, -NHS(O)₂CH₃, -NHS(O)₂-(p-toluene), -NHC(O)CH₃ and -NHC(O)C(O)N(CH₃)₂.

In another embodiment, for the compounds of formulas (I), (Ia), (Ib), (Ic) and (Id), R³ is -N(R⁴)₂, and each occurrence of R⁴ is independently selected from H and C₁-C₆ alkyl.

In another embodiment, for the compounds of formulas (I), (Ia), (Ib), (Ic) and (Id), R³ is -NHCH₂CH₃.

In one embodiment, for the compounds of formulas (I), (Ia), (Ib), (Ic) and (Id), R¹ is selected from methyl, ethyl, n-propyl and -CH₂CH₂OCH₃; R² is selected from: 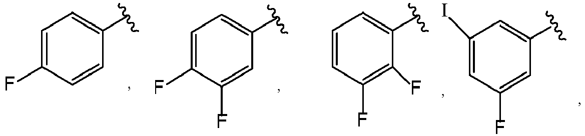 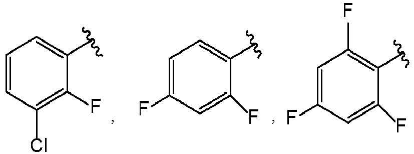 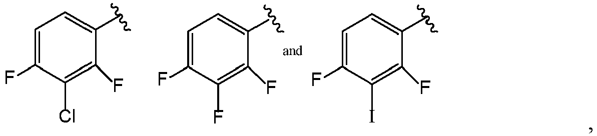 and R³ is selected from -NH₂, -NHCH₃, -NHCH₂CH₃, -NHCH₂CH₂CH₃, -NHCH₂-cyclopropyl, -NH-benzyl, -NHCH₂CH₂OCH₃, -NHCH₂CH₂CF₃, -NHCD₂CD₃, pyrrolidinyl, -N(CH₃)₂, azetidinyl, -N(CH₂)₃Cl, -NHS(O)₂CH₃, -NHS(O)₂-(p-toluene), -NHC(O)CH₃ and -NHC(O)C(O)N(CH₃)₂.

In another embodiment, for the compounds of formulas (I), (Ia), (Ib), (Ic) and (Id), R¹ is C₁-C₆ alkyl; R² is selected from: 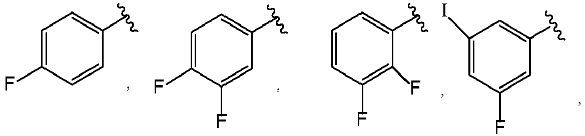 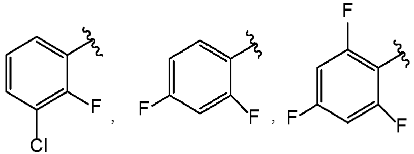 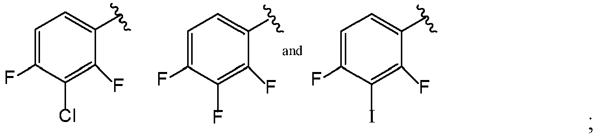 and R³ is -N(R⁴)₂, wherein each occurrence of R⁴ is independently selected from H and C₁-C₆ alkyl.

In another embodiment, for the compounds of formulas (I), (Ia), (Ib), (Ic) and (Id), R¹ is C₁-C₆ alkyl; R² is: 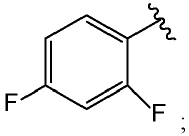 and R³ is -N(R⁴)₂, wherein each occurrence of R⁴ is independently selected from H and C₁-C₆ alkyl.

In another embodiment, for the compounds of formulas (I), (Ia), (Ib), (Ic) and (Id), R¹ is ethyl; R² is: 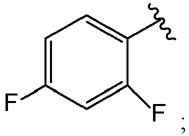 and R³ is selected from -NH₂, -NHCH₃, -NHCH₂CH₃, -NHCH₂CH₂CH₃, -NHCH₂-cyclopropyl, - NH-benzyl, -NHCH₂CH₂OCH₃, -NHCH₂CH₂CF₃, -NHCD₂CD₃, pyrrolidinyl, -N(CH₃)₂, azetidinyl, -N(CH₂)₃Cl, -NHS(O)₂CH₃, -NHS(O)₂-(p-toluene), -NHC(O)CH₃ and -NHC(O)C(O)N(CH₃)₂.

In another embodiment, for the compounds of formulas (I), (Ia), (Ib), (Ic) and (Id), R¹ is ethyl; R² is: 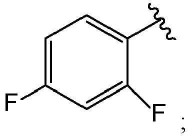 and R³ is -N(R⁴)₂, wherein each occurrence of R⁴ is independently selected from H and C₁-C₆ alkyl.

In one embodiment, variables R¹, R² and R³ for the compounds of formulas (I), (Ia), (Ib), (Ic) and (Id), are selected independently of each other.

In another embodiment, the compounds of formulas (I), (Ia), (Ib), (Ic) and (Id), are in substantially purified form.

Other embodiments of the present invention include the following (The references to methods of treatment in the subsequent paragraphs of this description are to be interpreted as references to the compounds, pharmaceutical compositions and medicaments of the present invention for use in a method for treatment of the human (or animal) body by therapy):
(a) A pharmaceutical composition comprising an effective amount of a Compound of Formula (I), and a pharmaceutically acceptable carrier.
(b) The pharmaceutical composition of (a), further comprising a second therapeutic agent selected from the group consisting of HIV antiviral agents, immunomodulators, and anti-infective agents.
(c) The pharmaceutical composition of (b), wherein the HIV antiviral agent is an antiviral selected from the group consisting of HIV protease inhibitors and HIV NNRTI inhibitors.
(d) A pharmaceutical combination that is (i) a Compound of Formula (I) and (ii) a second therapeutic agent selected from the group consisting of HIV antiviral agents, immunomodulators, and anti-infective agents; wherein the Compound of Formula (I) and the second therapeutic agent are each employed in an amount that renders the combination effective for inhibiting HIV replication, or for treating HIV infection and/or reducing the likelihood or severity of symptoms of HIV infection.
(e) The combination of (d), wherein the HIV antiviral agent is an antiviral selected from the group consisting of HIV protease inhibitors and HIV NNRTI inhibitors.
(f) A method of inhibiting HIV replication in a subject in need thereof which comprises administering to the subject an effective amount of a Compound of Formula (I).
(g) A method of treating HIV infection and/or reducing the likelihood or severity of symptoms of HIV infection in a subject in need thereof which comprises administering to the subject an effective amount of a Compound of Formula (I).
(h) The method of (g), wherein the Compound of Formula (I) is administered in combination with an effective amount of at least one second therapeutic agent selected from the group consisting of HIV antiviral agents, immunomodulators, and anti-infective agents.
(i) The method of (h), wherein the HIV antiviral agent is an antiviral selected from the group consisting of HIV protease inhibitors and HIV NNRTI inhibitors.
(j) A method of inhibiting HIV replication in a subject in need thereof which comprises administering to the subject the pharmaceutical composition of (a), (b) or (c) or the combination of (d) or (e).
(k) A method of treating HIV infection and/or reducing the likelihood or severity of symptoms of HIV infection in a subject in need thereof which comprises administering to the subject the pharmaceutical composition of (a), (b) or (c) or the combination of (d) or (e).

Additional embodiments of the present invention include the following:
(1) A pharmaceutical composition comprising an effective amount of a pharmaceutically acceptable salt of a Compound of Formula (I), and a pharmaceutically acceptable carrier.
(m) The pharmaceutical composition of (1), further comprising a second therapeutic agent selected from the group consisting of HIV antiviral agents, immunomodulators, and anti-infective agents.
(n) The pharmaceutical composition of (m), wherein the HIV antiviral agent is an antiviral selected from the group consisting of HIV protease inhibitors and HIV NNRTI inhibitors.
(o) A pharmaceutical combination that is (i) a pharmaceutically acceptable salt of a Compound of Formula (I) and (ii) a second therapeutic agent selected from the group consisting of HIV antiviral agents, immunomodulators, and anti-infective agents; wherein the pharmaceutically acceptable salt of the Compound of Formula (I) and the second therapeutic agent are each employed in an amount that renders the combination effective for inhibiting HIV replication, or for treating HIV infection and/or reducing the likelihood or severity of symptoms of HIV infection.
(p) The combination of (o), wherein the HIV antiviral agent is an antiviral selected from the group consisting of HIV protease inhibitors and HIV NNRTI inhibitors.
(q) A method of inhibiting HIV replication in a subject in need thereof which comprises administering to the subject an effective amount of a pharmaceutically acceptable salt of a Compound of Formula (I).
(r) A method of treating HIV infection and/or reducing the likelihood or severity of symptoms of HIV infection in a subject in need thereof which comprises administering to the subject an effective amount of a pharmaceutically acceptable salt of a Compound of Formula (I).
(s) The method of (r), wherein the pharmaceutically acceptable salt of the Compound of Formula (I) is administered in combination with an effective amount of at least one second therapeutic agent selected from the group consisting of HIV antiviral agents, immunomodulators, and anti-infective agents.
(t) The method of (s), wherein the HIV antiviral agent is an antiviral selected from the group consisting of HIV protease inhibitors and HIV NS5B polymerase inhibitors.
(u) A method of inhibiting HIV replication in a subject in need thereof which comprises administering to the subject the pharmaceutical composition of (1), (m) or (n) or the combination of (o) or (p).
(v) A method of treating HIV infection and/or reducing the likelihood or severity of symptoms of HIV infection in a subject in need thereof which comprises administering to the subject the pharmaceutical composition of (1), (m) or (n) or the combination of (o) or (p).

Further embodiments of the present invention include the following:
(w) A pharmaceutical composition comprising an effective amount of a Compound of Formula (I) or a pharmaceutically acceptable salt thereof, and a pharmaceutically acceptable carrier.
(x) The pharmaceutical composition of (w), further comprising a second therapeutic agent selected from the group consisting of HIV antiviral agents, immunomodulators, and anti-infective agents.
(y) The pharmaceutical composition of (x), wherein the HIV antiviral agent is an antiviral selected from the group consisting of HIV protease inhibitors and HIV NNRTI inhibitors.
(z) A pharmaceutical combination that is (i) a Compound of Formula (I) and (ii) or a pharmaceutically acceptable salt thereof, a second therapeutic agent selected from the group consisting of HIV antiviral agents, immunomodulators, and anti-infective agents; wherein the Compound of Formula (I) and the second therapeutic agent are each employed in an amount that renders the combination effective for inhibiting HIV replication, or for treating HIV infection and/or reducing the likelihood or severity of symptoms of HIV infection.
(aa) The combination of (z), wherein the HIV antiviral agent is an antiviral selected from the group consisting of HIV protease inhibitors and HIV NNRTI inhibitors.
(bb) A method of inhibiting HIV replication in a subject in need thereof which comprises administering to the subject an effective amount of a Compound of Formula (I) or a pharmaceutically acceptable salt thereof.
(cc) A method of treating HIV infection and/or reducing the likelihood or severity of symptoms of HIV infection in a subject in need thereof which comprises administering to the subject an effective amount of a Compound of Formula (I) or a pharmaceutically acceptable salt thereof.
(dd) The method of (cc), wherein the Compound of Formula (I) or pharmaceutically acceptable salt thereof, is administered in combination with an effective amount of at least one second therapeutic agent selected from the group consisting of HIV antiviral agents, immunomodulators, and anti-infective agents.
(ee) The method of (dd), wherein the HIV antiviral agent is an antiviral selected from the group consisting of HIV protease inhibitors and HIV NNRTI inhibitors.
(ff) A method of inhibiting HIV replication in a subject in need thereof which comprises administering to the subject the pharmaceutical composition of (w) (x) or (y) or the combination of (z) or (aa).
(gg) A method of treating HIV infection and/or reducing the likelihood or severity of symptoms of HIV infection in a subject in need thereof which comprises administering to the subject the pharmaceutical composition of (w) (x) or (y) or the combination of (z) or (aa).

The present invention also includes a compound of the present invention for use (i) in, (ii) as a medicament for, or (iii) in the preparation of a medicament for: (a) medicine; (b) inhibiting HIV replication or (c) treating HIV infection and/or reducing the likelihood or severity of symptoms of HIV infection. In these uses, the compounds of the present invention can optionally be employed in combination with one or more second therapeutic agents selected from HIV antiviral agents, anti-infective agents, and immunomodulators.

Additional embodiments of the invention include the pharmaceutical compositions, combinations and methods set forth in (a)-(gg) above and the uses set forth in the preceding paragraph, wherein the compound of the present invention employed therein is a compound of one of the embodiments, aspects, classes, sub-classes, or features of the compounds described above. In all of these embodiments, the compound may optionally be used in the form of a pharmaceutically acceptable salt or hydrate as appropriate.

It is further to be understood that the embodiments of compositions and methods provided as (a) through (gg) above are understood to include all embodiments of the compounds, including such embodiments as result from combinations of embodiments.

Non-limiting examples of the Compounds of Formula (I) include compounds 1-75 as set forth in the Examples below, and pharmaceutically acceptable salts thereof.

### EXAMPLES

### General Methods

The following examples serve only to illustrate the invention and its practice. The examples are not to be construed as limitations on the scope or spirit of the invention. In these examples, all temperatures are degrees Celsius unless otherwise noted, and "room temperature" refers to a temperature in a range of from about 20°C to about 25°C. Reactions sensitive to moisture or air were performed under nitrogen using anhydrous solvents and reagents. The progress of reactions was determined by either analytical thin layer chromatography (TLC) performed with E. Merck precoated TLC plates, silica gel 60F-254, layer thickness 0.25 mm or liquid chromatography-mass spectrum (LC-MS). For HPLC/MS data, two HPLC conditions used were as follows: 1) LC1 (SHIMADZU C18 Xtimate 3um 2.1×30 mm column with gradient 10:90-80:20 v/v CH3CN/H20 + v 0.0375 % TFA over 0.9 min then hold at 80:20 v/v CH3CN/H20 + v 0.0375% TFA for 0.6 min; flow rate 1.2 mL/min, UV wavelength 220 & 254 nm); 2) LC2 (Agilent C18 Xtimate 3um 2.1×30 mm column with gradient 10:90-80:20 v/v CH3CN/H2O + v 0.0375 % TFA over 3.0 min then hold at 80:20 v/v CH3CN/H20 + v 0.0375 % TFA for 0.5 min; flow rate 0.8 mL/min, UV wavelength 220 & 254 nm). Mass analysis was performed with electrospray ionization in positive ion detection mode. ¹H NMR spectra were recorded on Varian or Bruker instruments at 400-500 MHz. Concentration of solutions was carried out on a rotary evaporator under reduced pressure or by lyophilization. Flash chromatography was performed on pre-packed silica gel columns using a commercial MPLC system.

### EXAMPLE 1

Preparation of Intermediate Compound **Int-1a** and Compound **Int-1b**

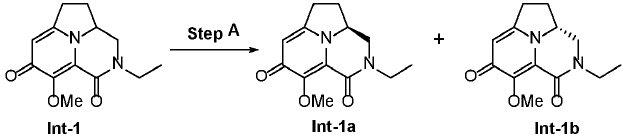

Compound Int-1 was prepared using the method described in PCT International Patent Publication No. WO2014/183532. The racemic compound Int-1 (2.0 g, 8.0 mmol) was further separated using a chiral preparative SFC ("Column: AS (250 mm ^{∗} 30 mm, 5 um), Mobile phase: Supercritical CO2/EtOH (base) = 100/40 at 50 mL/min Wavelength: 220 nm) to provide compound **Int-1a** (the first eluting compound) as an oil and compound **Int-1b** (the second eluting compound) as an oil.

### EXAMPLE 2

Preparation of Compound 1 and Compound 2

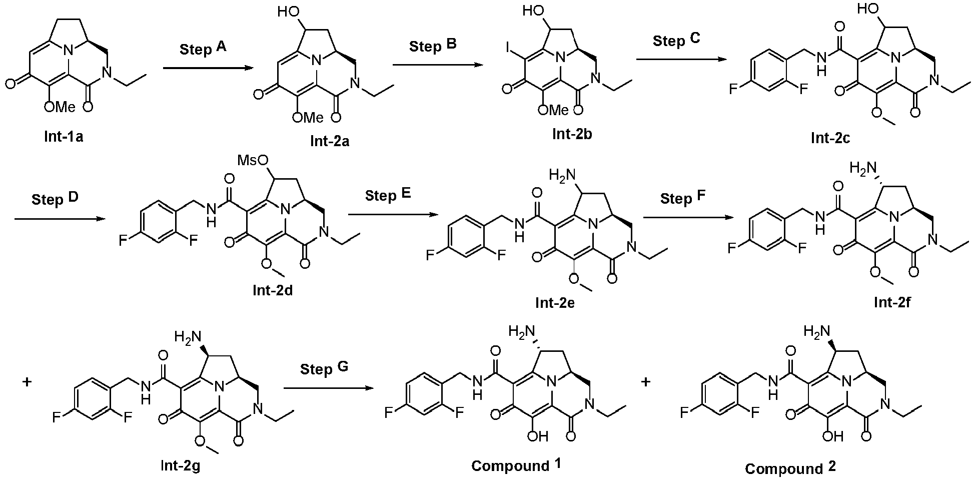

### Step A- Synthesis of Compound Int-2a

To a solution of compound **Int-1a** (1500 mg, 6.04 mmol) in THF (70 mL) was added dropwise LiHMDS (15.10 mL, 15.10 mmol, 1M in THF) at -78°C and the reaction solution was allowed to stir at -78°C for 1 hr. A solution of 3-phenyl-2-(phenylsulfonyl)-1,2-oxaziridine (2368 mg, 9.06 mmol) in THF (18 mL) was added to the above reaction solution. The resulting mixture was then warmed to 26 °C and allowed to stir for another 1.0 hours. The mixture was concentrated and purified using a silica gel column eluting with 10% MeOH in dichloromethane, followed by 20% MeOH in dichloromethane to provide compound **Int-2a** as an oil. **¹H NMR** (400MHz, CD₃OD): δ 6.55-6.70 (m, 1H), 5.11-5.27 (m, 1H), 4.40-4.63 (m, 1H), 3.74-3.88 (m, 4H), 3.54-3.68 (m, 3H), 2.39 (dd, J = 13.5, 5.7 Hz, 1H), 2.06-2.23 (m, 1H), 1.21 (t, J = 7.0 Hz, 3H). **MS:** *m*/*z* = 265.0 (M + 1).

### Step B- Synthesis of Compound Int-2b

To a solution of compound **Int-2a** (750 mg, 2.84 mmol) in MeOH (30 mL) was added 1-iodopyrrolidine-2,5-dione (1277 mg, 5.68 mmol) and 3-chlorobenzoperoxoic acid (1224 mg, 5.68 mmol). The mixture was allowed to stir at 70°C for 30 minutes. The reaction was quenched with aqueous 1 M Na₂SO₃ solution (10 mL), filtered and concentrated *in vacuo.* The crude product was purified using a silica gel column eluting with 5% MeOH in dichloromethane and 10% MeOH in dichloromethane to provide compound **Int-2b** as a solid. **¹H NMR** (400MHz, CD₃OD): δ5.21-5.33 (m, 1H), 4.96 (brs, 1H), 3.87 (s, 4H), 3.45-3.66 (m, 3H), 2.42 (dd, J = 13.5, 5.7 Hz, 1H), 2.19-2.28 (m, 1H), 1.21 (t, J = 7.0 Hz, 3H). **MS:** *m*/*z* = 391.0 (M + 1).

### Step C- Synthesis of Compound Int-2c

To a solution of compound **Int-2b** (500 mg, 1.281 mmol) in DMSO (10 mL) was added (2,4-difluorophenyl)methanamine (917 mg, 6.41 mmol), *N*-ethyl-*N*-isopropylpropan-2-amine (1.656 g, 12.81 mmol) and Pd(PPh₃)₄ (296 mg, 0.256 mmol). The mixture was allowed to stir at 80°C under a CO balloon for 1 hour. The reaction was quenched with water (10 mL) and the mixture was extracted with EtOAc (20 mL × 3). The combined organic extracts were washed with brine (30 mL), the organic layer was dried over anhydrous Na₂SO₄, filtered and concentrated *in vacuo.* The crude product was purified using a preparative TLC plate eluting with 10% MeOH /dichloromethane to provide compound **Int-2c** as a solid. **MS:** *m*/*z* = 391.0 (M +1).

### Step D- Synthesis of Compound Int-2d

To a solution of compound **Int-2c** (150 mg, 0.346 mmol) in CH₂Cl₂ (5 mL) was added triethylamine (0.145 mL, 1.038 mmol) and methanesulfonyl chloride (79 mg, 0.692 mmol) at 0°C. The resulting mixture was allowed to stir at 18°C for 2 hours. The reaction was quenched by water (2 mL) and the aqueous was extracted with EtOAc (10 mL × 3). The combined organics were washed with brine (10 mL), and the organic layer was dried over anhydrous Na₂SO₄, filtered and evaporated. The crude product was purified using a preparative TLC plate eluting with 10% MeOH in dichloromethane to provide compound **Int-2d** as a solid. **MS:** *m*/*z* = 391.0 (M + 1).

### Step E- Synthesis of Compound Int-2e

To a solution of compound **Int-2d** (150 mg, 0.293 mmol) in EtOH (10 mL) was added ammonia (0.419 mL, 2.93 mmol, 7 M in MeOH) and the mixture was allowed to stir at 75°C 2 hours. The reaction solution was concentrated *in vacuo* and purified using a preparative TLC plate eluting with 5% CH₂Cl₂ / MeOH to provide compound **Int-2e** as a mixture of stereoisomers. **MS:** *m*/*z* = 433.2 (M + 1).

### Step F- Synthesis of Compound Int-2f and Compound Int-2g

The stereoisomers of compound **Int-2e** was further separated by SFC ("Column: AD (250 mm ^{∗} 30 mm, 10 um) Mobile phase:45% Base-MeOH (contained 0.1% NH₃H₂O) in CO₂ Flow rate: 80 mL/min Wavelength: 220 nm") to provide compound **Int-2f** (the first eluting compound ) as an oil and compound **Int-2g** (the second eluting compound) as an oil.

Compound **Int-2f: ¹H NMR** (400MHz, CDCl₃): δ 10.96 (brs, 1H), 7.30-7.43 (m, 1H), 6.74-6.90 (m, 2H), 5.32 (d, J = 14.8 Hz, 1H), 4.87 (brs, 1H), 4.50-4.71 (m, 2H), 3.94-4.10 (m, 3H), 3.53-3.68 (m, 3H), 2.28-2.48 (m, 2H), 2.01 (br. s., 1H), 1.26 (brs, 3H). MS: *m*/*z* = 433.2 (M + 1).

Compound **Int-2g: ¹H NMR** (400MHz, CDCl₃): δ 10.91 (brs, 1H), 7.29-7.42 (m, 1 H), 6.73-6.87 (m, 2H), 5.03 (t, J = 8.2 Hz, 1H), 4.54-4.66 (m, 2H), 4.22-4.37 (m, 1H), 3.93-4.03 (m, 3H), 3.67-3.76 (m, 1H), 3.55-3.65 (m, 3H), 2.93 (dt, J = 12.9, 6.8 Hz, 1H), 1.68-1.80 (m, 1H), 1.24 (d, J = 8.2 Hz, 3H). **MS:** *m*/*z* = 433.2 (M + 1).

### Step G- Synthesis of Compound 1 and Compound 2

To a solution of compound **Int-2f** (15 mg, 0.035 mmol) in acetonitrile (3 mL) was added TFA (0.2 mL) at 25°C. The mixture was allowed to stir at 25°C for 0.5 hours. Then magnesium bromide (7.66 mg, 0.042 mmol) was added to the mixture and the reaction was allowed to stir for 1 hour. The mixture was filtered and purified by a preparative HPLC (Column: Phenomenex Synergi C18 250 ^{∗} 50 mm ^{∗} 10 um; Condition: 0.1% TFA-ACN; Gradient: 14% to 44%; B, 0∼8 minutes. Flow Rate: 30 mL/min) to provide compound **1** as a solid. **¹H NMR** (400MHz, CD₃OD): δ 7.40-7.50 (m, 1H), 6.91-7.01 (m, 2H), 5.47 (d, J = 8.5 Hz, 1H), 4.66 (brs, 3H), 3.91-4.01 (m, 1H), 3.55-3.76 (m, 3H), 2.47-2.73 (m, 2H), 1.26 (t, J = 7.2 Hz, 3H). **MS:** m/z = 419.2.

To a solution of compound **Int-2g** (25 mg, 0.058 mmol) in acetonitrile (2.0 mL) was added TFA (0.1 mL) at 25°C. The mixture was allowed to stir at 25°C for 0.5 hours. Then magnesium bromide (15.97 mg, 0.087 mmol) was added to the stirred mixture, and allowed to stir for another 1 hour. The mixture was filtered and purified using preparative HPLC (Column: Phenomenex Synergi C18 250 ^{∗} 50 mm ^{∗} 10 um; Condition: 0.1% TFA-ACN; Gradient: 15% to 45%; B, 0~8 minutes. Flow Rate: 30 mL/min) to provide **compound 2** as a solid. **¹H NMR** (400MHz, CD₃OD): δ 7.39-7.50 (m, 1H), 6.90-6.99 (m, 2H), 5.29 (brs, 1H), 4.61-4.69 (m, 3H), 3.98 (dd, J = 12.8, 4.0 Hz, 1H), 3.61-3.75 (m, 3H), 3.12 (d, J = 1.5 Hz, 1H), 2.08 (brs, 1H), 1.23-1.27 (m, 3H). **MS:** *m*/*z* = 419.2.2 (M + 1).

### EXAMPLE 3

### Preparation of Compound 3 and Compound 4

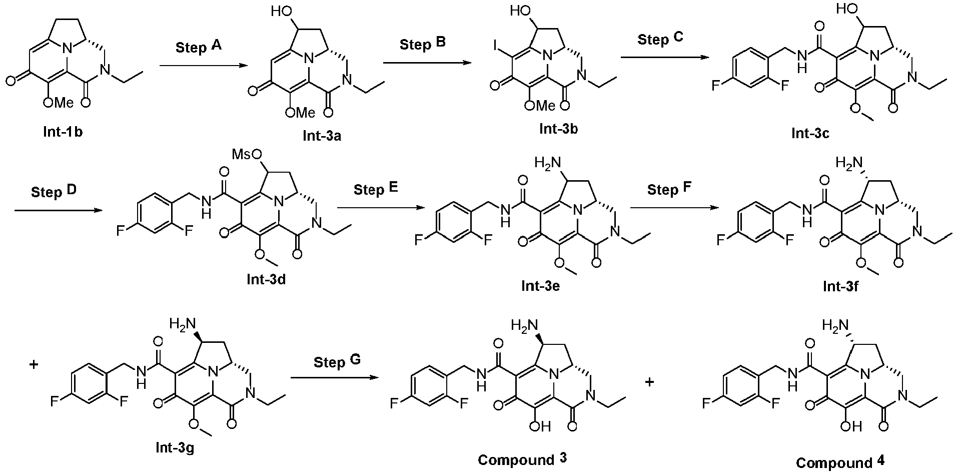

Starting with compound **Int-1a** in place of compound **Int-lb,** compounds **3** and **4** were prepared using the method described in Example 2.

Compound **3: ¹H NMR** (400MHz, CDCl₃): δ 7.45 (brs, 1H), 6.81-7.03 (m, 2H), 5.47 (d, J = 18.8 Hz, 1H), 4.65 (brs, 3H), 3.94 (d, J = 11.7 Hz, 1H), 3.49-3.79 (m, 3H), 2.48-2.71 (m, 2H), 1.16-1.40 (m, 3H). **MS:** *m*/*z* = 419.0 (M + 1).

Compound **4: ¹H NMR** (400MHz, CDCl₃): δ 7.45 (d, J=6.6 Hz, 1H), 6.85-7.04 (m, 2H), 5.29 (brs, 1H), 4.55-4.76 (m, 3H), 3.93-4.04 (m, 1H), 3.57-3.80 (m, 3H), 3.12 (d, J = 6.6 Hz, 1H), 2.08 (d, J = 7.0 Hz, 1H), 1.26 (t, J = 7.0 Hz, 3H). **MS:** *m*/*z* = 419.0 (M + 1).

### EXAMPLE 4

### Preparation of Compound 5 and Compound 6

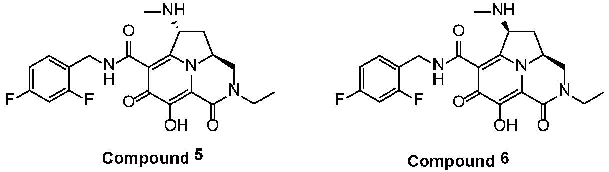

Compounds 5 and 6 were prepared from compound **Int-1a**, using the method described in Example 2, and replacing ammonia with methylamine in Step E.

Compound **5: ¹H NMR** (400MHz, CD₃OD): δ 7.45 (brs, 1H), 6.96 (d, J = 10.1 Hz, 2H), 5.44 (brs, 1H), 4.65 (brs, 3H), 3.95 (d, J = 11.5 Hz, 1H), 3.58-3.80 (m, 3H), 2.87 (brs, 4H), 2.49 (brs, 1H), 1.25 (t, J = 6.8 Hz, 3H). **MS:** *m*/*z* = 433.1 (M + 1).

Compound **6: ¹H NMR** (400MHz, CD₃OD): δ 7.37-7.47 (m, 1H), 6.87-7.02 (m, 2H), 5.28 (t, J = 8.2 Hz, 1H), 4.53-4.73 (m, 3H), 3.61-4.03 (m, 4H), 3.03-3.17 (m, 1H), 2.83 (s, 3H), 2.14-2.28 (m, 1H), 1.24 (t, J = 7.0 Hz, 3H). **MS:** *m*/*z* = 433.1 (M + 1).

### EXAMPLE 5

### Preparation of Compound 7 and Compound 8

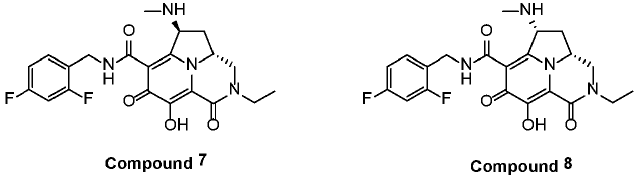

Compounds 7 and **8** were prepared from compound Int-lb, using the method described in Example 2, and replacing ammonia with methylamine in Step E.

Compound **7: ¹H NMR** (400MHz, CD₃OD): δ 7.43 (q, J = 7.8 Hz, 1H), 6.86-7.06 (m, 2H), 5.41 (d, J = 8.6 Hz, 1H), 4.55-4.79 (m, 3H), 3.93 (dd, J = 12.7, 3.3 Hz, 1H), 3.53-3.76 (m, 3H), 2.71-2.94 (m, 4H), 2.42-2.57 (m, 1H), 1.24 (t, J = 7.0 Hz, 3H). **MS:** *m*/*z* = 433.1 (M + 1).

Compound **8: ¹H NMR** (400MHz, CD₃OD): δ 7.38-7.49 (m, 1H), 6.87-7.04 (m, 2H), 5.29 (t, J = 8.4 Hz, 1H), 4.61-4.76 (m, 3H), 3.97 (dd, J = 12.9, 3.9 Hz, 1H), 3.60-3.81 (m, 3H), 3.04-3.17 (m, 1H), 2.83 (s, 3H), 2.12-2.25 (m, 1H), 1.25 (t, J = 7.0 Hz, 3H). **MS:** *m*/*z=*433.1 (M + 1).

### EXAMPLE 6

### Preparation of Compound 9 and Compound 10

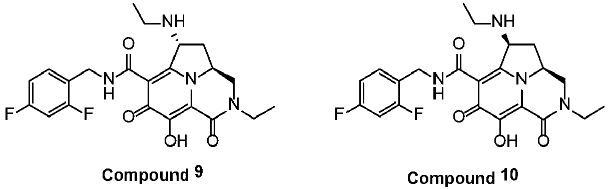

Compounds **9** and **10** were prepared from compound **Int-1a**, using the method described in Example 2, and replacing ammonia with ethylamine in Step E.

Compound **9: ¹H NMR** (400MHz, CD₃OD): δ 7.39-7.53 (m, 1H), 6.88-7.02 (m, 2H), 5.44 (d, J = 8.6 Hz, 1H), 4.55-4.78 (m, 3H), 3.93 (dd, J = 12.7, 3.7 Hz, 1H), 3.53-3.79 (m, 3H), 2.80 (dd, J = 14.7, 6.1 Hz, 1H), 2.46 (dt, J=14.7, 9.5 Hz, 1H), 1.40 (t, J = 7.0 Hz, 3H), 1.24 (t, J=7.0 Hz, 3H). **MS:** *m*/*z* = 447.2 (M + 1).

Compound **10: ¹H NMR** (400MHz, CD₃OD): δ 7.37-7.52 (m, 1H), 6.87-7.03 (m, 2H), 5.33 (t, J = 8.0 Hz, 1H), 4.57-4.74 (m, 3H), 3.97 (dd, J = 12.9, 3.1 Hz, 1H), 3.54-3.83 (m, 3H), 3.18-3.25 (m, 2H), 3.04-3.14 (m, 1H), 2.22 (d, J = 9.4 Hz, 1H), 1.39 (t, J = 7.2 Hz, 3H), 1.24 (t, J = 7.0 Hz, 3H). **MS:** *m*/*z* = 447.2 (M + 1).

### EXAMPLE 7

### Preparation of Compound 11 and Compound 12

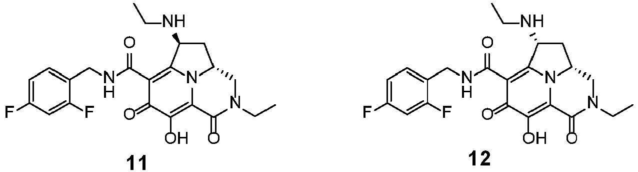

Compounds **11** and **12** were prepared from compound **Int-lb,** using the method described in Example **2,** and replacing ammonia with ethylamine in Step E.

Compound **11: ¹H NMR** (400MHz, CD₃OD): δ 7.38-7.58 (m, 1H), 6.82-7.04 (m, 2H), 5.43 (d, J = 8.6 Hz, 1H), 4.50-4.79 (m, 3H), 3.88-4.02 (m, 1H), 3.50-3.75 (m, 3H), 2.81 (dd, J = 14.5, 5.9 Hz, 1H), 2.41-2.54 (m, 1H), 1.40 (t, J = 6.7 Hz, 3H), 1.23 (t, J = 7.2 Hz, 3H). **MS:** *m*/*z* = 447.2 (M + 1).

Compound **12: ¹H NMR** (400MHz, CD₃OD): δ 7.39-7.50 (m, 1H), 6.86-7.02 (m, 2H), 5.34 (t, J = 8.6 Hz, 1H), 4.60-4.77 (m, 3H), 3.98 (dd, J = 12.9, 3.9 Hz, 1H), 3.57-3.80 (m, 3H), 3.18-3.25 (m, 2H), 3.04-3.12 (m, 1H), 2.11-2.23 (m, 1H), 1.39 (t, J = 7.2 Hz, 3H), 1.24 (t, J = 7.2 Hz, 3H). **MS:** *m*/*z* = 447.2 (M + 1).

### EXAMPLE 8

### Preparation of Compounds 13-32

Starting from compound **Int-lb,** and using the method described in Example 7, the following compounds were prepared.

| Compound # | Structure | Rt (min) | MS (M+H) |
|---|---|---|---|
| **13** | 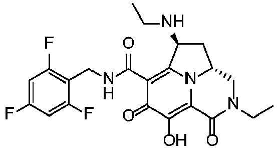 | 2.17 (LC5) | 465.0 |
| **14** | 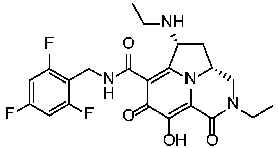 | 2.26 (LC5) | 465.0 |
| **15** | 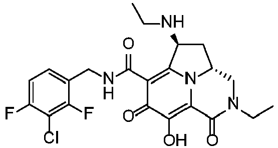 | 2.39 (LC5) | 481.0 |
| **16** | 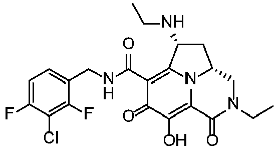 | 2.42 (LC5) | 481.0 |
| **17** | 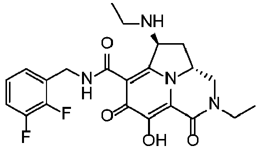 | 2.19 (LC5) | 447.0 |
| **18** | 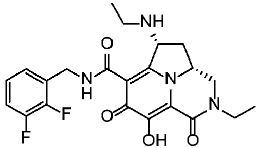 | 2.21 (LC5) | 447.0 |
| **19** | 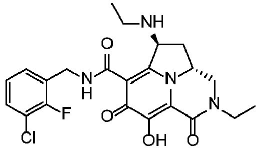 | 2.33(LC5) | 463.0 |
| **20** | 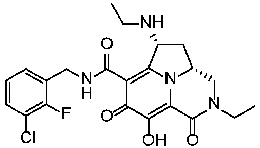 | 2.35 (LC5) | 463.0 |
| **21** | 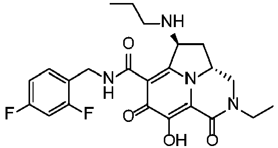 | 2.32 (LC5) | 461.0 |
| **22** | 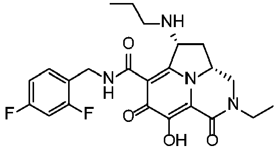 | 2.32 (LC5) | 461.0 |
| **23** | 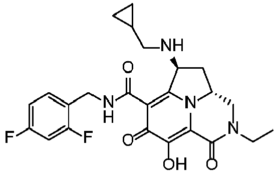 | 2.44 (LC5) | 473.2 |
| **24** | 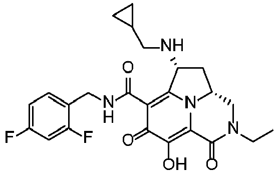 | 2.33 (LC5) | 473.0 |
| **25** | 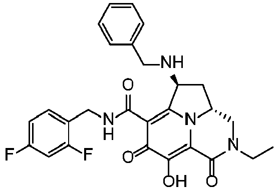 | 2.47 (LC5) | 509.0 |
| **26** | 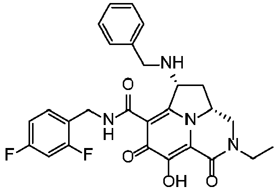 | 2.33 (LC5) | 509.2 |
| 27 | 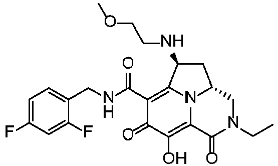 | 2.26 (LC5) | 477.0 |
| **28** | 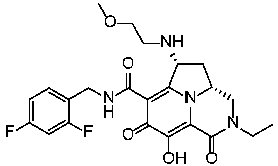 | 2.38 (LC5) | 477.0 |
| **29** | 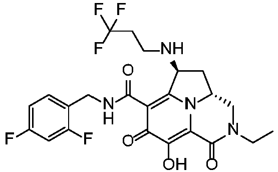 | 2.43 (LC5) | 515.0 |
| **30** | 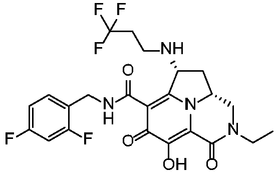 | 2.43 (LC5) | 515.0 |
| **31** | 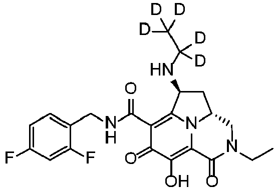 | 2.35 (LC5) | 452.0 |
| **32** | 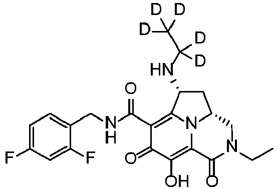 | 2.32 (LC5) | 452.0 |

### EXAMPLE 9

Preparation of Compounds **Int-4a to Int-4d**

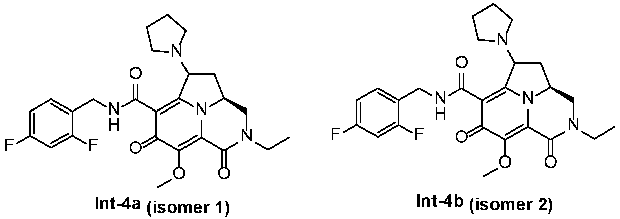

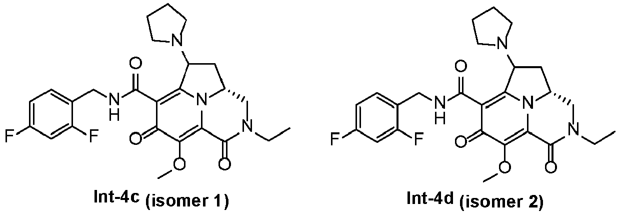

Compounds **Int-4a** and **Int-4b** were prepared from compound **Int-2d,** using the method described in Step E and Step F in Example 2, and replacing ammonia with pyrrolidine in Step E.

Compound **Int-4a: ¹H NMR** (400MHz, CDCl₃): δ 10.83 (brs, 1H), 7.29-7.42 (m, 1H), 6.71-6.86 (m, 2H), 5.54 (d, J = 5.1 Hz, 1H), 4.45-4.84 (m, 4H), 4.00-4.09 (m, 3H), 3.34-3.78 (m, 6H), 2.80 (brs, 2H), 2.54 (brs, 2H), 1.96 (d, J = 12.9 Hz, 2H), 1.21-1.24 (m, 3H). **MS:** *m*/*z* = 487.1 (M + 1).

Compound **Int-4b: ¹H NMR** (400MHz, CDCl₃): δ 7.31 (d, J = 6.7 Hz, 1H), 6.72-6.89 (m, 2H), 5.83 (brs, 1H), 4.44-4.82 (m, 6H), 4.03 (s, 3H), 3.24-3.68 (m, 6H), 3.09 (brs, 1H), 1.97-2.42 (m, 5H), 1.20-1.24 (m, 3H). MS: *m*/*z* = 487.1 (M + 1).

Compounds **Int-4c** and **Int-4d** were prepared from compound **Int-3d,** using the method described in Step E and Step F in Example 2, and replacing ammonia with pyrrolidine in Step E.

Compound **Int-4c: ¹H NMR** (400MHz, CDCl₃): δ 7.36 (brs, 1H), 6.82 (d, J = 8.4 Hz, 2H), 5.70 (brs, 1H), 4.68 (d, J = 16.1 Hz, 1H), 4.13-4.61 (m, 7H), 4.04 (s, 3H), 3.39-3.83 (m, 7H), 2.55-2.69 (m, 2H), 1.24 (t, J = 6.9 Hz, 3H). **MS:** *m*/*z* = 487.1 (M + 1).

Compound **Int-4d: ¹H NMR** (400MHz, CDCl₃): δ 7.29-7.43 (m, 1H), 6.73-6.84 (m, 2H), 5.53 (d, J = 5.3 Hz, 1H), 4.36-4.81 (m, 6H), 4.01-4.10 (m, 2H), 3.46-3.77 (m, 5H), 2.74 (brs, 2H), 2.44-2.54 (m, 2H), 1.85-2.02 (m, 2H), 1.18-1.30 (m, 3H). **MS:** *m*/*z*=487.1 (M + 1).

### EXAMPLE 10

### Preparation of Compound 33

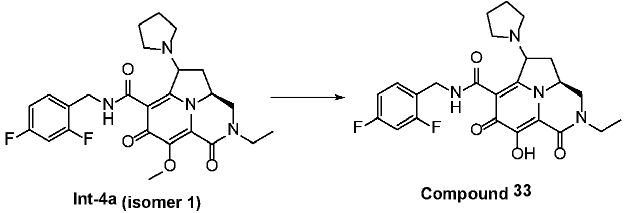

To a solution of compound **Int-4a** (20 mg, 0.041 mmol) in DMF (3 mL) was added lithium chloride (17.43 mg, 0.411 mmol). The resulting solution was heated at 80°C and allowed to stir at this temperature for 2 h under N₂ atmosphere. The crude reaction mixture was purified using preparative HPLC (Column: Phenomenex Synergi C18 150 ^{∗} 30 mm ^{∗} 4 um; Condition: 0.1% TFA-ACN; Gradient: 18% to 48%; B, 2~8 minutes; Flow Rate: 30 mL/min) to provide compound 33 as a single stereoisomer (white solid). **¹H NMR** (400MHz, CD₃OD): δ 11.05 (brs, 1H), 7.40-7.52 (m, 1H), 6.87-7.03 (m, 2H), 5.72 (d, J = 9.4 Hz, 1H), 4.94-5.05 (m, 1H), 4.56-4.74 (m, 2H), 3.93 (dd, J = 12.9, 3.9 Hz, 2H), 3.59-3.76 (m, 3H), 3.35 (d, J=15.3 Hz, 2H), 3.05 (brs, 1H), 2.89 (dd, J = 15.3, 6.7 Hz, 1H), 2.50 (dt, J = 15.4, 9.7 Hz, 1H), 2.15 (brs, 4H), 1.24 (t, J = 7.2 Hz, 3H). **MS:** *m*/*z* = 473.2 (M + 1).

### EXAMPLE 11

### Preparation of Compounds 34-36

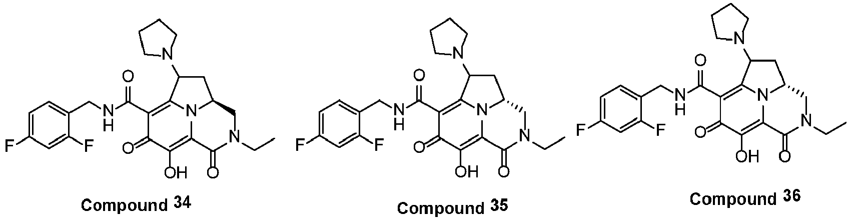

Using the method described in Example 10 and starting from compound **Int-4b**, compound **34** was prepared as a single stereoisomer.

Compound **34: ¹H NMR** (400MHz, CD₃OD): δ 11.59 (brs, 1H), 7.40-7.57 (m, 1H), 6.88-7.06 (m, 2H), 5.66 (brs, 1H), 4.50-4.61 (m, 3H), 3.55-4.02 (m, 5H), 2.96-3.24 (m, 4H), 2.00-2.35 (m, 5H), 1.25 (t, J = 7.2 Hz, 3H); **MS:** *m*/*z* = 473.2 (M + 1).

Using the method described in Example 10, and starting from compound **Int-4c**, compound **35** was prepared as a single stereoisomer.

Compound **35: ¹H NMR** (400MHz, CD₃OD): δ 11.61 (brs, 1H), 7.37-7.52 (m, 1H), 6.88-7.06 (m, 2H), 5.68 (t, J = 8.4 Hz, 1H), 4.58-4.75 (m, 3H), 3.81-4.02 (m, 3H), 3.51-3.75 (m, 2H), 3.00-3.24 (m, 4H), 2.02-2.34 (m, 5H), 1.25 (t, J = 7.0 Hz, 3H). **MS:** *m*/*z* = 473.2 (M + 1).

By employing the method described in Example 9, starting from compound **Int-4d,** compound **36** was prepared as a single stereoisomer.

Compound **36: ¹H NMR** (400MHz, CD₃OD): δ 11.05 (brs, 1H), 7.38-7.49 (m, 1H), 6.88-7.03 (m, 2H), 5.72 (d, J = 9.4 Hz, 1H), 4.97-5.07 (m, 1H), 4.56-4.74 (m, 2H), 3.57-3.96 (m, 5H), 3.37 (brs, 2H), 3.05 (brs, 1H), 2.89 (dd, J = 15.3, 6.7 Hz, 1H), 2.51 (dt, J = 15.5, 9.9 Hz, 1H), 2.16 (brs, 4H), 1.24 (t, J = 7.2 Hz, 3H). **MS:** *m*/*z* = 473.2 (M + 1).

### EXAMPLE 12

### Preparation of Compound Int-5

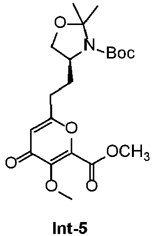

Compound **Int-5** was prepared using the method described in PCT International Patent Publication No. WO2014/183532.

### EXAMPLE 13

### Preparation of Compound 37

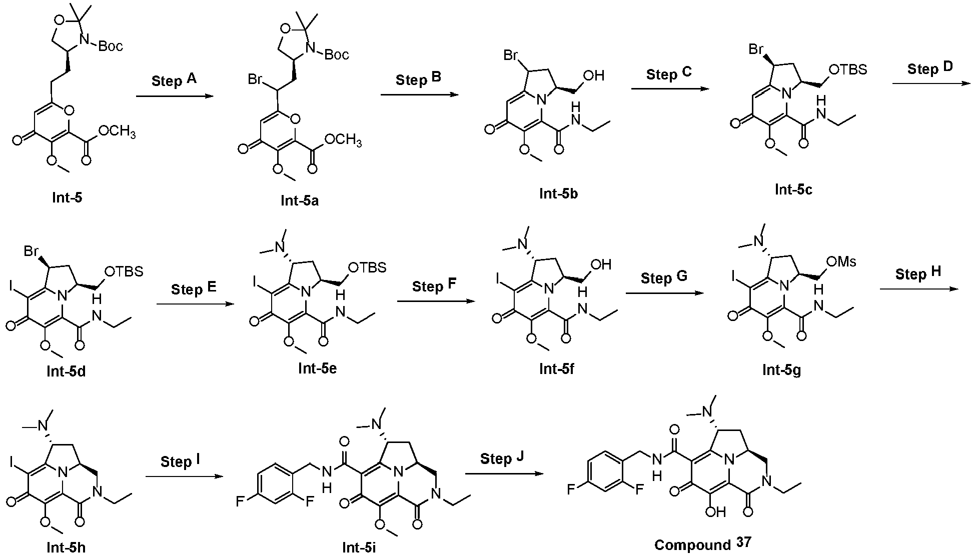

### Step A- Synthesis of Compound Int-5a

In a flame dried 1 L flask under nitrogen, compound **Int-5** (33.4 g, 81 mmol) was dissolved in THF (334 mL) and the solution was cooled to -70°C. Lithium bis(trimethylsilyl)amide (1 M solution in THF) (89 mL, 89 mmol) was then added and the reaction was allowed to stir at -75°C for 30 minutes. A solution of 1,3-dibromo-5,5-dimethylhydantoin (8.70 g, 30.4 mmol) in 125 mL of THF was slowly added, while keeping the internal temperature below -70°C. The reaction was then allowed to stir at -75°C for 10 minutes. The reaction was quenched with 40 mL MeOH, then 300 mL of sat NH₄Cl solution was then added, and the resulting mixture was extracted with 400 mL EtOAc. The organic layer was washed twice with 400 mL water, then once with 300 mL 20% NaCl solution, then dried over anhydrous MgSO₄, filtered and then concentrated *in vacuo.* The residue obtained was dissolved in THF (390 mL) and the resulting solution was treated with diethyl phosphite (3.53 mL, 27.3 mmol), then N-ethyl-N-isopropylpropan-2-amine (4.78 mL, 27.3 mmol) and stirred at room temperature for 25 minutes. Additional diethyl phosphite (0.88 mL) and DIPEA (1.17 mL) were added. The reaction was allowed to stir at room temperature for additional 15 minutes. The mixture was diluted with EtOAc (1L), poured into a separatory funnel and washed 4 times with 0.5 N HCl (500 mL), once with sat. aq. NaHCO₃ (500 mL) and once with brine (500 mL). The organic phase was dried over anhydrous MgSO₄, filtered and concentrated *in vacuo* to provide compound **Int-5a,** which was used without further purification. **MS:** *m*/*z* = 490 (M + 1).

### Step B- Synthesis of Compound Int-5b

A solution of crude compound **Int-5a** (90.3 g, 184 mmol) in DCM (913 mL) was treated with TFA (141 mL, 1842 mmol) at room temperature and stirred overnight. The mixture was concentrated *in vacuo* to near dryness, then azeotroped 3 times with 300 mL EtOH. The residue was dissolved in EtOH (913 mL) and heated to 75°C for 45 minutes. The mixture was cooled to 4°C with an ice bath. Ethanamine (70% in water) (95 g, 1473 mmol) was added to the mixture slowly, keeping the internal temperature below 11°C. The mixture was stirred in the cold bath for 10 min upon completion of amine addition. The mixture was concentrated *in vacuo* and the resulting residue was azeotroped with ACN (2 × 400 mL) to provide compound **Int-5b,** which was used without further purification. **MS:** *m*/*z* = 345 (M + 1).

### Step C- Synthesis of Compound Int-5c

In a 2 L flask under nitrogen, the crude compound **Int-5b** (113 g, 184 mmol) was dissolved in 1.1 L of dichloromethane. To this was added imidazole (39.7 g, 583 mmol) and TBSCl (83.6 g, 554 mmol). It was allowed to stir at room temperature for 20 minutes. Additional imidazole (13.3 g, 195 mmol) and TBSCl (27.9 g, 185 mmol) was added. The reaction was allowed to stir for additional 20 minutes. The crude solution was washed twice with 1100 mL water. The aqueous layer was back extracted with 400 mL of dichloromethane. The combined organics were dried over anhydrous MgSO₄, filtered and concentrated *in vacuo.* The residue obtained was coevaporated three times with 400 mL toluene to remove TBSOH. The residue was purified by a silica gel column (3 kg) eluting with heptane/EtOAc:EtOH (3:1 v/v) gradient 0 to 80% to provide compound **Int-5c**. **MS:** *m*/*z* = 459 (M + 1).

### Step D- Synthesis of Compound Int-5d

To a solution of compound **Int-5c** (1.0 g, 2.177 mmol) in MeOH (10 mL) was added m-CPBA (0.751 g, 4.35 mmol) and NIS (1.224 g, 4.35 mmol). The reaction was allowed to stir at 75°C for 2 hours. The mixture was concentrated *in vacuo* and then quenched with aq. Na₂SO₃ (30 mL), 10% aq. NaOH (20 mL) and the aqueous layer was extracted with dichloromethane (50 mL × 3). The organic phase was dried over anhydrous Na₂SO₄, filtered, and concentrated *in vacuo.* The residue was purified using a silica gel column eluting with 5% MeOH in dichloromethane to provide compound **Int-5d** as a solid. **¹H NMR** (400 MHz, CDCl₃) δ 5.26-5.38 (m, 1H), 4.01-4.10 (m, 2H), 3.83 (s, 3H), 3.54-3.65 (m, 1H), 3.36-3.50 (m, 1H), 3.16-3.29 (m, 1H), 2.85-2.93 (m, 1H), 1.32 (s, 3H), 0.90 (s, 9H), 0.09 (s, 6H). **MS:** *m*/*z* = 585.2 (M + 1).

### Step E-Synthesis of Compound Int-5e

To a solution of compound **Int-5d** (600 mg, 1.025 mmol) in EtOH (10 mL) was added dimethylamine (1400 mg, 10.25 mmol, 33% in water) and the mixture was allowed to stir at 80°C for 2 hours. The reaction was concentrated *in vacuo* and the residue was purified using a preparative TLC plate eluting with 5% MeOH in dichloromethane to provide compound **Int-5e** as a solid. **¹H NMR** (400 MHz, CDCl₃) δ5.48-5.62 (m, 1H), 4.41 (dd, J = 6.17, 8.82 Hz, 1H), 3.98 (s, 3H), 3.39-3.69 (m, 5H), 2.39-2.53 (m, 1H), 2.20 (s, 6H), 2.06 (brs, 1H), 1.25-1.29 (m, 3H), 0.76 (s, 9H), -0.17-0.05 (m, 6H). **MS:** *m*/*z* = 550.2 (M + 1).

### Step F- Synthesis of Compound Int-5f

To a solution of compound **Int-5e** (300 mg, 0.546 mmol) in dioxane (20 mL) was added HCl (0.546 mL, 2.184 mmol, 4M in dioxane) and the mixture was allowed to stir at 20°C for 2 hours. The mixture was filtered to provide compound **Int-5f** as a solid, which was used without further purification **¹H NMR** (400 MHz, CDCl₃) δ 5.46-5.49 (m, 1H), 5.14-5.21 (m, 1H), 3.80-3.94 (m, 4H), 3.61-3.67 (m, 1H), 3.42 (dd, J = 7.39, 8.71 Hz, 2H), 3.25-3.34 (m, 6H), 2.91 (ddd, J = 5.62, 8.43, 14.39 Hz, 2H), 2.75 (dd, J = 4.08, 8.93 Hz, 1H), 1.24 (t, J = 7.28 Hz, 3H). **MS:** *mlz* = 435.2 (M + 1).

### Step G- Synthesis of Compound Int-5g

To a solution of compound **Int-5f** (200 mg, 0.459 mmol) in dichloromethane (10 mL) was added *N*-ethyl-*N*-isopropylpropan-2-amine (297 mg, 2.297 mmol) and methanesulfonyl chloride (158 mg, 1.378 mmol) at 0°C. The mixture was allowed to stir at 20°C for 2 hours. The reaction was quenched with MeOH (5 mL), then concentrated *in vacuo* and the residue obtained was purified using a silica gel column eluting with 5% MeOH in dichloromethane to provide compound **Int-5g** as a solid. **MS:** *m*/*z* = 514.1 (M + 1).

### Step H- Synthesis of Compound Int-5h

A mixture of Cs₂CO₃ (381 mg, 1.169 mmol) in DMF (30 mL) was allowed to stir at 80°C for 30 min, then a solution of compound **Int-5g** (200 mg, 0.390 mmol) in DMF (10 mL) was added. The mixture was allowed to stir at 80°C for 1 hour. The mixture was filtered. The filtrate was concentrated *in vacuo* and the residue obtained was purified using a preparative TLC plate eluting with 5% MeOH in dichloromethane to provide compound **Int-5h** as a solid. **¹H NMR** (400 MHz, CDCl₃) δ 4.57-4.70 (m, 1H), 4.27-4.34 (m, 1H), 4.05 (s, 3H), 3.52-3.62 (m, 4H), 2.53-2.64 (m, 1H), 2.30 (s, 6H), 1.79-1.89 (m, 1H), 1.21 (t, J = 7.17 Hz, 3H). **MS:** *m*/*z* = 418.1 (M + 1).

### Step I- Synthesis of Compound Int-5i

To a solution of compound **Int-5h** (50 mg, 0.120 mmol) in DMSO (5 mL) under a CO balloon was added 2,4-difluorobenzylamine (51.5 mg, 0.360 mmol), *N*-ethyl-*N*-isopropylpropan-2-amine (77 mg, 0.599 mmol) and Pd(Ph₃P)₄ (27.7 mg, 0.024 mmol). The mixture was allowed to stir at 80°C under a CO balloon for 2 hours. The mixture was poured into EtOAc(30 mL), filtered, concentrated *in vacuo* and the residue obtained was purified using a preparative TLC plate eluting with 5% MeOH in dichloromethane to provide compound **Int-5i** as a solid. **¹H NMR** (400 MHz, MeOD) δ 7.45-7.51 (m, 1H), 6.93-6.95 (m, 2H), 4.77-4.78 (m, 1H), 4.60 (s, 2H), 3.80 (s, 3H), 3.76-3.78 (m, 1H), 3.59-3.62 (m, 3H), 3.17-3.19 (m, 1H), 2.63-2.67 (m, 1H), 2.23 (s, 6H), 1.91-1.97 (m, 1H), 1.93-1.21 (m, 3H). **MS:** *m*/*z* = 461.1 (M + 1).

### Step J- Synthesis of Compound 37

To a solution of compound **Int-5i** (30 mg, 0.065 mmol) in DMF (5 mL) was added lithium chloride (27.6 mg, 0.652 mmol). The resulting solution was heated at 80°C under N₂ for 8 hours. The crude reaction mixture was cooled to room temperature and purified by a prep-HPLC (Column: Phenomenex Synergi C18 150 ^{∗} 30 mm ^{∗} 4 um; Condition: 0.1% TFA-ACN; Gradient: 16% to 46%; B, 2 - 8 minutes. Flow Rate: 30 mL/min) to provide compound **37** as a solid. **¹H NMR** (400 MHz, CDCl₃) δ 11.00 (brs, 1H), 7.41-7.46 (m, 1H), 6.91-6.98 (m, 2H), 5.55 (d, J = 9.26 Hz, 1H), 4.92-4.98 (m, 1H), 4.64-4.70 (m, 2H), 3.92-3.96 (m, 1H), 3.60-3.71 (m, 3H), 3.06-3.18 (m, 3H), 2.95-3.02 (m, 1H), 2.73 (brs, 3H), 2.43-2.49 (m, 1H), 1.22-1.26 (m, 3H). **MS:** *m*/*z* = 447.1 (M + 1).

### EXAMPLE 14

### Preparation of Compound 38 and Compound 39

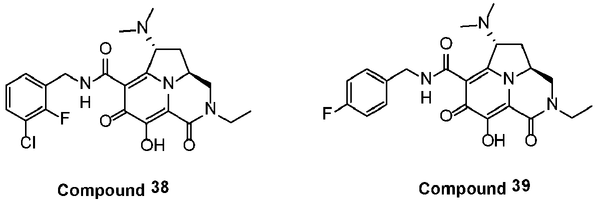

Compound **38** was made from compound **Int-5h,** using the methods described in Step I and Step J of Example 13, and replacing 2,4-difluorobenzylamine with 3-chloro-2-fluorobenzylamine in Step I.

Compound **38: ¹H NMR** (400 MHz, CD₃OD) δ 11.05 (brs, 1H), 7.39 (s, 2H), 7.10-7.14 (m, 1H), 5.55 (d, J = 9.26 Hz, 1H), 4.93-4.98 (m, 1H), 4.68-4.75 (m, 2H), 3.91-3.96 (m, 1H), 3.60-3.72 (m, 3H), 3.05-3.22 (m, 3H), 2.98-3.03 (m, 1H), 2.61-2.87 (m, 3H), 2.43-2.49 (m, 1H), 1.22-1.26 (m, 3H). **MS:** *m*/*z* = 473.1 (M + 1).

Compound **39** was made from compound **Int-5h,** using the methods described in Step I and Step J of Example 13, and replacing 2,4-difluorobenzylamine with 4-fluorobenzylamine in Step I.

Compound **39: ¹H NMR** (400 MHz, CD₃OD) δ 10.98 (brs, 1H), 7.36-7.39 (m, 2H), 7.03-7.07 (m, 2H), 5.55 (d, J=9.26 Hz, 1H), 4.93-4.98 (m, 1H), 4.62 (brs, 2H), 3.92-3.96 (m, 1H), 3.59-3.71 (m, 3H), 3.06-3.22 (m, 3H), 2.98-3.03 (m, 1H), 2.56-2.90 (m, 3H), 2.43-2.50 (m, 1H), 1.24 (s, 3H). **MS:** *m*/*z* = 429.1 (M + 1).

### EXAMPLE 15

### Preparation of Compound Int-6

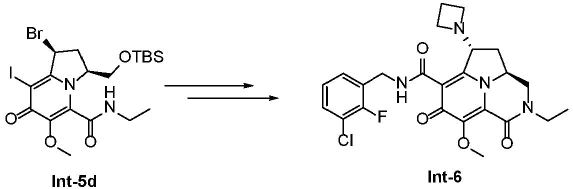

Using the methods described in Steps E through J of Example 12, starting from compound **Int-5d,** compound **Int-6** was prepared as a single stereoisomer. Dimethylamine was replaced with azetidine in Step E and 2,4-difluorobenzylamine was replaced with 3-chloro-2-fluorobenzylamine in Step I.

Compound **Int-6: ¹H NMR** (400 MHz, CDCl₃): δ 11.07 (s, 1H), 7.23-7.33 (m, 2H), 7.03-7.06 (m, 1H), 6.11-6.12 (d, *J=* 4.8 Hz, 1H), 5.02 (s, 1H), 4.56-4.65 (m, 3H), 4.02-4.06 (m, 5H), 3.62-3.69 (m, 2H), 3.49-3.56 (m, 2H), 2.76-2.78 (*d, J* = 11.2 Hz, 1H), 2.45 (s, 2H), 2.21 (s, 1H), 1.17-1.21 (m, 3H). MS (M+H)⁺: 489.2.

### EXAMPLE 16

### Preparation of Compound 40 and Compound 41

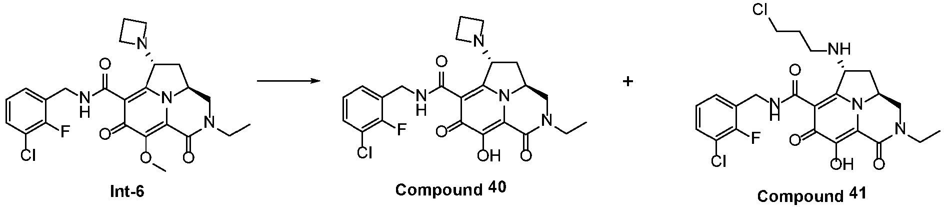

To a solution of compound **Int-6** (30 mg, 0.061 mmol) in 2 mL of DMF was added lithium chloride (26.0 mg, 0.614 mmol). The resulting solution was heated to 78°C for 12 h under N₂. The mixture was cooled to room temperature and purified by a prep-HPLC using (Column: Phenomenex Synergi C18 150 ^{∗} 30 mm ^{∗} 4 um; Condition: 0.1 % TFA-ACN; Gradient: 25 % to 55 %; B, 0~8 minutes; Flow Rate: 30 mL/min) to provide compound **40** as a solid and compound **41** as a solid.

Compound **40: ¹H NMR** (400 MHz, CDCl₃) δ 10.94 (s, 1H), 7.25-7.34 (m, 2H), 7.03-7.07 (m, 1H), 6.01-6.03 (d, *J* = 6.8 Hz, 1H), 5.08-5.11 (m, 1H), 4.66-4.68 (d, *J* = 5.2 Hz, 2H), 4.51-4.55 (m, 2H), 3.97 (m, 2H), 3.60-3.72 (m, 4H), 2.71-2.75 (m, 1H), 2.40 (s, 2H), 2.23 (m, 1H), 1.23-1.26 (m, 3H). **MS** (M+H)⁺: 475.1.

Compound **41: ¹H NMR** (400 MHz, CDCl₃) δ 11.02 (s, 1H), 7.28-7.37 (m, 2H), 7.06-7.10 (m, 1H), 5.06-5.08 (d, *J=* 8.4 Hz, 2H), 4.61-4.66 (m, 2H), 3.78-3.79 (d, *J=* 7.2 Hz, 2H), 3.59-3.70 (m, 4H), 3.46 (m, 1H), 3.18-3.32 (m, 2H), 2.29-2.38 (m, 3H), 1.21-1.29 (m, 3H). **MS** (M+H)⁺: 511.2.

### EXAMPLE 17

### Preparation of Compound 42

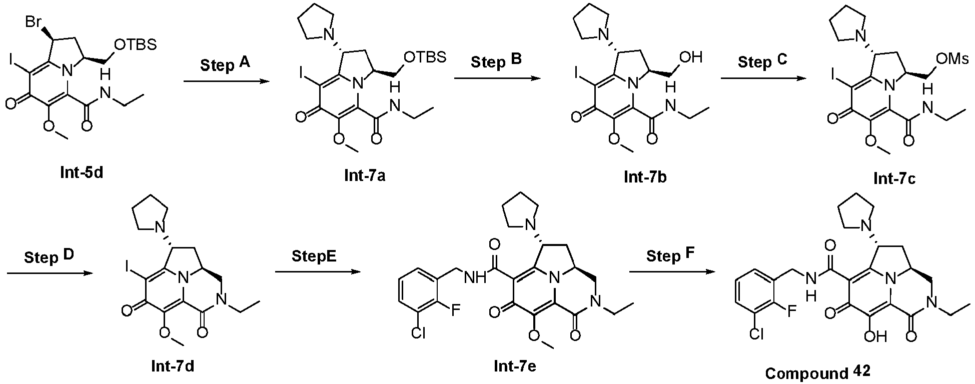

### Step A- Synthesis of Compound Int-7a

To a solution of compound **Int-5d** (300 mg, 0.513 mmol) in EtOH (1 mL) was added pyrrolidine (365 mg, 5.13 mmol) and the mixture was allowed to stir at 80°C for 40 minutes. The mixture was cooled to room temperature. The solvent was removed under vacuum. The residue was purified using a silica gel column eluting with 5% MeOH in dichloromethane to provide compound **Int-7a** as a solid. **MS:** *m*/*z* = 576 (M + 1).

### Step B- Synthesis of Compound Int-7b

To a solution of compound **Int-7a** (40 mg, 0.069 mmol) in 1,4-dioxane (1 mL) was added HCl/dioxane (0.069 mL, 0.278 mmol, 4M) and the mixture was allowed to stir at 20°C for 5 hours. The mixture was concentrated *in vacuo* to provide compound **Int-7b** as a solid, which was used without purification. **MS:** *m*/*z* = 462 (M + 1).

### Step C- Synthesis of Compound Int-7c

To a mixture of compound **Int-7b** (140 mg, 0.303 mmol) in dichloromethane (4 mL) was added triethylamine (0.212 mL, 1.517 mmol) and MsCl (0.035 mL, 0.455 mmol). The reaction was allowed to stir at 20°C for 2 hours. The mixture was concentrated *in vacuo* and purified using a preparative TLC plate eluting with 5% MeOH in dichloromethane to provide compound **Int-7c** as a solid. **MS:** *m*/*z* = 540 (M + 1).

### Step D- Synthesis of Compound Int-7d

A solution of Cs₂CO₃ (199 mg, 0.612 mmol) in DMF (3 mL) was allowed to stir at 80°C for 30 minutes. A solution of compound **Int-7c** (110 mg, 0.204 mmol) in DMF (3 mL) was then added. The mixture was allowed to stir at 80°C for 1 hour. The mixture was cooled to room temperature. It was then filtered. The filtrate was concentrated *in vacuo* and purified using a preparative TLC plate eluting with 7% MeOH in dichloromethane to provide compound **Int-7d** as a solid. **MS:** *m*/*z* = 444 (M + 1).

### Step E- Synthesis of Compound Int-7e

To a solution of compound **Int-7d** (20 mg, 0.045 mmol) in DMSO (3 mL) was added diisopropylethylamine (0.039 mL, 0.226 mmol), (3-chloro-2-fluorophenyl)methanamine (21.60 mg, 0.135 mmol) and Pd(Ph₃P)₄ (10.43 mg, 9.02 µmol). The mixture was allowed to stir at 80°C for 2 h under a CO balloon. The mixture was cooled to room temperature and filtered. The filtrate was purified using preparative HPLC (Column: YMC-Actus Pro C18 150 ^{∗} 30 mm ^{∗} 5 um; Condition: 0.1% TFA-ACN; Gradient: 20% to 50%; B, 0~11 minutes; Flow Rate: 40 mL/min) to provide compound **Int-7e** as a solid. **¹H NMR** (400MHz, CD₃Cl) δ 11.12 (t, J = 5.6 Hz, 1H), 7.30-7.33 (m, 1H), 7.21-7.23 (m, 1H), 7.02-7.06 (m, 1H), 6.05 (d, J = 6.8 Hz, 1H), 5.19-5.24 (m, 1H), 4.64-4.70 (m, 2H), 4.08 (s, 3H), 3.64-3.78 (m, 3H), 3.44-3.53 (m, 2H), 3.15-3.32 (m, 3H), 2.02-2.24 (m, 6H), 1.22 (t, J = 7.1 Hz, 3H). **MS:** *m*/*z* = 503.3 (M + 1).

### Step F- Synthesis of Compound 42

To a solution of compound **Int-7e** (20 mg, 0.040 mmol) in DMF (3 mL) was added lithium chloride (16.86 mg, 0.398 mmol). The mixture was allowed to stir at 80°C for 2 hours. The mixture was cooled to room temperature and filtered. The filtrate was purified using preparative HPLC (Column: YMC-Actus Pro C18 150 ^{∗} 30 mm ^{∗} 5 um; Condition: 0.1% TFA-ACN; Gradient: 20% to 50%; B, 0∼11 minutes; Flow Rate: 40 mL/min) to provide compound **42** as a solid. **¹H NMR** (400MHz, CD₃OD) δ 11.12 (s, 1H), 7.34-7.43 (m, 2H), 7.12-7.16 (m, 1H), 5.73 (d, J = 9.2 Hz, 1H), 5.0-5.03 (m, 1H), 4.71-4.80 (m, 2H), 3.72-3.95 (m, 2H), 3.63-3.68 (m, 3H), 3.31-3.35 (m, 2H), 2.88-2.91 (m, 2H), 2.49-2.53 (m, 1H), 2.03-2.15 (m, 4H), 1.25 (t, J = 7.2 Hz, 3H). **MS:** *m*/*z* = 489.1 (M + 1).

### EXAMPLE 18

### Preparation of Compound 43

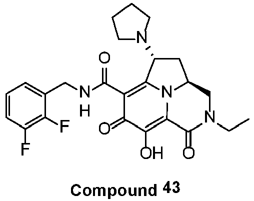

Starting from compound **Int-7d,** by using the method described in Step E and Step F in Example 17, compound **43** was prepared as a single stereoisomer, except replacing 3-chloro-2-fluorobenzylamine in Step E with 2,3-difluorobenzylamine.

Compound **43**: **¹H NMR** (400 MHz, CDCl₃) δ 11.03 (brs, 1H), 6.92-7.19 (m, 3H), 5.95 (d, *J* = 6.6 Hz, 1H), 5.23-5.40 (m, 1H), 4.65-4.66 (m, 2H), 3.76-3.84 (m, 1H), 3.57-3.68 (m, 4H), 3.17-3.30 (m, 3H), 2.26-2.35 (m, 1H), 2.07-2.10 (m, 5H), 1.20-1.30 (m, 3H). **MS:** *m*/*z* = 473.2 (M + 1).

### EXAMPLE 19

### Preparation of Compound 44 and Compound 45

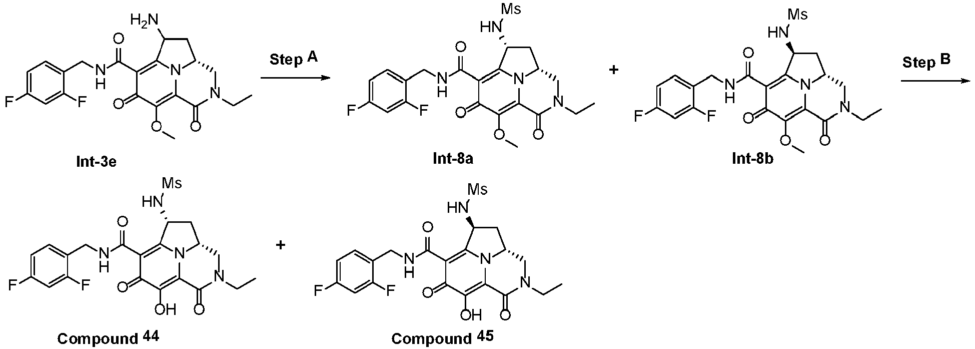

### Step A- Synthesis of Compound Int-8a and Compound Int-8b

To a solution of compound **Int-3e** (60 mg, 0.139 mmol) in CH₂Cl₂ (5 mL) was added triethylamine (0.058 mL, 0.416 mmol) and methanesulfonyl chloride (31.8 mg, 0.278 mmol) at 0°C, and the mixture was allowed to stir at 18°C for 1 hour. The reaction was quenched with water (2 mL) and the aqueous was extracted with CH₂Cl₂ (10 mL × 3). The combined organics were washed with brine (10 mL), dried over anhydrous Na₂SO₄, filtered and evaporated. The crude product was purified using a preparative TLC plate eluting with 10% MeOH in dichloromethane to provide the product as a mixture of stereoisomers, which was further separated by a chiral preparative SFC ("Column: YMC-Actus Pro C18 150 ^{∗} 30 5um; Condition: 0.1% TFA-ACN; Gradient: 20% to 50%; B, 1.1∼11 minutes; Flow Rate: 40 mL/min) to provide compound **Int-8a** (the first eluting isomer) as an oil and compound **Int-8b** (the second eluting isomer) as an oil.

Compound **Int-8a: ¹H NMR** (400 MHz, CDCl₃): δ 7.27-7.39 (m, 1H), 6.71-6.88 (m, 2H), 5.29-5.44 (m, 1H), 4.50-4.70 (m, 2H), 4.36 (d, J = 8.2 Hz, 1H), 4.02 (s, 3H), 3.84 (t, J = 12.1 Hz, 1H), 3.54-3.69 (m, 3H), 2.92-3.07 (m, 4H), 2.53 (dt, J = 13.4, 8.8 Hz, 1H), 1.17-1.24 (m, 3H). MS: *m*/*z* = 511.2 (M + 1).

Compound **Int-8b: ¹H NMR** (400 MHz, CDCl₃): δ 10.84 (brs, 1H), 7.31 (d, J = 7.0 Hz, 1H), 6.80 (d, J = 8.6 Hz, 2H), 5.78 (brs, 1H), 5.60 (d, J = 7.0 Hz, 1H), 4.88 (brs, 1H), 4.59 (brs, 2H), 4.01 (brs, 3H), 3.51-3.65 (m, 4H), 3.10 (brs, 4H), 2.03 (brs, 2H), 1.24-1.33 (m, 3H). **MS:** *m*/*z* = 511.2 (M + 1).

### Step B- Synthesis of Compound 44 and Compound 45

To a solution of compound **Int-8a** (42 mg, 0.082 mmol)) in CH₃CN (5 mL) was added magnesium bromide (151 mg, 0.823 mmol) and the resulting mixture was allowed to stir at 18°C for 2 hours. The mixture was filtered and the filtrate was purified by a prep-HPLC (Column: YMC-Actus Pro C18 150 ^{∗} 30 5um; Condition: 0.1% TFA-ACN; Gradient: 20% to 50%; B, 1.1 ~ 11 minutes; Flow Rate: 40 mL/min) to provide compound **44** as a red solid. **¹H NMR** (400 MHz, CDCl₃): δ 11.12 (brs, 1H), 7.28-7.41 (m, 2H), 6.72-6.87 (m, 2H), 5.28 (brs, 1H), 4.60 (d, J = 4.7 Hz, 2H), 4.38 (d, J = 7.8 Hz, 1H), 3.83 (t, J = 12.1 Hz, 1H), 3.63-3.73 (m, 2H), 3.49-3.59 (m, 1H), 2.93-3.06 (m, 4H), 2.56 (d, J = 4.7 Hz, 1H), 1.25 (t, J = 7.0 Hz, 3H). **MS:** *m*/*z*=497.2 (M + 1).

To a solution of compound **Int-8b** (5 mg, 9.79 µmol)) in CH₃CN (5 mL) was added magnesium bromide (18.03 mg, 0.098 mmol) and stirred at 18 °C for 2 hours. The mixture was filtered and the filtrate was purified by a prep-HPLC (Column: YMC-Actus Pro C18 150 ^{∗} 30 5um; Condition: 0.1% TFA-ACN; Gradient: 20% to 50%; B, 1.1 ~ 11 minutes; Flow Rate: 40 mL/min) to compound **45** as a solid. **¹H NMR** (400 MHz, CDCl₃): δ 10.81 (brs, 1H), 7.28-7.38 (m, 1H), 6.75-6.88 (m, 2H), 5.80 (brs, 1H), 5.61 (brs, 1H), 4.88 (brs, 1H), 4.57 (d, J = 5.5 Hz, 2H), 3.55-3.74 (m, 4H), 3.02-3.15 (m, 4H), 2.11 (brs, 1H), 1.25 (t, J = 7.2 Hz, 3H). **MS:** *m*/*z* = 497.2 (M + 1).

### EXAMPLE 20

### Preparation of Compound 46-53

Starting from compound **Int-1a** and **Int-lb,** and using the method described in Example **19,** the following compounds were prepared.

| Compound # | Structure | Rt (min) | MS (M+H)⁺ |
|---|---|---|---|
| **46** | 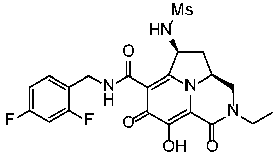 | 2.55 (LC5) | 497.1 |
| **47** | 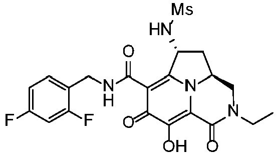 | 2.56 (LC5) | 497.1 |
| **48** | 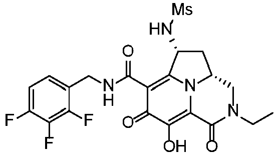 | 2.69 (LC5) | 515.0 |
| **49** | 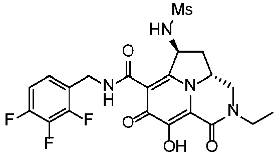 | 2.58 (LC5) | 515.0 |
| **50** | 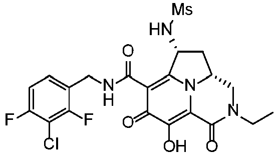 | 2.82 (LC5) | 530.9 |
| **51** | 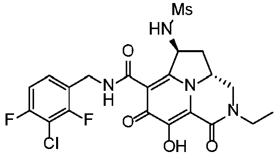 | 2.68 (LC5) | 531.1 |
| **52** | 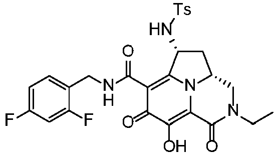 | 3.08(LC5) | 573.2 |
| **53** | 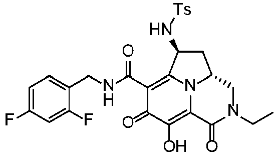 | 3.07(LC5) | 573.2 |

### EXAMPLE 21

### Preparation of Compound 54 and Compound 55

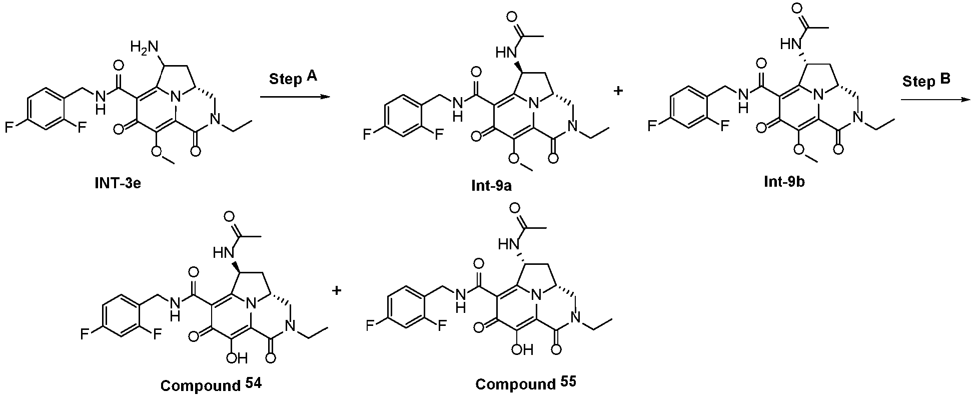

### Step A- Synthesis of Compound Int-9a and Compound Int-9b

To a solution of compound **Int-3e** (65 mg, 0.150 mmol) in 3 mL of dichloromethane was added triethylamine (0.063 mL, 0.451 mmol) and acetyl chloride (23.60 mg, 0.301 mmol) at 0°C. The reaction mixture was allowed to stir at 0°C for 0.5 hours. The mixture was purified using a preparative TLC plate eluting with 5% MeOH in dichloromethane to provide the product was a mixture of stereoisomers, which was further separated by a chiral preparative SFC (Column: AD (250 mm^{∗}30 mm, I.D., 10 um) Mobile phase: Supercritical CO₂/IPA-base = 65/35, Flow rate: 80 mL/min Wavelength: 220 nm) to provide compound **Int-9a** (the first eluting compound) as an oil and compound **Int-9b** (the second eluting compound) as a colorless oil.

Compound **Int-9a: ¹H NMR** (400 MHz, CDCl₃) δ 11.18 (brs, 1H), 7.28-7.38 (m, 1H), 7.21 (d, J = 5.8 Hz, 1H), 6.76-6.89 (m, 2H), 5.54 (dd, J = 6.1, 10.0 Hz, 1H), 5.31-5.38 (m, 1H), 4.60-4.70 (m, 1H), 4.49-4.58 (m, 1H), 4.04 (s, 3H), 3.62-3.73 (m, 2H), 3.51-3.58 (m, 2H), 2.68 (dd, J = 6.9, 13.5 Hz, 1H), 2.12-2.22 (m, 1H), 1.93 (s, 3H), 1.22-1.26 (m, 3H). **MS:** *m*/*z* = 475.2 (M + 1).

Compound **Int-9b: ¹H NMR** (400 MHz, CDCl₃) δ 10.74 (brs, 1H), 7.39 (brs, 1H), 7.28-7.36 (m, 1H), 6.79 (q, J = 7.96 Hz, 2H), 5.48-5.57 (m, 1H), 4.54-4.62 (m, 1H), 4.46-4.53 (m, 1H), 4.31-4.43 (m, 1H), 4.12 (t, J = 12.3 Hz, 1H), 3.95 (s, 3H), 3.58 (tt, J = 6.8, 13.4 Hz, 2H), 3.49 (d, J = 3.5 Hz, 1H), 2.82 (td, J = 9.4, 12.9 Hz, 1H), 2.38-2.49 (m, 1H), 1.83 (s, 3H), 1.21 (t, J = 7.0 Hz, 3H). **MS:** *m*/*z* = 475.2 (M + 1).

### Step B- Synthesis of Compound 54 and Compound 55

To a solution of compound **Int-9a** (10 mg, 0.021 mmol) in 2 mL of DMF was added lithium chloride (8.94 mg, 0.211 mmol). The resulting solution was heated to 80°C for 6 h under N₂. The mixture was purified by prep-HPLC using (Column: YMC-Actus Pro C18 150 ^{∗} 30 mm ^{∗} 5 um; Condition: 0.1% TFA-ACN; Gradient: 19% to 49%; B, 0~11 minutes; Flow Rate: 40 mL/min) to provide compound **54** as a solid. **¹H NMR** (400 MHz, CDCl₃) δ 11.07 (brs, 1H), 7.29-7.40 (m, 1H), 7.10 (d, *J=* 5.5 Hz, 1H), 6.71-6.93 (m, 2H), 5.52 (dd, *J* = 6.6, 9.4 Hz, 1H), 5.33 (brs, 1H), 4.55-4.67 (m, 2H), 3.51-3.81 (m, 4H), 2.66 (dd, *J* = 6.6, 13.3 Hz, 1H), 2.13-2.27 (m, 1H), 1.90 (s, 3H), 1.27 (t, *J=* 7.0 Hz, 3H). **MS:** *m*/*z* = 461.0 (M + 1).

To a solution of compound **Int-9b** (40 mg, 0.084 mmol) in 2 mL of DMF was added lithium chloride (35.7 mg, 0.843 mmol). The resulting solution was heated to 80°C for 16 h under N₂. The mixture was purified by prep-HPLC using (Column: YMC-Actus Pro C18 150 ^{∗} 30 mm ^{∗} 5 um; Condition: 0.1% TFA-ACN; Gradient: 19% to 49%; B, 0~11 minutes; Flow Rate: 40 mL/min) to provide compound **55** as a solid. **¹H NMR** (400 MHz, CDCl₃) δ 10.91 (brs, 1H), 7.28-7.40 (m, 1H), 7.13 (d, *J=* 4.3 Hz, 1H), 6.74-6.88 (m, 2H), 5.38 (brs, 1H), 4.52-4.68 (m, 2H), 4.43 (d, *J* = 8.2 Hz, 1H), 4.13 (t, *J* = 12.1 Hz, 1H), 3.71 (qd, *J* = 7.1, 13.8 Hz, 1H), 3.47-3.63 (m, 2H), 2.73-2.88 (m, 1H), 2.52-2.68 (m, 1H), 1.82 (s, 3H), 1.26 (t, *J=* 7.2 Hz, 3H). **MS:** *m*/*z* = 461.0 (M +1).

### EXAMPLE 22

### Preparation of Compound 56 and Compound 57

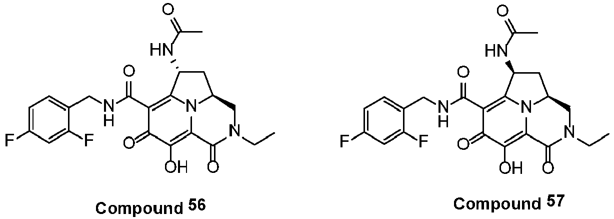

Starting from compound **Int-2e**, and using the method described in Example 21, compounds **56** and **57** were prepared.

Compound **56: ¹H NMR** (400 MHz, CDCl₃) δ 11.10 (brs, 1H), 7.29-7.38 (m, 1H), 7.08 (d, J = 5.1 Hz, 1H), 6.82 (d, J = 3.5 Hz, 2H), 5.47-5.57 (m, 1H), 5.35 (brs, 1H), 4.54-4.70 (m, 2H), 3.54-3.77 (m, 4H), 2.67 (dd, J = 6.6, 13.3 Hz, 1H), 2.12-2.27 (m, 1H), 1.90 (s, 3H), 1.27 (t, J = 7.0Hz, 3H). **MS:** m/z = 461.1 (M + 1).

Compound 57: **¹H NMR** (400 MHz, CDCl₃) δ 10.91 (brs, 1H), 7.29-7.38 (m, 1H), 7.25 (brs, 1H), 6.76-6.87 (m, 2H), 5.38 (brs, 1H), 4.51-4.69 (m, 2H), 4.45 (d, J = 7.4 Hz, 1H), 4.11 (t, J = 11.9 Hz, 1H), 3.70 (qd, J = 7.1, 14.0 Hz, 1H), 3.47-3.63 (m, 2H), 2.81 (d, J = 8.6 Hz, 1H), 2.57 (d, J = 5.9 Hz, 1H), 1.83 (s, 3H), 1.26 (t, J = 7.0 Hz, 3H). MS: *m*/*z* = 461.1 (M + 1).

### EXAMPLE 23

### Preparation of Compound 58

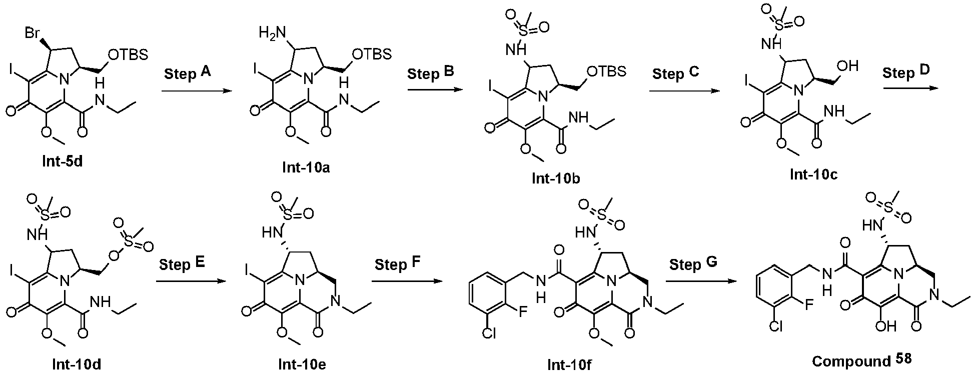

### Step A- Synthesis of Compound Int-10a

A mixture of compound **Int-5d** (500 mg, 0.854 mmol) in NH₃/MeOH (6 mL, 7 N) was allowed to stir at 35°C for 5.5 hours. The reaction was concentrated *in vacuo* to provide crude compound **Int-10a** as a solid, which was used without further purification. **MS:** *m*/*z* = 522 (M + 1).

### Step B- Synthesis of Compound Int-10b

To a solution of compound **Int-10a** (3.0 g, 3.84 mmol) in 20 mL of dichloromethane was added triethylamine (5.35 mL, 38.4 mmol) and methanesulfonyl chloride (3.23 g, 19.47 mmol) at 0°C. The mixture was allowed to stir at 20°C for 1.5 hours. The reaction was quenched with 5 mL of MeOH, and concentrated *in vacuo.* The residue was purified using a silica gel column (12 g) eluting with 5 % MeOH in dichloromethane to provide compound **Int-10b** as a solid. **MS:** *m*/*z* = 600.2 (M + 1).

### Step C- Synthesis of Compound Int-10c

To a solution of compound **Int-10b** (100 mg, 0.167 mmol) in 5 mL of THF was added TBAF (0.250 mL, 0.250 mmol) at 0°C. The mixture was allowed to stir at 20°C for 1.5 hours. The solvent was removed under vacuum, and the residue was purified using a preparative HPLC (Column: Waters XSELECT C18 150 ^{∗} 30 mm ^{∗} 5 um; Condition: 0.1 % TFA-ACN; Gradient: 0 % to 28 %; B, 0∼11 minutes; Flow Rate: 25 mL/min) to provide compound **Int-10c** as a solid. **¹H NMR** (400 MHz, CDCl₃): δ 7.92 (t, J = 5.8 Hz, 1H), 5.16-5.32 (m, 3H), 4.83 (brs, 4H), 4.12 (d, J = 13.2 Hz, 1H), 3.70 (s, 3H), 3.14-3.18 (m, 3H), 3.07 (dd, J = 13.8, 9.5 Hz, 1H), 3.44-3.57 (m, 1H), 1.32-1.36 (m, 3H). **MS:** *m*/*z* = 485.5 (M + 1).

### Step D- Synthesis of Compound Int-10d

To a solution of compound **Int-10c** (30 mg, 0.062 mmol) in 3 mL of dichloromethane was added TEA (0.026 mL, 0.185 mmol) and MsCl (7.23 µL, 0.093 mmol) at 0°C. The reaction was allowed to stir at 20°C for 0.5 hours. It was quenched with 2 mL of MeOH. The resulting solution was concentrated *in vacuo.* The residue was purified using a silica gel column (12 g) eluting with 5 % MeOH in dichloromethane to provide compound **Int-10d** as a solid. **MS:** *m*/*z* = 564.0 (M + 1).

### Step E- Synthesis of Compound Int-10e

To a solution of compound **Int-10d** (30 mg, 0.053 mmol) in 2 mL of DMF was added Cs₂CO₃ (53.0 mg, 0.160 mmol). The mixture was allowed to stir at 80°C for 1 hour. The solvent was removed under vacuum. The residue was purified using a preparative TLC plate eluting with 10 % MeOH in dichloromethane to provide compound **Int-10e** as a solid. **¹H NMR** (400 MHz, CD₃OD): δ 5.46 (s, 3H), 3.85 (s, 2H), 3.56-3.66 (m, 2H), 3.27 (brs, 4H), 3.14-3.18 (m, 2H), 3.65 (dd, J = 13.5, 5.6 Hz, 1H), 3.30 (ddd, J = 13.3, 10.2, 7.0 Hz, 1H), 1.14-1.24 (m, 3H). **MS:** *m*/*z* = 467.8 (M + 1).

### Step F- Synthesis of Compound Int-10f

To a solution of compound **Int-10e** (25 mg, 0.054 mmol) in 3 mL of DMSO was added (3-chloro-2-fluorophenyl)methanamine (43.7 mg, 0.268 mmol), DIEA (0.093 mL, 0.535 mmol) and Pd(Ph₃P)₄ (13.36 mg, 10.70 µmol). The mixture was allowed to stir at 80°C under a CO balloon for 1.5 hours. The mixture was cooled to room temperature and filtered. The filtrate was purified using a preparative HPLC (Column: YMC-Actus Pro C18 150 ^{∗} 30 mm ^{∗} 5 um; Condition: 0.1 % TFA-ACN; Gradient: 23 % to 53 %; B, 0∼11 minutes; Flow Rate: 40 mL/min) to provide compound **Int-10f** as a solid. **¹H NMR** (400 MHz, CDCl₃): δ 10.81 (brs, 1H), 7.28-7.34 (m, 1H), 7.20-7.26 (m, 1H), 7.00-7.08 (m, 1H), 6.12 (brs, 1H), 5.76 (d, J = 5.5 Hz, 1H), 4.90-4.99 (m, 1H), 4.64-4.72 (m, 1H), 4.49-4.57 (m, 1H), 3.98 (s, 3H), 3.51-3.70 (m, 4H), 3.16 (s, 3H), 3.06 (dd, J = 13.6, 5.7 Hz, 1H), 1.98-3.11 (m, 1H), 1.25 (t, J = 7.2 Hz, 3 H). **MS:** *m*/*z* = 527.1 (M + 1).

### Step G- Synthesis of Compound 58

To a solution of compound **Int-10f** (12 mg, 0.023 mmol) in 2 mL of DMF was added lithium chloride (9.65 mg, 0.228 mmol). The mixture was allowed to stir at 80°C for 10 hours. The mixture was filtered and the filtrate was purified using a preparative HPLC (Column: Phenomenex Synergi C18 150 ^{∗} 30 mm ^{∗} 4 um; Condition: 0.1 % TFA-ACN; Gradient: 33 % to 63 %; B, 0∼8 minutes. Flow Rate: 30 mL/min) to provide afford compound **58** as a solid. **¹H NMR** (400 MHz, CDCl₃): δ 10.86 (brs, 1H), 7.27-7.35 (m, 2H), 7.05 (t, J = 7.7 Hz, 1H), 5.83 (brs, 1H), 5.64 (d, J = 4.6 Hz, 1H), 4.90 (brs, 1H), 4.66 (d, J = 4.6 Hz, 2H), 3.72-3.79 (m, 1H), 3.58-3.69 (m, 3H), 3.06-3.16 (m, 4H), 3.10-3.19 (m, 1H), 1.28 (t, J = 7.2 Hz, 3H). **MS:** *m*/*z* = 513.1 (M + 1).

### EXAMPLE 24

### Preparation of Compound 59

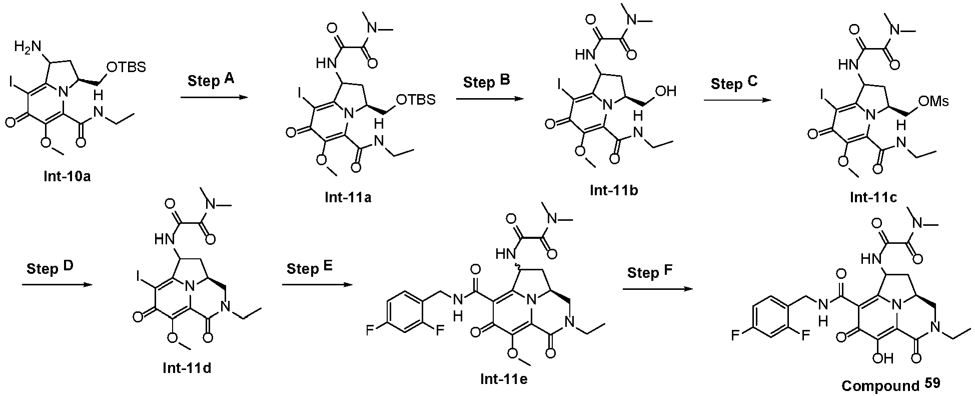

### Step A- Synthesis of Compound Int-11a

To a solution of compound **Int-10a** (800 mg, 1.534 mmol) in dichloromethane (20 mL) was added HOBt (470 mg, 3.07 mmol), EDC (588 mg, 3.07 mmol), diisopropylethylamine (0.536 mL, 3.07 mmol) and 2-(dimethylamino)-2-oxoacetic acid (269 mg, 2.301 mmol). The mixture was allowed to stir at 20°C for 40 minutes. The reaction was quenched with water (10 mL). The organics was isolated. The aqueous layer was extracted with dichloromethane (10 mL × 3). The combined organics were dried over anhydrous Na₂SO₄, filtered and concentrated *in vacuo.* The residue was purified using a silica gel column eluting with 25% EtOAc in petroleum ether to provide compound **Int-11a** as a solid. **¹H NMR** (400 MHz, CD₃OD) δ 5.36-5.49 (m, 1H), 4.95-5.06 (m, 1H), 3.78-3.80 (m, 3H), 3.34-3.42 (m, 2H), 3.15-3.18 (m, 3H), 2.93-2.94 (m, 3H), 1.32 (dd, J = 6.7, 3.4 Hz, 1H), 1.17-1.21 (m, 3H), 0.76-0.86 (m, 9H), -0.14-0.00 (m, 6H). **MS:** *m*/*z* = 621.1 (M + 1).

### Step B- Synthesis of Compound Int-11b

To a solution of compound **Int-11a** (600 mg, 0.967 mmol) in MeOH (3 mL) was added 4N HCl in EtOAc (30 mL, 120 mmol). The mixture was allowed to stir at 20°C for 40 minutes. The reaction mixture was concentrated *in vacuo* to dryness to provide compound **Int-11b** as a solid, which was used without further purification. **¹H NMR** (400 MHz, CD₃OD) δ 5.26 (dd, J = 9.2, 2.1 Hz, 1H), 3.89-3.90 (m, 3H), 3.68-3.75 (m, 3H), 3.56-3.63 (m, 2H), 3.46-3.51 (m, 2H), 3.24 (s, 3H), 3.01-3.02 (m, 3H), 2.80 (ddd, J = 13.3, 9.0, 2.0 Hz, 1H), 2.51-2.61 (m, 1H), 1.28 (t, J = 7.3 Hz, 3H). **MS:** *m*/*z* = 507.1 (M + 1).

### Step C- Synthesis of Compound Int-11c

To a solution of compound **Int-11b** (445 mg, 0.879 mmol) in dichloromethane (3 mL) was added triethylamine (0.735 mL, 5.27 mmol), MsCl (0.103 mL, 1.318 mmol) at 0°C. The mixture was allowed to stir at 20°C for 40 minutes. The reaction was quenched with water (1 mL) and the resulting mixture was concentrated *in vacuo.* The residue was purified using a preparative TLC plate eluting with 10% MeOH in dichloromethane to provide compound **Int-11c** as a solid, which was used without further purification. **MS:** *m*/*z* = 585.2 (M + 1).

### Step D- Synthesis of Compound Int-11d

To a solution of compound **Int-11c** (160 mg, 0.274 mmol) in DMF (1 mL) was added Cs₂CO₃ (89 mg, 0.274 mmol). The mixture was allowed to stir at 80°C for 15 minutes. The mixture was filtered and the filtrate was concentrated *in vacuo.* The residue was purified using a preparative TLC plate eluting with 10% MeOH in dichloromethane to provide compound **Int-11d** as a solid. **¹H NMR** (400 MHz, CDCl₃) δ 8.69-8.82 (m, 1H), 5.40 (t, J = 7.6 Hz, 1H), 5.04-5.17 (m, 1H), 4.09 (q, J = 7.0 Hz, 1H), 3.71-3.82 (m, 3H), 3.63-3.71 (m, 1H), 3.53-3.61 (m, 2H), 3.43 (dd, J = 14.1, 7.0 Hz, 1H), 3.24-3.33 (m, 3H), 2.97-3.05 (m, 3H), 2.51 (dd, J = 13.5, 6.1 Hz, 1H), 2.26 (dd, J = 9.0, 4.3 Hz, 1H), 1.98-2.06 (m, 2H), 1.17-1.21 (m, 3H). **MS:** *m*/*z* = 489.1 (M + 1).

### Step E- Synthesis of Compound Int-11e

To a solution of compound **Int-11d** (50 mg, 0.102 mmol) in DMSO (3 mL) was added (2,4-difluorophenyl)methanamine (29.3 mg, 0.205 mmol), diisopropylethylamine (0.072 mL, 0.410 mmol) and Pd(Ph₃P)₄ (59.2 mg, 0.051 mmol). The mixture was heated at 80°C under a CO balloon for 1.5 hours. The mixture was cooled to room temperature and filtered. The filtrate was purified using a reverse phase HPLC (Column: Waters XSELECT C18 150 ^{∗} 30 mm ^{∗} 5 um; Condition: 0.1% TFA-ACN; Gradient: 0% to 28%; B, 0∼11 minutes; Flow Rate: 25 mL/min) to provide compound **Int-11e** as a solid. **¹H NMR** (400 MHz, CD₃OD) δ 7.35-7.44 (m, 1H), 6.86-6.97 (m, 2H), 6.16 (d, J = 7.4 Hz, 1H), 4.47-4.56 (m, 2H), 3.77-3.86 (m, 4H), 3.51-3.71 (m, 3H), 2.99 (s, 3H), 2.87-2.89 (m, 3H), 2.44 (dd, J = 13.3, 6.3 Hz, 1H), 2.23-2.37 (m, 1H), 1.21 (t, J = 7.0 Hz, 3H). **MS:** *m*/*z* = 532.3 (M + 1).

### Step F- Synthesis of Compound 59

To a solution of compound **Int-11e** (10 mg, 0.019 mmol) in DMF (3 mL) was added lithium chloride (3.99 mg, 0.094 mmol). The mixture was heated at 80°C for 12 hours. The mixture was cooled to room temperature and purified using a reverse HPLC (Column: Waters XSELECT C18 150 ^{∗} 30 mm ^{∗} 5 um; Condition: 0.1% TFA-ACN; Gradient: 0% to 28%; B, 0∼11 minutes; Flow Rate: 25 mL/min) to provide compound **59** as a solid. **¹H NMR** (400 MHz, CDCl₃) δ 10.86 (s, 1H), 8.31 (d, J = 5.7 Hz, 1H), 7.32-7.39 (m, 1H), 6.74-6.86 (m, 2H), 5.79-5.87 (m, 1H), 5.25 (d, J = 4.4 Hz, 1H), 4.50-4.66 (m, 2H), 3.58-3.76 (m, 4H), 3.13 (s, 3H), 2.95 (s, 3H), 2.65 (dd, J = 13.5, 6.6 Hz, 1H), 2.26 (d, J = 13.2 Hz, 1H), 1.25-1.29 (m, 3H). **MS:** *m*/*z* = 518.1 (M + 1).

### EXAMPLE 25

### Preparation of Compound 60

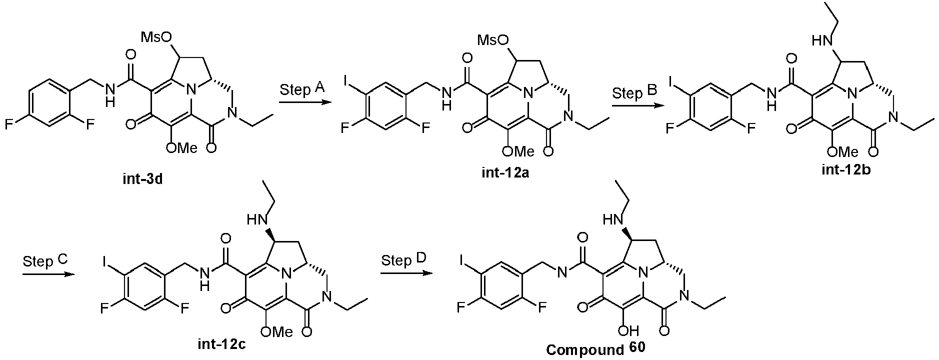

### Step A- Synthesis of Compound Int-12a

To a solution of **Int-3d** (350 mg, 0.704 mmol) in concentrated sulfuric acid (1.4 mL, 26.3 mmol) at 0°C was added *N*-iodosuccinimide (155 mg, 0.689 mmol) portionwise. The resulting mixture was stirred at 0 °C for 3 h before it was poured into ice water (10 mL). The reaction mixture was extracted with EtOAc (3×10 mL). The combined organics were dried over sodium sulfate and filtered. The filtrate was concentrated *in vacuo.* The residue was purified using preparative TLC plate with 9% MeOH in dichloromethane as eluant to provide **int-12a** as a yellow solid. **MS:** *m*/*z* = 638.1 (M+H)⁺.

### Step B- Synthesis of Compound Int-12b

The mixture of **int-12a** (400 mg, 0.628 mmol) and ethanamine (0.359 mL, 2.51 mmol) in EtOH (5 mL) was stirred at 80 °C for 3 h in a sealed tube. The mixture was cooled to room temperature and concentrated *in vacuo.* The residue was purified using a silica gel column eluting with 10% MeOH in dichloromethane to provide compound ***Int-12b*** as a yellow solid. **MS:** *m*/*z* = 587.1 (M+H)⁺.

### Step C- Synthesis of Compound Int-12c

The mixture of Int**-12b** (200 mg, 0.341 mmol) and potassium carbonate (189 mg, 1.364) in MeOH (3 mL) was stirred at 50°C for 15 h. The reaction mixture was cooled to room temperature and filtered. The filtrate was loaded onto SFC (Column: AD (250 mm ^{∗} 30 mm, 10 um); Mobile phase: 45% Base-IPA (contained 0.1% NH₃ O) in CO₂; Flow rate: 80 mL/min; Wavelength: 220 nm). The desired fractions were collected and concentrated to provide pure stereoisomer **Int-12c** as a yellow solid. **MS:** *m*/*z* = 587.1 (M+H)⁺.

### Step D- Synthesis of Compound 60

The mixture of **Int-12c** (150 mg, 0.256 mmol) and magnesium bromide (471 mg, 2.56 mmol) in acetonitrile (3 mL) was stirred at 45°C for 3 h. The mixture was cooled to room temperature and purified using a reverse HPLC (Column: Boston Green ODS 150 mm ^{∗} 30 mm, 5um; Condition: water (0.1% TFA)/ACN; Grandaunt: 24% to 54%; B, 0-8 min; Flow Rate: 30 mL/min) to provide compound **60** as a yellow solid. **¹H NMR** (400 Hz, CD₃OD) δ 7.80 (t, J = 7.6 Hz, 1H), 7.04 (t, J = 9.0 Hz, 1H), 5.02 (d, J = 7.8 Hz, 1H), 4.81 (s, 1H), 4.57 (q, J = 15.5 Hz, 2H), 3.78 (d, J = 12.5 Hz, 1H), 3.46-3.68 (m, 3H), 2.68-2.79 (m, 2H), 2.50 (dd, J = 13.5, 6.1 Hz, 1H), 2.04-2.17 (m, 1H), 1.19 (q, J = 6.8 Hz, 6H). **MS:** *m*/*z* = 572.9 (M+H)⁺.

### EXAMPLE 26

### Preparation of Compound Int-13c

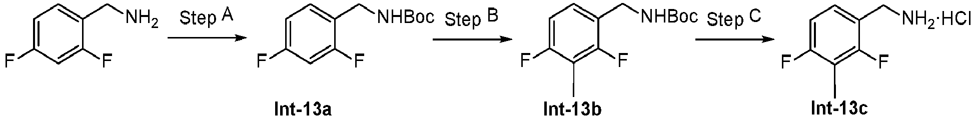

### Step A- Synthesis of Compound Int-13a

A mixture of (2,4-difluorophenyl)methanamine (1 g, 6.99 mmol), triethylamine (2.92 mL, 20.96 mmol) and di-tert-butyl dicarbonate (1.947 mL, 8.38 mmol) in DCM (10 mL) was stirred at 15°C for 1 h. The reaction mixture was concentrated *in vacuo.* The residue was purified using a silica gel column eluting with 0-30% EtOAc in petroleum ether to provide **Int-13a** as a white solid. **¹H NMR** (400 Hz, CDCl₃) δ 7.20-7.30 (m, 1H), 6.65-6.82 (m, 2H), 4.82 (s, 1H), 4.24 (d, J = 5.1 Hz, 2H), 1.37 (s, 9H).

### Step B- Synthesis of Compound Int-13b

To a stirred solution of ***Int-13a*** (800 mg, 3.29 mmol) in THF (10 mL) at 0°C was added sodium hydride (158 mg, 3.95 mmol, 60% w/w), and the mixture was stirred at 0°C for 30 min under nitrogen atmosphere. Then isobutyllithium (3.79 mL, 4.93 mmol) was added to the above mixture at -78°C and the resulting mixture was stirred at this temperature for 30 min followed by addition of a solution of I₂ (1252 mg, 4.93 mmol) in THF (5 mL). The resulting mixture was stirred at -78°C for 30 min, and then allowed to warm to room temperature. It was quenched by addition of aqueous NH₄Cl (15 mL), and the mixture was extracted with EtOAc (3×15 mL). The combined organics were dried over sodium sulfate and filtered. The filtrate was concentrated *in vacuo.* The residue was purified using a silica gel column eluting with 0-20% EtOAc in petroleum ether to provide **Int-13b** as a yellow oil. **¹H NMR** (400 Hz, CDCl₃) δ 7.20-7.33 (m, 1H), 6.73-6.85 (m, 1H), 4.86 (br s, 1H), 4.27 (br d, J=5.8 Hz, 2H), 1.31-1.41 (m, 9H).

### Step C- Synthesis of Compound Int-13c

The mixture of ***Int-13c*** (800 mg, 2.167 mmol) and hydrogen chloride in methanol (10 mL, 40.0 mmol, 4M) was stirred at 15°C for 1 h. The reaction mixture was concentrated *in vacuo* to provide **Int-13c** as a white solid. **MS:** *m*/*z* = 269.9 (M+H)⁺.

### EXAMPLE 27

### Preparation of Compound 61

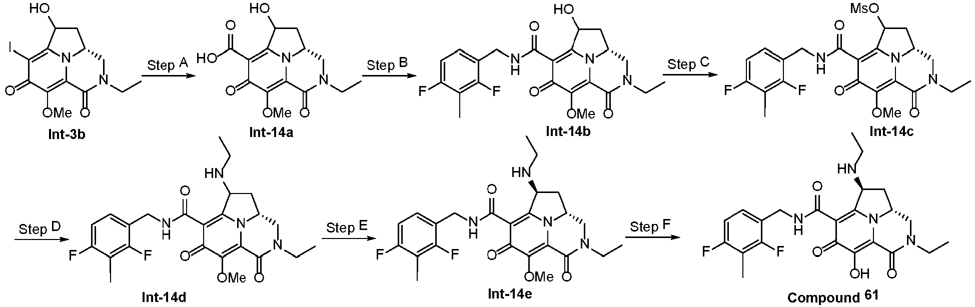

### Step A- Synthesis of Compound Int-14a

The mixture of **Int-3b** (1.5 g, 3.84 mmol), *N*,*N*-diisopropylethylamine (2.69 mL, 15.38 mmol) and tetrakis(triphenylphosphine)palladium (2.221 g, 1.922 mmol) in DMSO (20 mL) and Water (4 mL) was degassed and purged with CO 3 times, then the mixture was stirred under CO (15 psi) at 80°C for 2 h. The resulting reaction was cooled to room temperature and filtered. The filtrate was purified using a preparative HPLC (Column: Phenomenex Synergi Max-RP 250^{∗}80mm^{∗}10 um; Condition: water (0.1%TFA)-ACN; Grandaunt: 10% to 40%; Flow Rate: 150 mL/min) to provide Int**-14a** as a yellow solid. **MS:** *m*/*z* = 309.0 (M + H)⁺.

### Step B- Synthesis of Compound Int-14b

The mixture of ***Int-14a*** (481 mg, 1.561 mmol), ***Int-13c*** (350 mg, 1.301 mmol), (benzotriazol-1-yloxy)tris(dimethylamino)phosphonium hexafluorophosphate (690 mg, 1.561 mmol) and *N*,*N*-diisopropylethylamine (0.682 mL, 3.90 mmol) in CH₂Cl₂ (10 mL) was stirred at 15°C for 12 hours. The reaction mixture was poured into water (30 mL) and extracted with DCM (3×30 mL). The combined organics were dried over sodium sulfate and filtered. The filtrate was concentrated *in vacuo.* The residue was purified using a silica gel column eluting with 0-10% MeOH in dichloromethane to provide Int**-14b** as a yellow solid. **MS:** *m*/*z* = 560.1 (M+H)⁺.

### Step C- Synthesis of Compound Int-14c

To a solution of **Int-14b** (400 mg, 0.715 mmol) and triethylamine (0.797 mL, 5.72 mmol) in CH₂Cl₂ (8 mL) at 0°C was added methanesulfonyl chloride (0.223 mL, 2.86 mmol). The reaction mixture was stirred at 20°C for 15 min under nitrogen atmosphere. The resulting mixture was poured into water (15 mL) and extracted with DCM (3×15 mL). The combined organics were dried over sodium sulfate and filtered. The filtrate was concentrate *in vacuo.* The residue was purified using silica gel column eluting with 0-10% MeOH in dichloromethane to provide **Int-14c** as a yellow solid. **MS:** *m*/*z* = 638.1 (M+H)⁺.

### Step D- Synthesis of Compound Int-14d

The mixture of Int**-14c** (500 mg, 0.784 mmol) and ethanamine (0.319 mL, 2.353 mmol) in EtOH (20 mL) was stirred at 80°C for 2 h. The resulting reaction was cooled to room temperature and concentrated *in vacuo.* The residue was purified using a preparative HPLC (Column: Phenomenex Synergi Max-RP 250 mm ^{∗} 80 mm, 10 um; Condition: water (0.1% TFA)-CAN; Gradient Time: 20 min) to provide **Int-14d** as a white solid. **MS:** *m*/*z* = 587.1 (M + H)+.

### Step E- Synthesis of Compound Int-14e

The mixture of **Int-14d** (140 mg, 0.239 mmol) and potassium carbonate (66.0 mg, 0.478 mmol) in MeOH (3 mL) was stirred at 50°C for 8 h. The resulting reaction was cooled to room temperature and filtered. The filtrate was loaded onto SFC (Column: AD (250 mm ^{∗} 30 mm, 10 um); Mobile phase: 45% Base-IPA (contained 0.1% NH3·H2O) in CO2; Flow rate: 80 mL/min; Wavelength: 220 nm) to provide single stereoisomer **Int-14e** as a yellow solid. **MS:** *m*/*z* = 587.1 (M + H)⁺.

### Step F- Synthesis of Compound 61

The mixture of **Int-14e** (51 mg, 0.087 mmol) and magnesium bromide (64 mg, 0.348 mmol) in acetonitrile (1 mL) was stirred at 45°C for 3 h. The mixture was cooled to room temperature and purified using a reverse HPLC (Column: Boston Green ODS 150 mm ^{∗} 30 mm, 5um; Condition: water (0.1% TFA)/ACN; Grandaunt: 24% to 54%; B, 0-8 min; Flow Rate: 30 mL/min) to provide compound **61** as a yellow solid. **¹H NMR** (400 Hz, CD₃OD) δ 7.44-7.49 (m, 1H), 6.95-6.99 (m, 1H), 5.44 (d, J = 8.4 Hz, 1H), 4.63-4.74 (m, 2H), 3.95 (d, J = 12.0 Hz, 1H), 3.57-3.73 (m, 3H), 2.81-2.84 (m, 1H), 2.46-2.49 (m, 1H), 2.04-2.17 (m, 1H), 1.41 (t, J = 6.8 Hz, 3H), 1.25 (t, J = 6.8 Hz, 3H). **MS:** *m*/*z* = 573.0 (M+H)⁺.

### EXAMPLE 28

### Preparation of Compounds Int-15-P1 and Int-15-P2

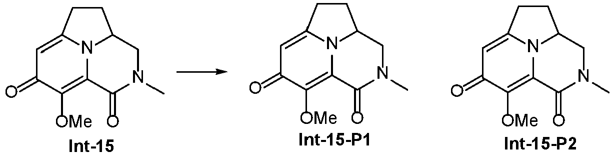

Compound **Int-15** was prepared using the method described in PCT International Patent Publication No. WO2014/183532. The racemic compound **Int-15** (4.0 g, 17.25 mmol) was further separated using a chiral preparative SFC ("Column: AD (250 mm ^{∗} 30 mm, 5 um), Mobile phase: Supercritical CO₂/EtOH (base) = 100/40 at 50 mL/min Wavelength: 220 nm) to provide compound **Int-15-P1** (the first eluting compound) as a yellow solid and compound **Int-15-P2** (the second eluting compound) as a yellow solid.

Compound **Int-15-P1: ¹H NMR** (400 MHz, CDCl₃) δ 6.33 (s, 1H), 4.43-4.55 (m, 1H), 3.95 (s, 3H), 3.54-3.68 (m, 2H), 3.12 (s, 3H), 3.00-3.07 (m, 2H), 2.38-2.47 (m, 1H), 1.94 (q, J = 11.0 Hz, 1H). **MS:** m/z = 235.1 (M + H).

Compound **Int-15-P2:** ¹H NMR (400 MHz, CDCl₃) δ 6.39 (s, 1H), 4.43-4.55 (m, 1H), 4.00 (s, 3H), 3.56-3.72 (m, 2H), 3.15 (s, 3H), 3.01-3.10 (m, 2H), 2.39-2.52 (m, 1H), 1.94 (q, J = 11.0 Hz, 1H). **MS:** m/z = 235.1 (M + H).

### EXAMPLE 29

### Preparation of Compounds 62-64

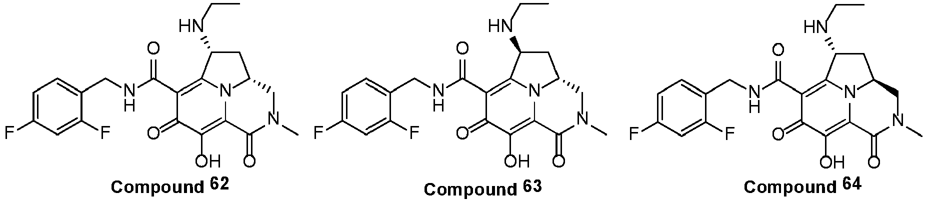

Starting from compound **Int-15-P1** and **Int-15-P2**, and using the method described in Example **4,** the compounds **62-64** were prepared.

Compound **62: ¹H NMR** (400 MHz, CDCl₃) δ 11.51 (s, 1H), 7.29-7.41 (m, 1H), 6.78-6.92 (m, 2H), 4.99 (s, 1H), 4.46-4.68 (m, 3H), 4.07 (t, J = 11.69 Hz, 1H), 3.66 (d, J = 11.91 Hz, 1H), 3.30 (br s, 1H), 3.17 (s, 3H), 3.07 (d, J = 8.38 Hz, 2H), 2.89 (s, 1H), 1.45 (t, J = 7.17 Hz, 3H). **MS:** *m*/*z* = 433.0 (M+H)⁺.

Compound 63: **¹H NMR** (400 MHz, CDCl₃) δ 10.90 (s, 1H), 7.28-7.40 (m, 1H), 6.77-6.91 (m, 2H), 5.01-5.18 (m, 2H), 4.45-4.65 (m, 2H), 3.78-3.79 (m, 2H), 3.33-3.47 (m, 1H), 3.15 (s, 5H), 2.23-2.39 (m, 1H), 1.42 (s, 3H). **MS:** *m*/*z* = 433.0 (M+H)⁺.

Compound **64: ¹H NMR** (400 MHz, CDCl₃) δ 11.10 (s, 1 H), 7.30-7.40 (m, 1H), 6.80-6.91 (m, 2H), 5.44 (d, J=8.4 Hz, 1H), 4.98 (s, 1H), 4.43-4.65 (m, 2H), 3.75-3.89 (m, 1H), 3.67-3.82 (m, 1H), 3.33-3.38 (m, 2H), 3.16 (s, 3H), 2.77-2.90 (m, 1H), 2.30-2.45 (m, 1H), 1.42 (s, 3H). **MS:** *m*/*z* = 433.2 (M+H).

### EXAMPLE 30

### Preparation of Compounds Int-16-P1 and Int-16-P2

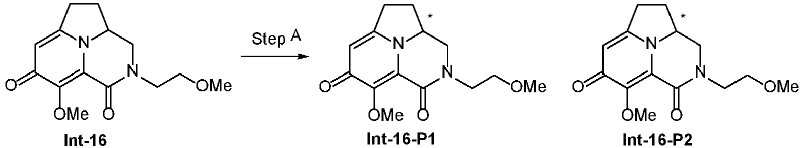

Compound **Int-16** was prepared using the method described in PCT International Patent Publication No. WO2014/183532. The racemic compound **Int-16** (3.6 g, 11.64 mmol) was further separated using a chiral preparative SFC ("Column: OJ (250 mm ^{∗} 30 mm, 5 um), Mobile phase: Supercritical CO₂/EtOH (base) = 100/40 at 50 mL/min Wavelength: 220 nm) to provide compound **Int-16-P1** (the first eluting compound) as a yellow solid and compound **Int-16-P2** (the second eluting compound) as a yellow solid.

Compound **Int-16-P1: ¹H NMR** (400 MHz, CDCl₃) δ 6.40 (s, 1H), 4.38-4.48 (m, 1H), 3.99 (s, 3H), 3.54-3.81 (m, 6H), 3.32 (s, 3H), 2.96-3.08 (m, 2H), 2.35-2.50 (m, 1H), 1.80-2.00 (m, 1H). **MS:** *m*/*z* = 279.1(M + H).

Compound **Int-16-P2: ¹H NMR** (400 MHz, CDCl₃) δ 6.38 (s, 1H), 4.36-4.48 (m, 1H), 3.97 (s, 3H), 3.50-3.79 (m, 6H), 3.32 (s, 3H), 2.98-3.13 (m, 2H), 2.36-2.48 (m, 1H), 1.81-2.00 (m, 1H). **MS:** m/z = 279.1(M + H).

### EXAMPLE 31

### Preparation of Compounds 65-67

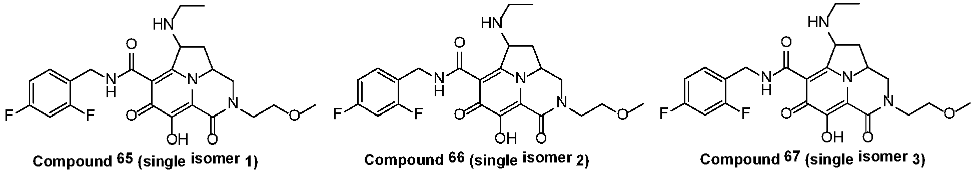

Starting from compound **Int-16-P1** and **Int-16-P2**, and using the method described in Example **4,** the compounds **65-67** were prepared.

Compound **65: ¹H NMR** (400 MHz, CDCl₃) δ 7.46 (d, J = 6.6 Hz, 1H), 6.88-7.02 (m, 2H), 5.45 (d, J = 7.1 Hz, 1H), 4.66 (d, J = 10.8 Hz, 2H), 4.03 (d, J = 10.6 Hz, 1H), 3.54-3.86 (m, 6H), 3.36 (s, 5H), 2.86 (s, 1H), 2.47 (s, 1H), 2.37-2.49 (m, 1H), 1.41 (s, 3H).**MS:** *m*/*z* = 477.3 (M + H)⁺.

Compound **66: ¹H NMR** (400 MHz, CDCl₃) δ 7.44 (q, J = 7.5 Hz, 1H), 6.89-7.01 (m, 2H), 5.35 (t, J = 7.8 Hz, 1H), 4.59-4.74 (m, 3H), 3.97-4.17 (m, 1H), 4.05 (d, J = 12.3 Hz, 1H), 3.73-3.92 (m, 3H), 3.64 (s, 2H), 3.33-3.35 (m, 1H), 3.20-3.33 (m, 4H), 3.05-3.15 (m, 1H), 2.19-2.33 (m, 1H), 1.41 (t, J = 6.9 Hz, 3H). **MS:** *m*/*z* = 477.3 (M + H).

Compound **67: ¹H NMR** (400 MHz, CDCl₃) δ 7.46 (d, J = 6.6 Hz, 1H), 6.88-7.02 (m, 2H), 5.45 (d, J = 7.1 Hz, 1H), 4.66 (d, J = 10.8 Hz, 2H), 4.03 (d, J = 10.6 Hz, 1H), 3.54-3.86 (m, 6H), 3.36 (s, 5H), 2.86 (s, 1H), 2.47 (s, 1H), 2.37-2.49 (m, 1H), 1.41 (s, 3H). **MS:** *m*/*z* = 477.3 (M + H).

### EXAMPLE 32

### Preparation of Compounds Int-17-P1 and Int-17-P2

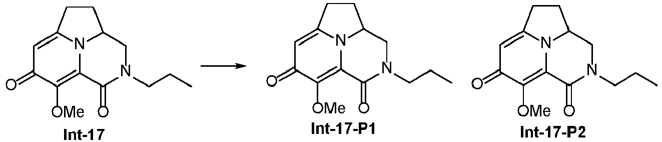

Compound **Int-17** was prepared using the method described in PCT International Patent Publication No. WO2014/183532. The racemic compound **Int-17** (4.0 g, 17.25 mmol) was further separated using a chiral preparative SFC ("Column: AD (250 mm ^{∗} 30 mm, 5 um), Mobile phase: Supercritical CO₂/EtOH (base) = 100/40 at 50 mL/min Wavelength: 220 nm) to provide compound **Int-17-P1** (the first eluting compound) as a yellow solid and compound **Int-17-P2** (the second eluting compound) as a yellow solid.

Compound **Int-17-P1: ¹H NMR** (400 MHz, CDCl₃) δ 6.40 (s, 1H), 4.36-4.51 (m, 1H), 4.01 (s, 3H), 3.56-3.65 (m, 2H), 3.49-3.56 (m, 2H), 3.00-3.12 (m, 2H), 2.41-2.51 (m, 1H), 1.89-2.02 (m, 1H), 1.59-1.70 (m, 2H), 0.96 (t, J = 7.4 Hz, 3H). **MS:** *m*/*z* = 263.1 (M + H)⁺.

Compound **Int-17-P2: ¹H NMR** (400 MHz, CDCl₃) δ 6.38 (s, 1H), 4.37-4.50 (m, 1H), 3.99 (s, 3H), 3.56-3.65 (m, 2H), 3.48-3.55 (m, 2H), 2.99-3.14 (m, 2H), 2.40-2.51 (m, 1H), 1.90-2.02 (m, 1H), 1.59-1.70 (m, 2H), 0.95 (t, J = 7.3 Hz, 3H). **MS:** *m*/*z* = 263.1 (M + H)⁺.

### EXAMPLE 33

### Preparation of Compounds 68-71

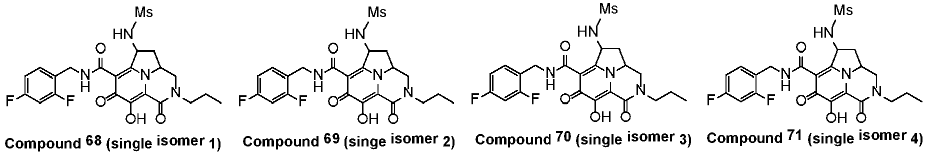

Starting from compound **Int-16-P1** and **Int-16-P2**, and using the method described in Example **19,** the compounds **68-71** were prepared.

Compound **68: ¹H NMR** (400 MHz, CDCl₃) δ 11.00 (s, 1H), 7.29-7.41 (m, 2H), 6.76-6.88 (m, 2H), 5.31 (s, 1H), 4.60 (d, J = 4.5 Hz, 2H), 4.44 (s, 1H), 3.77-3.88 (m, 1H), 3.68 (d, J = 11.3 Hz, 1H), 3.52 (t, J = 7.3 Hz, 2H), 2.98 (s, 4H) , 1.68 (m, 2H), 0.98 (t, J = 7.3 Hz, 3H). **MS:** *m*/*z* = 511.1 (M + H)⁺.

Compound **69: ¹H NMR** (400 MHz, CDCl₃) δ 10.64 (s, 1H), 7.30-7.40 (m, 1H), 6.83 (q, J = 8.3 Hz, 2H), 6.10 (s, 1H), 5.68 (s, 1H), 4.85 (br. s., 1H), 4.46-4.64 (m, 2H), 3.61-3.77 (m, 2H), 3.43-3.58 (m, 2H), 3.10 (s, 3H), 2.98-3.06 (m, 1H), 1.69 (qd, J = 7.4, 14.6 Hz, 2H), 0.99 (t, J = 7.4 Hz, 3H). **MS:** *m*/*z* = 511.2 (M + H)⁺.

Compound **70: ¹H NMR** (400 MHz, CD₃OD) δ 7.50-7.40 (m, 1H), 7.00-6.88 (m, 2H), 5.93 (d, J = 6.3 Hz, 1H), 4.82-4.76 (m, 1H), 4.69-4.52 (m, 2H), 3.92 (dd, J = 3.9, 12.9 Hz, 1H), 3.69 (t, J = 12.3 Hz, 1H), 3.56 (t, J = 7.2 Hz, 2H), 3.06 (s, 3H), 2.72 (dd, J = 5.3, 13.1 Hz, 1H), 2.30-2.16 (m, 1H), 1.78-1.64 (m, 2H), 0.98 (t, J = 7.2 Hz, 3H). **MS:** m/z = 511.1 (M + H)⁺.

Compound **71: ¹H NMR** (400 MHz, CD₃OD) δ 7.51-7.41 (m, 1H), 6.97-6.82 (m, 2H), 5.46 (br s, 1H), 4.59 (br s, 2H), 4.47 (br s, 1H), 3.83 (br s, 1H), 3.69 (br s, 1H), 3.59-3.45 (m, 2H), 3.04 (br s, 1H), 2.95 (s, 3H), 2.14 (br s, 1H), 1.72-1.62 (m, 2H), 1.01-0.88 (m, 3H). **MS:** m/z = 511.1 (M + H)⁺.

### EXAMPLE 34

### Preparation of Compounds 72-75

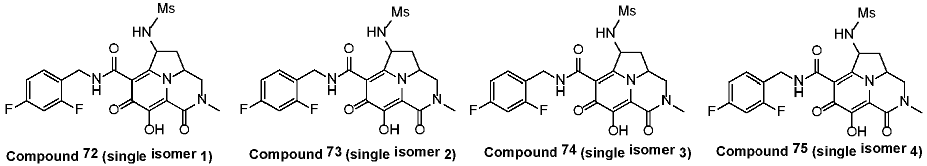

Starting from compound **Int-15-P1** and **Int-15-P2**, and using the method described in Example **19,** the compounds **72-75** were prepared.

Compound **72: ¹H NMR** (400 MHz, CD₃OD) δ 7.50 (d, J = 6.4 Hz, 1H), 6.86-7.01 (m, 2H), 5.50 (t, J = 8.1 Hz, 1H), 4.62 (s, 2H), 4.54 (br. s., 1H), 3.87 (d, J = 12.6 Hz, 1H), 3.72-3.80 (m, 1H), 3.05-3.20 (m, 4H), 2.98 (s, 3H), 2.10-2.24 (m, 1H). **MS:** *m*/*z* = 483.0 (M + H)⁺.

Compound **73: ¹H NMR** (400 MHz, CD₃OD) δ 7.33-7.54 (m, 1H), 6.82-7.02 (m, 2H), 5.93 (d, J = 6.2 Hz, 1H), 4.75-4.84 (m, 1H), 4.50-4.68 (m, 2H), 3.89 (d, J = 10.1 Hz, 1H), 3.68 (t, J = 12.0 Hz, 1H), 3.16 (s, 3H), 3.05 (s, 3H), 2.71 (d, J = 8.6 Hz, 1H), 2.22 (s, 1H). **MS:** *m*/*z* = 482.9 (M + H)+.

Compound **74: ¹H NMR** (400 MHz, CDCl₃) δ 11.15 (s, 1H), 7.30-7.37 (m, 1H), 6.75-6.87 (m, 2H), 5.35 (br s, 1H), 4.62 (br s, 2H), 4.44 (br s, 1H), 3.91 (t, J = 11.8 Hz, 1H), 3.70 (d, J = 11.9 Hz, 1H), 3.20 (s, 3H), 2.98 (s, 4H), 2.59 (br s, 1H). **MS:** *m*/*z* = 483.0 (M + H)⁻.

Compound **75: ¹H NMR** (400 MHz, CDCl₃) δ 10.76 (br s, 1H), 7.29-7.39 (m, 1H), 6.76-6.89 (m, 2H), 5.89 (br s, 1H), 5.65 (br s, 1H), 4.91 (br s, 1H), 4.50-4.64 (m, 2H), 3.63-3.77 (m, 2H), 3.17 (s, 3H), 3.07 (s, 4H), 2.12 (s, 1H). **MS:** *m*/*z* = 483.0 (M + H)⁺.

### EXAMPLE 35

### Assessing Antiviral Potency Using An HIV-1 Infection Assay

HIV-1 replication was monitored using MT4-gag-GFP clone D3 (hereafter designate MT4-GFP), which are MT-4 cells modified to harbor a GFP reporter gene, the expression of which is dependent on the HIV-1 expressed proteins tat and rev. Productive infection of an MT4-GFP cell with HIV-1 results in GFP expression approximately 24h post-infection.

MT4-GFP cells were maintained at 37°C/5% CO₂/90% relative humidity in RPMI 1640 supplemented with 10% fetal bovine serum, 100 U/ml penicillin/streptomycin, and 400µg/ml G418 to maintain the reporter gene. For infections, MT4-GFP cells were placed in the same medium lacking G418 and infected overnight with H9IIIB or NL4-3 virus at an approximate multiplicity of infection of 0.01 in the same incubation conditions. Cells were then washed and resuspended in either RPMI 1640 containing no serum at 1.6 × 10⁵ cells/mL (serum free conditions), 10% normal human serum at 1.6 × 10⁵ cells/mL (10% NHS conditions) or in 100% normal human serum at 2 × 10⁵ cells/mL (100% NHS conditions). Compound plates were prepared by dispensing compounds taken up in DMSO into wells of 384 well poly D lysine-coated plates (0.2µl/well) using an ECHO acoustic dispenser. Each compound was tested in a 10 point serial 3-fold dilution (typical final concentrations: 4.2 µM - 0.21 nM). Controls included no inhibitor (DMSO only) and a combination of three antiviral agents (efavirenz, indinavir, and the integrase strand transfer inhibitor L-002254051 at final concentrations of 4µM each). Cells were added (50µL/well) to compound plates and the infected cells were maintained at 37°C/5% CO₂/90% relative humidity.

Infected cells were quantified at two time points, ∼48h and∼ 72h post-infection, by counting the number of green cells in each well using an Acumen eX3 scanner. The increase in the number of green cells over ∼24h period gives the reproductive ratio, R₀, which is typically 5-15 and has been shown experimentally to be in logarithmic phase (data not shown). Inhibition of R₀ is calculated for each well, and IC₅₀ value was determined using non-linear 4-parameter curve fitting.

Illustrative compounds of the present invention were tested using this assay protocol and results are presented in the table below.

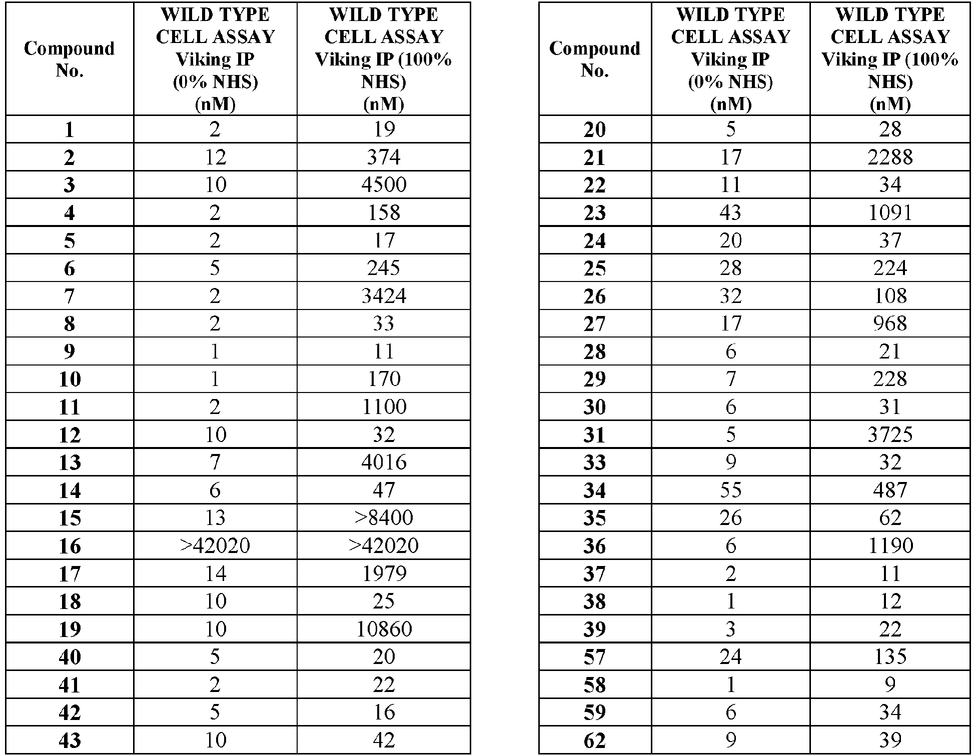

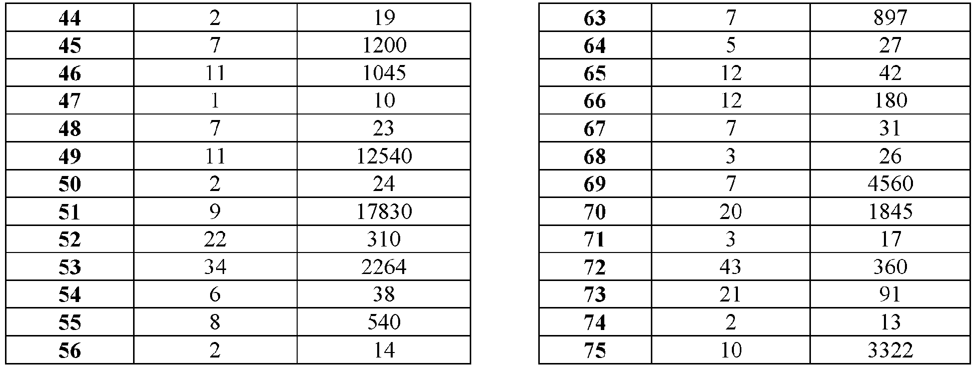

### Uses of the Fused Tricyclic Heterocyclic Compounds

The Fused Tricyclic Heterocyclic Compounds may be useful in human and veterinary medicine for treating or preventing HIV infection in a subject. In one embodiment, the Fused Tricyclic Heterocyclic Compounds can be inhibitors of HIV viral replication. In a specific embodiment, the Fused Tricyclic Heterocyclic Compounds are inhibitors of HIV-1. Accordingly, the Fused Tricyclic Heterocyclic Compounds may be useful for treating HIV infections and AIDS. In accordance with the invention, the Fused Tricyclic Heterocyclic Compounds can be administered to a subject in need of treatment or prevention of HIV infection.

Accordingly, in one embodiment, the invention provides methods for treating HIV infection in a subject comprising administering to the subject an effective amount of at least one Fused Tricyclic Heterocyclic Compound or a pharmaceutically acceptable salt or prodrug thereof. In a specific embodiment, the present invention provides methods for treating AIDS in a subject comprising administering to the subject an effective amount of at least one Fused Tricyclic Heterocyclic Compound or a pharmaceutically acceptable salt or prodrug thereof.

### Treatment or Prevention of HIV Infection

The Fused Tricyclic Heterocyclic Compounds may be useful in the inhibition of HIV, the inhibition of HIV integrase, the treatment of HIV infection and/or reduction of the likelihood or severity of symptoms of HIV infection and the inhibition of HIV viral replication and/or HIV viral production in a cell-based system. For example, the Fused Tricyclic Heterocyclic Compounds may be useful in treating infection by HIV after suspected past exposure to HIV by such means as blood transfusion, exchange of body fluids, bites, accidental needle stick, or exposure to subject blood during surgery or other medical procedures.

Accordingly, in one embodiment, the invention provides methods for treating HIV infection in a subject, the methods comprising administering to the subject an effective amount of at least one Fused Tricyclic Heterocyclic Compound or a pharmaceutically acceptable salt or prodrug thereof. In a specific embodiment, the amount administered is effective to treat or prevent infection by HIV in the subject. In another specific embodiment, the amount administered is effective to inhibit HIV viral replication and/or viral production in the subject. In one embodiment, the HIV infection has progressed to AIDS.

The Fused Tricyclic Heterocyclic Compounds are also useful in the preparation and execution of screening assays for antiviral compounds. For example the Fused Tricyclic Heterocyclic Compounds may be useful for identifying resistant HIV cell lines harboring mutations, which are excellent screening tools for more powerful antiviral compounds. Furthermore, the Fused Tricyclic Heterocyclic Compounds may be useful in establishing or determining the binding site of other antivirals to the HIV Integrase.

The compositions and combinations of the present invention may be useful for treating a subject suffering from infection related to any HIV genotype.

### Combination Therapy

In another embodiment, the present methods for treating or preventing HIV infection can further comprise the administration of one or more additional therapeutic agents which are not Fused Tricyclic Heterocyclic Compounds.

In one embodiment, the additional therapeutic agent is an antiviral agent.

In another embodiment, the additional therapeutic agent is an immunomodulatory agent, such as an immunosuppressive agent.

Accordingly, in one embodiment, the present invention provides methods for treating a viral infection in a subject, the method comprising administering to the subject: (i) at least one Fused Tricyclic Heterocyclic Compound (which may include two or more different Fused Tricyclic Heterocyclic Compounds), or a pharmaceutically acceptable salt or prodrug thereof, and (ii) at least one additional therapeutic agent that is other than a Fused Tricyclic Heterocyclic Compound, wherein the amounts administered are together effective to treat or prevent a viral infection.

When administering a combination therapy of the invention to a subject, therapeutic agents in the combination, or a pharmaceutical composition or compositions comprising therapeutic agents, may be administered in any order such as, for example, sequentially, concurrently, together, simultaneously and the like. The amounts of the various actives in such combination therapy may be different amounts (different dosage amounts) or same amounts (same dosage amounts). Thus, for non-limiting illustration purposes, a Fused Tricyclic Heterocyclic Compound and an additional therapeutic agent may be present in fixed amounts (dosage amounts) in a single dosage unit (*e.g*., a capsule, a tablet and the like).

In one embodiment, at least one Fused Tricyclic Heterocyclic Compound is administered during a time when the additional therapeutic agent(s) exert their prophylactic or therapeutic effect, or *vice versa.*

In another embodiment, at least one Fused Tricyclic Heterocyclic Compound and the additional therapeutic agent(s) are administered in doses commonly employed when such agents are used as monotherapy for treating a viral infection.

In another embodiment, at least one Fused Tricyclic Heterocyclic Compound and the additional therapeutic agent(s) are administered in doses lower than the doses commonly employed when such agents are used as monotherapy for treating a viral infection.

In still another embodiment, at least one Fused Tricyclic Heterocyclic Compound and the additional therapeutic agent(s) act synergistically and are administered in doses lower than the doses commonly employed when such agents are used as monotherapy for treating a viral infection.

In one embodiment, at least one Fused Tricyclic Heterocyclic Compound and the additional therapeutic agent(s) are present in the same composition. In one embodiment, this composition is suitable for oral administration. In another embodiment, this composition is suitable for intravenous administration. In another embodiment, this composition is suitable for subcutaneous administration. In still another embodiment, this composition is suitable for parenteral administration.

Viral infections and virus-related disorders that may be treated or prevented using the combination therapy methods of the present invention include, but are not limited to, those listed above.

In one embodiment, the viral infection is HIV infection.

In another embodiment, the viral infection is HIV infection that has progressed to AIDS.

The at least one Fused Tricyclic Heterocyclic Compound and the additional therapeutic agent(s) can act additively or synergistically. A synergistic combination may allow the use of lower dosages of one or more agents and/or less frequent administration of one or more agents of a combination therapy. A lower dosage or less frequent administration of one or more agents may lower toxicity of therapy without reducing the efficacy of therapy.

In one embodiment, the administration of at least one Fused Tricyclic Heterocyclic Compound and the additional therapeutic agent(s) may inhibit the resistance of a viral infection to these agents.

As noted above, the present invention is also directed to use of a compound of Formula I with one or more anti-HIV agents. An "anti-HIV agent" is any agent which is directly or indirectly effective in the inhibition of HIV reverse transcriptase or another enzyme required for HIV replication or infection, the treatment or prophylaxis of HIV infection, and/or the treatment, prophylaxis or delay in the onset or progression of AIDS. It is understood that an anti-HIV agent is effective in treating, preventing, or delaying the onset or progression of HIV infection or AIDS and/or diseases or conditions arising therefrom or associated therewith. For example, the compounds of this invention may be effectively administered, whether at periods of pre-exposure and/or post-exposure, in combination with effective amounts of one or more anti-HIV agents selected from HIV antiviral agents, immunomodulators, antiinfectives, or vaccines useful for treating HIV infection or AIDS. Suitable HIV antivirals for use in combination with the compounds of the present invention include, for example, those listed in Table A as follows:

**Table A**

| **Name** | **Type** |
|---|---|
| abacavir, ABC, Ziagen^{®} | nRTI |
| abacavir +lamivudine, Epzicom^{®} | nRTI |
| abacavir + lamivudine + zidovudine, Trizivir^{®} | nRTI |
| amprenavir, Agenerase^{®} | PI |
| atazanavir, Reyataz^{®} | PI |
| AZT, zidovudine, azidothymidine, Retrovir^{®} | nRTI |
| darunavir, Prezista^{®} | PI |
| ddC, zalcitabine, dideoxycytidine, Hivid^{®} | nRTI |
| ddI, didanosine, dideoxyinosine, Videx^{®} | nRTI |
| ddI (enteric coated), Videx EC^{®} | nRTI |
| delavirdine, DLV, Rescriptor^{®} | nnRTI |
| dolutegravir, Tivicay^{®} | II |
| doravirine | nnRTI |
| efavirenz, EFV, Sustiva^{®}, Stocrin^{®} | nnRTI |
| efavirenz + emtricitabine + tenofovir DF, Atripla^{®} | nnRTI + nRTI |
| EFdA (4'-ethynyl-2-fluoro-2'-deoxyadenosine) | nRTI |
| emtricitabine, FTC, Emtriva^{®} | nRTI |
| emtricitabine + tenofovir DF, Truvada^{®} | nRTI |
| emvirine, Coactinon^{®} | nnRTI |
| enfuvirtide, Fuzeon^{®} | FI |
| enteric coated didanosine, Videx EC^{®} | nRTI |
| etravirine, TMC-125 | nnRTI |
| fosamprenavir calcium, Lexiva^{®} | PI |
| GSK-744 | II |
| indinavir, Crixivan^{®} | PI |
| lamivudine, 3TC, Epivir^{®} | nRTI |
| lamivudine + zidovudine, Combivir^{®} | nRTI |
| lopinavir | PI |
| lopinavir + ritonavir, Kaletra^{®} | PI |
| maraviroc, Selzentry^{®} | EI |
| nelfinavir, Viracept^{®} | PI |
| nevirapine, NVP, Viramune^{®} | nnRTI |
| rilpivirine, TMC-278 | nnRTI |
| ritonavir, Norvir^{®} | PI |
| saquinavir, Invirase^{®}, Fortovase^{®} | PI |
| stavudine, d4T,didehydrodeoxythymidine, Zerit^{®} | nRTI |
| tenofovir DF (DF = disoproxil fumarate), TDF, Viread^{®} | nRTI |
| tipranavir, Aptivus^{®} | PI |

| | |
|---|---|
| EI = entry inhibitor; FI = fusion inhibitor; PI = protease inhibitor; nRTI = nucleoside reverse transcriptase inhibitor; II =integrase inhibitor; nnRTI = non-nucleoside reverse transcriptase inhibitor. Some of the drugs listed in the table are used in a salt form; e.g., abacavir sulfate, indinavir sulfate, atazanavir sulfate, nelfinavir mesylate. | |

In one embodiment, one or more anti-HIV drugs are selected from, lamivudine, abacavir, ritonavir, darunavir, atazanavir, emtricitabine, tenofovir, rilpivirine and lopinavir.

In another embodiment, the compound of formula (I) is used in combination with lamivudine.

In still another embodiment, the compound of formula (I) is used in combination atazanavir.

In another embodiment, the compound of formula (I) is used in combination with darunavir.

In another embodiment, the compound of formula (I) is used in combination with rilpivirine.

In one embodiment, the compound of formula (I) is used in combination with lamivudine and abacavir.

In another embodiment, the compound of formula (I) is used in combination with emtricitabine and tenofovir.

In another embodiment, the compound of formula (I) is used in combination with ritonavir and lopinavir.

In one embodiment, the compound of formula (I) is used in combination with abacavir and lamivudine.

In another embodiment, the compound of formula (I) is used in combination with lopinavir and ritonavir.

In one embodiment, the present invention provides pharmaceutical compositions comprising (i) a compound of formula (I) or a pharmaceutically acceptable salt or prodrug thereof; (ii) a pharmaceutically acceptable carrier; and (iii) one or more additional anti-HIV agents selected from lamivudine, abacavir, ritonavir and lopinavir, or a pharmaceutically acceptable salt or prodrug thereof, wherein the amounts present of components (i) and (iii) are together effective for the treatment or prophylaxis of infection by HIV or for the treatment, prophylaxis, or delay in the onset or progression of AIDS in the subject in need thereof.

In another embodiment, the present invention provides a method for the treatment or prophylaxis of infection by HIV or for the treatment, prophylaxis, or delay in the onset or progression of AIDS in a subject in need thereof, which comprises administering to the subject (i) a compound of formula (I) or a pharmaceutically acceptable salt or prodrug thereof and (ii) one or more additional anti-HIV agents selected from lamivudine, abacavir, ritonavir and lopinavir, or a pharmaceutically acceptable salt or prodrug thereof, wherein the amounts administered of components (i) and (ii) are together effective for the treatment or prophylaxis of infection by HIV or for the treatment, prophylaxis, or delay in the onset or progression of AIDS in the subject in need thereof.

It is understood that the scope of combinations of the compounds of this invention with anti-HIV agents is not limited to the HIV antivirals listed in Table A, but includes in principle any combination with any pharmaceutical composition useful for the treatment or prophylaxis of AIDS. The HIV antiviral agents and other agents will typically be employed in these combinations in their conventional dosage ranges and regimens as reported in the art, including, for example, the dosages described in the Physicians' Desk Reference, Thomson PDR, Thomson PDR, 57th edition (2003), the 58th edition (2004), the 59th edition (2005), and the like. The dosage ranges for a compound of the invention in these combinations are the same as those set forth above.

The doses and dosage regimen of the other agents used in the combination therapies of the present invention for the treatment or prevention of HIV infection may be determined by the attending clinician, taking into consideration the approved doses and dosage regimen in the package insert; the age, sex and general health of the subject; and the type and severity of the viral infection or related disease or disorder. When administered in combination, the Fused Tricyclic Heterocyclic Compound(s) and the other agent(s) may be administered simultaneously (i.e., in the same composition or in separate compositions one right after the other) or sequentially. This is particularly useful when the components of the combination are given on different dosing schedules, e.g., one component is administered once daily and another component is administered every six hours, or when the pharmaceutical compositions are different, *e.g*., one is a tablet and one is a capsule. A kit comprising the separate dosage forms is therefore advantageous.

### Compositions and Administration

When administered to a subject, the Fused Tricyclic Heterocyclic Compounds may be administered as a component of a composition that comprises a pharmaceutically acceptable carrier or vehicle. The present invention provides pharmaceutical compositions comprising an effective amount of at least one Fused Tricyclic Heterocyclic Compound and a pharmaceutically acceptable carrier. In the pharmaceutical compositions and methods of the present invention, the active ingredients will typically be administered in admixture with suitable carrier materials suitably selected with respect to the intended form of administration, *i.e.,* oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral non-toxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Solid form preparations include powders, tablets, dispersible granules, capsules, cachets and suppositories. Powders and tablets may be comprised of from about 0.5 to about 95 percent inventive composition. Tablets, powders, cachets and capsules may be used as solid dosage forms suitable for oral administration.

Moreover, when desired or needed, suitable binders, lubricants, disintegrating agents and coloring agents may also be incorporated in the mixture. Suitable binders include starch, gelatin, natural sugars, corn sweeteners, natural and synthetic gums such as acacia, sodium alginate, carboxymethylcellulose, polyethylene glycol and waxes. Among the lubricants there may be mentioned for use in these dosage forms, boric acid, sodium benzoate, sodium acetate, sodium chloride, and the like. Disintegrants include starch, methylcellulose, guar gum, and the like. Sweetening and flavoring agents and preservatives may also be included where appropriate.

Liquid form preparations include solutions, suspensions and emulsions and may include water or water-propylene glycol solutions for parenteral injection.

Liquid form preparations may also include solutions for intranasal administration.

Also included are solid form preparations which are intended to be converted, shortly before use, to liquid form preparations for either oral or parenteral administration. Such liquid forms include solutions, suspensions and emulsions.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Additionally, the compositions of the present invention may be formulated in sustained release form to provide the rate controlled release of any one or more of the components or active ingredients to optimize therapeutic effects, *i.e.,* antiviral activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

In one embodiment, the one or more Fused Tricyclic Heterocyclic Compounds are administered orally.

In another embodiment, the one or more Fused Tricyclic Heterocyclic Compounds are administered intravenously.

In one embodiment, a pharmaceutical preparation comprising at least one Fused Tricyclic Heterocyclic Compound is in unit dosage form. In such form, the preparation is subdivided into unit doses containing effective amounts of the active components.

Compositions may be prepared according to conventional mixing, granulating or coating methods, respectively, and the present compositions can contain, in one embodiment, from about 0.1% to about 99% of the Fused Tricyclic Heterocyclic Compound(s) by weight or volume. In various embodiments, the present compositions can contain, in one embodiment, from about 1% to about 70% or from about 5% to about 60% of the Fused Tricyclic Heterocyclic Compound(s) by weight or volume.

The compounds of Formula I may be administered orally in a dosage range of 0.001 to 1000 mg/kg of mammal (e.g., human) body weight per day in a single dose or in divided doses. One dosage range is 0.01 to 500 mg/kg body weight per day orally in a single dose or in divided doses. Another dosage range is 0.1 to 100 mg/kg body weight per day orally in single or divided doses. For oral administration, the compositions may be provided in the form of tablets or capsules containing 1.0 to 500 milligrams of the active ingredient, particularly 1, 5, 10, 15, 20, 25, 50, 75, 100, 150, 200, 250, 300, 400, and 500 milligrams of the active ingredient for the symptomatic adjustment of the dosage to the subject to be treated. The specific dose level and frequency of dosage for any particular subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

For convenience, the total daily dosage may be divided and administered in portions during the day if desired. In one embodiment, the daily dosage is administered in one portion. In another embodiment, the total daily dosage is administered in two divided doses over a 24 hour period. In another embodiment, the total daily dosage is administered in three divided doses over a 24 hour period. In still another embodiment, the total daily dosage is administered in four divided doses over a 24 hour period.

The unit dosages of the Fused Tricyclic Heterocyclic Compounds may be administered at varying frequencies. In one embodiment, a unit dosage of a Fused Tricyclic Heterocyclic Compound may be administered once daily. In another embodiment, a unit dosage of a Fused Tricyclic Heterocyclic Compound may be administered twice weekly. In another embodiment, a unit dosage of a Fused Tricyclic Heterocyclic Compound may be administered once weekly. In still another embodiment, a unit dosage of a Fused Tricyclic Heterocyclic Compound may be administered once biweekly. In another embodiment, a unit dosage of a Fused Tricyclic Heterocyclic Compound may be administered once monthly. In yet another embodiment, a unit dosage of a Fused Tricyclic Heterocyclic Compound may be administered once bimonthly. In another embodiment, a unit dosage of a Fused Tricyclic Heterocyclic Compound may be administered once every 3 months. In a further embodiment, a unit dosage of a Fused Tricyclic Heterocyclic Compound may be administered once every 6 months. In another embodiment, a unit dosage of a Fused Tricyclic Heterocyclic Compound may be administered once yearly.

The amount and frequency of administration of the Fused Tricyclic Heterocyclic Compounds will be regulated according to the judgment of the attending clinician considering such factors as age, condition and size of the subject as well as severity of the symptoms being treated. The compositions of the invention can further comprise one or more additional therapeutic agents, selected from those listed above herein.

## Claims

1. A compound having the formula: 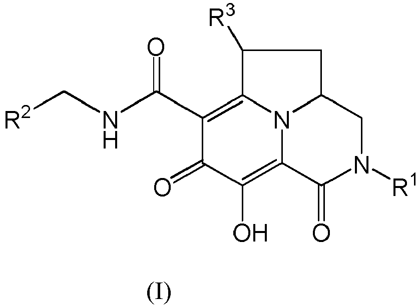 or a pharmaceutically acceptable salt thereof,
wherein:
R¹ is C₁-C₆ alkyl or -(C₁-C₆ alkenyl)-O-(C₁-C₆ alkyl);
R² is phenyl, which is optionally substituted with up to 3 ring substituent groups, each independently selected from halo;
R³ is -N(R⁴)₂;
each occurrence of R⁴ is independently selected from H, C₁-C₆ alkyl, C₁-C₆ haloalkyl, C₃-C₇ cycloalkyl, -CH₂-(C₃-C₇ cycloalkyl), phenyl, benzyl, -C(O)-C(O)-N(R⁵)₂,-S(O)₂-C₁-C₆ alkyl, -S(O)₂-phenyl, -(C₁-C₆ alkenyl)-O-(C₁-C₆ alkyl) and -C(O)-C₁-C₆ alkyl, where the phenyl moiety of a -S(O)₂-phenyl group can be optionally substituted with a C₁-C₆ alkyl group; or both R⁴ groups, and the common nitrogen atom to which they are attached, join to form an azetidinyl, piperidinyl, or pyrrolidinyl group; and
each occurrence of R⁵ is independently selected from H and C₁-C₆ alkyl.

2. The compound of claim 1, having the formula (Ia), (Ib), (Ic) and (Id):

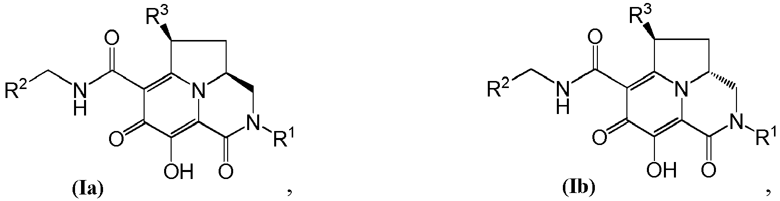

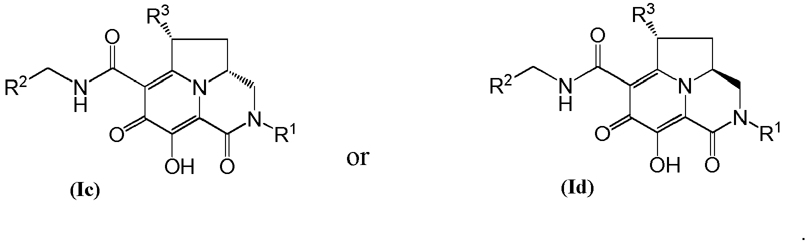

3. The compound of claim 1 or 2, wherein R¹ is C₁-C₆ alkyl.

4. The compound of claim 3, wherein R¹ is ethyl.

5. The compound of any of claims 1 to 4, wherein R² is selected from:

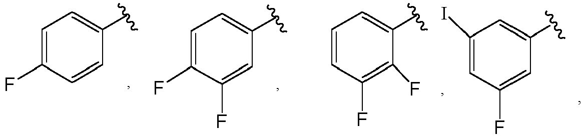

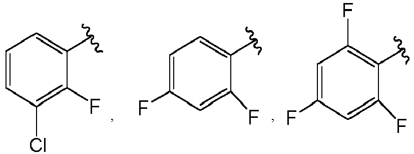

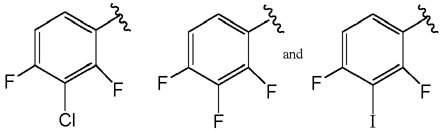

6. The compound of claim 5, wherein R² is:

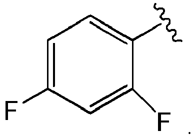

7. The compound of any of claims 1 to 6, wherein R³ is -N(R⁴)₂, wherein each occurrence of R⁴ is independently selected from H and C₁-C₆ alkyl.

8. The compound of claim 7, wherein R³ is -NHCH₂CH₃.

9. The compound of claim 1 or 2, wherein R¹ is selected from methyl, ethyl, n-propyl and -CH₂CH₂OCH₃; R² is selected from: 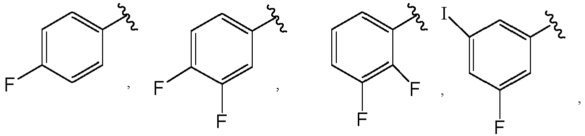 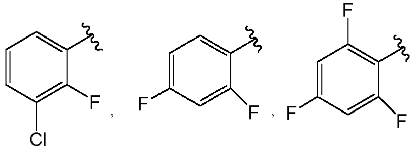 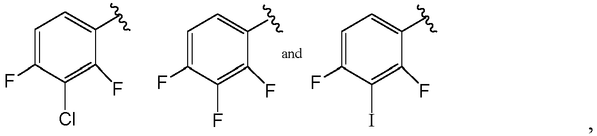 and
R³ is selected from -NH₂, -NHCH₃, -NHCH₂CH₃, -NHCH₂CH₂CH₃, -NHCH₂-cyclopropyl, -NH-benzyl, -NHCH₂CH₂OCH₃, -NHCH₂CH₂CF₃, -NHCD₂CD₃, pyrrolidinyl, -N(CH₃)₂, azetidinyl, -NHCH₂CH₂CH₂Cl, -NHS(O)₂CH₃, -NHS(O)₂-(p-toluene), -NHC(O)CH₃ and -NHC(O)C(O)N(CH₃)₂.

10. A compound of claim 1 selected from: 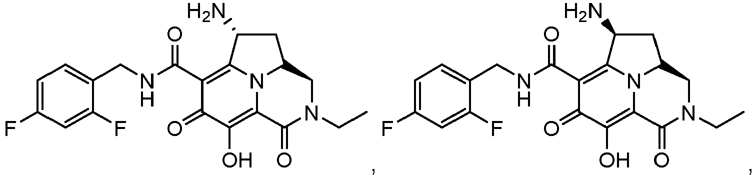 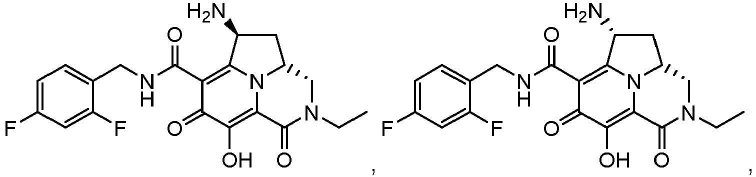 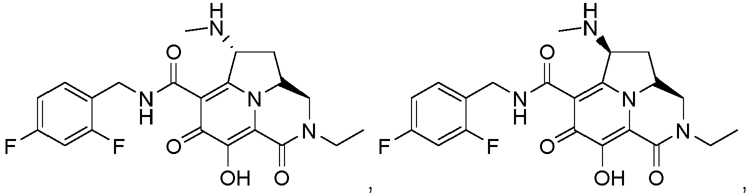 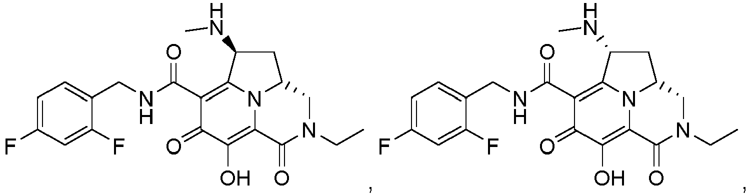 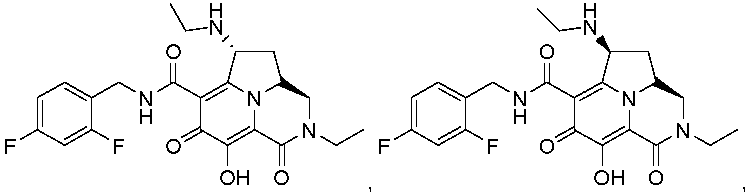 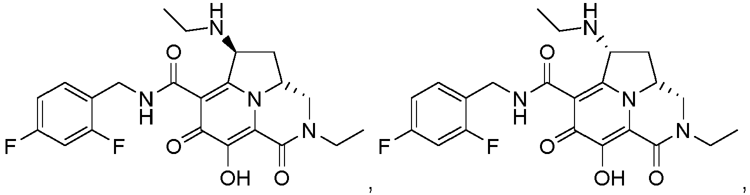 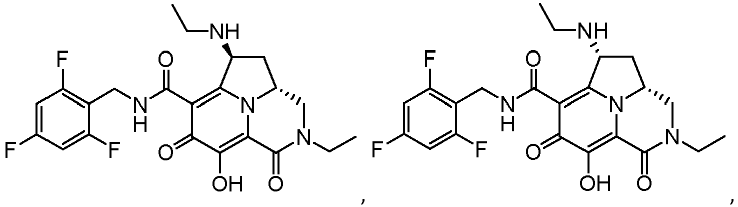 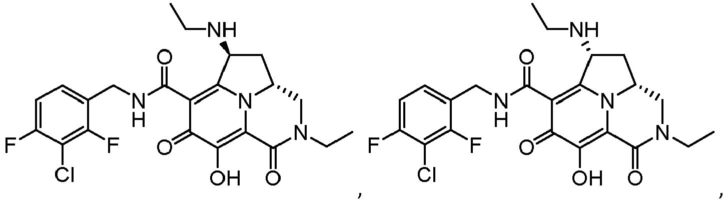 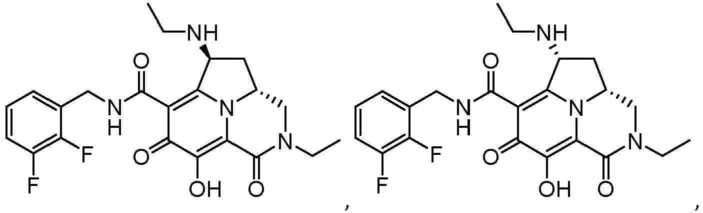 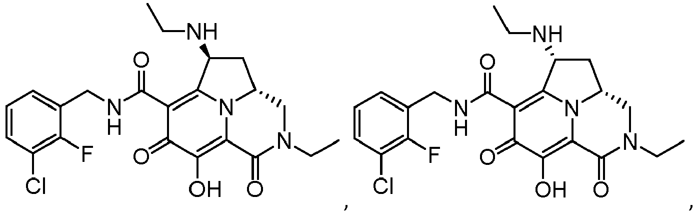 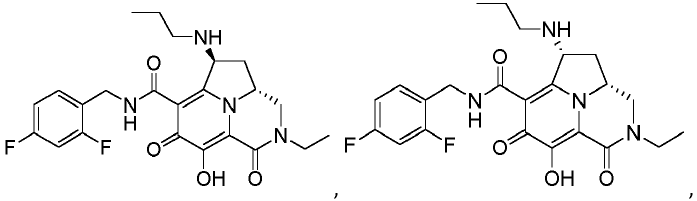 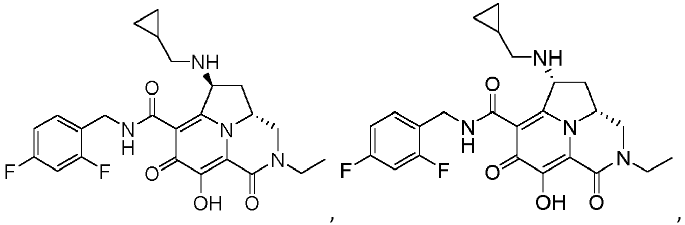 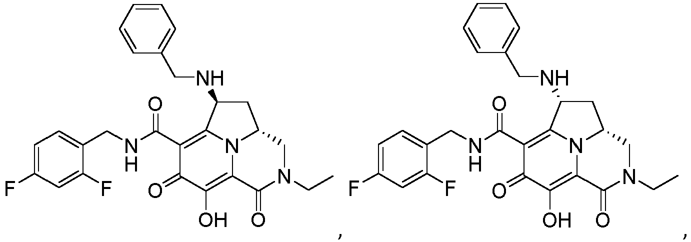 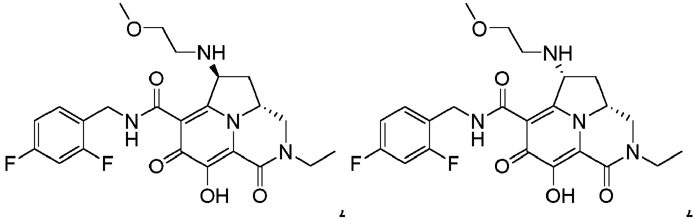 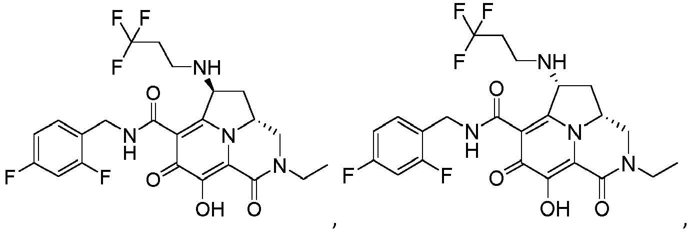 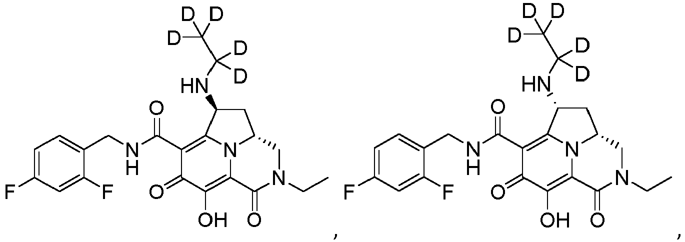 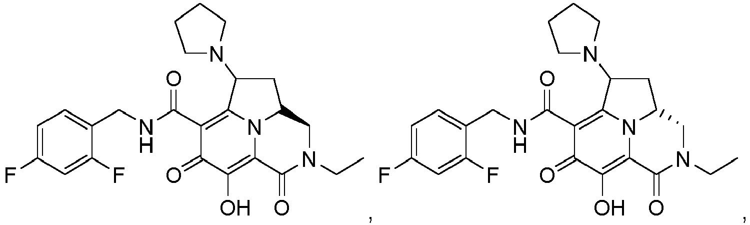 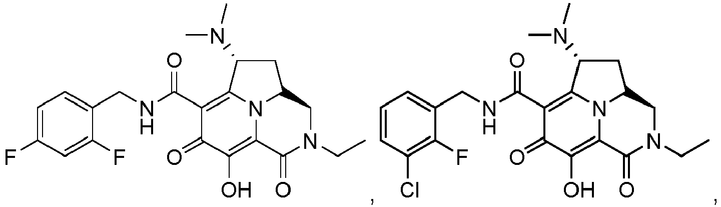 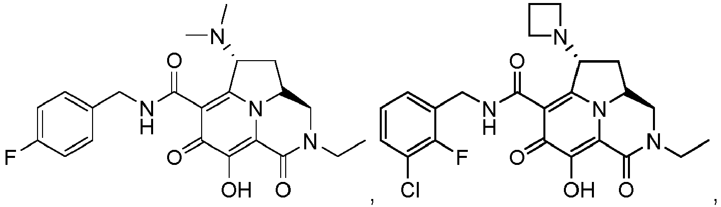 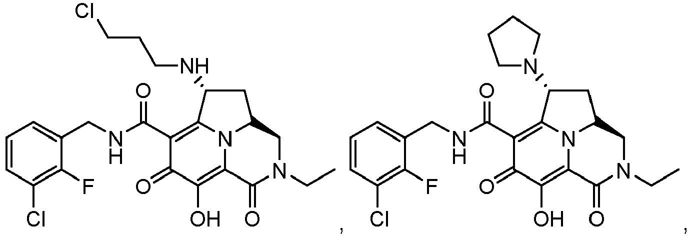 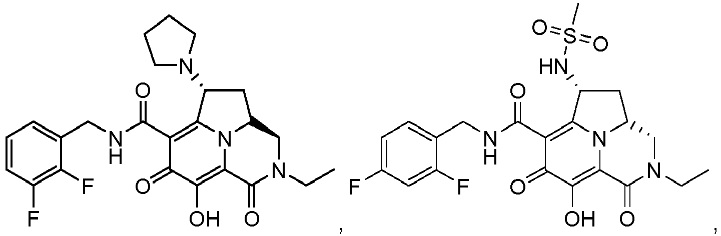 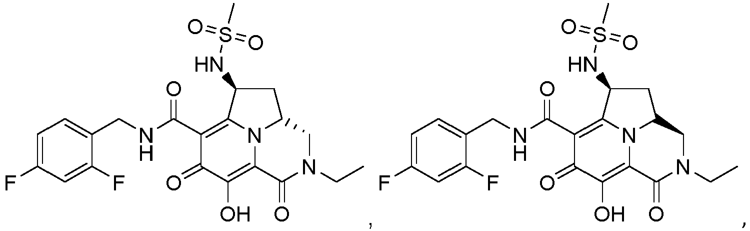 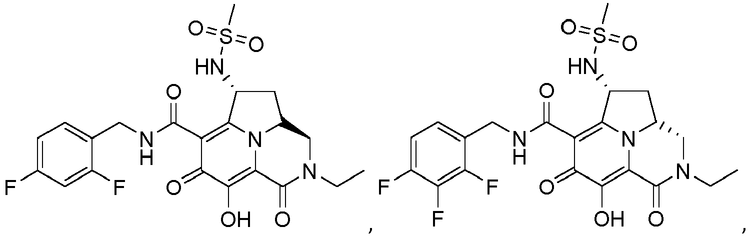 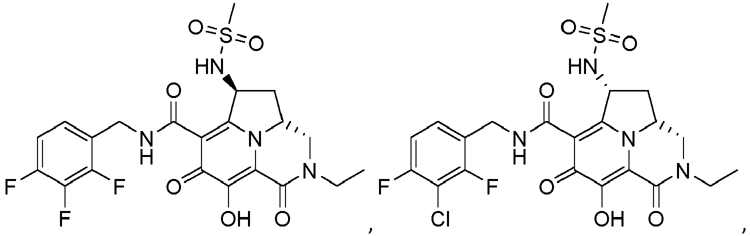 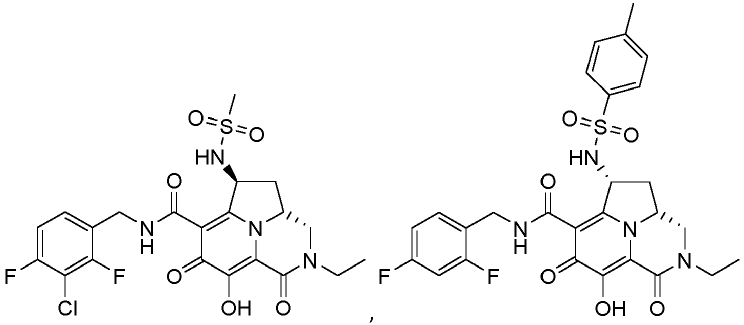 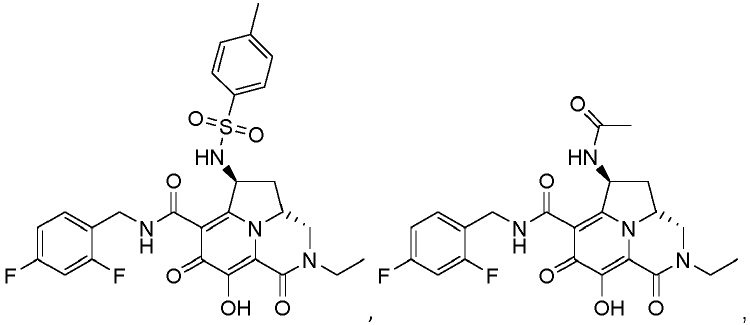 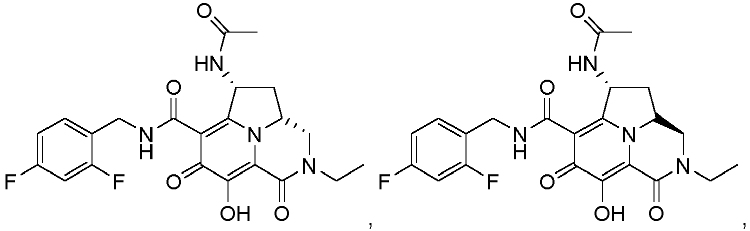 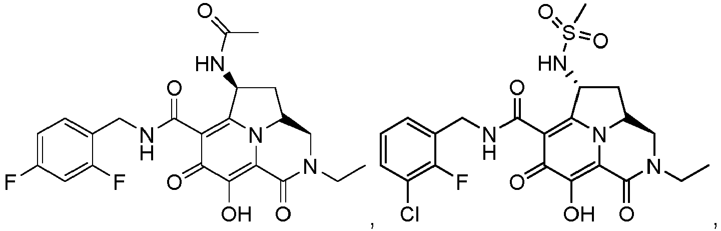 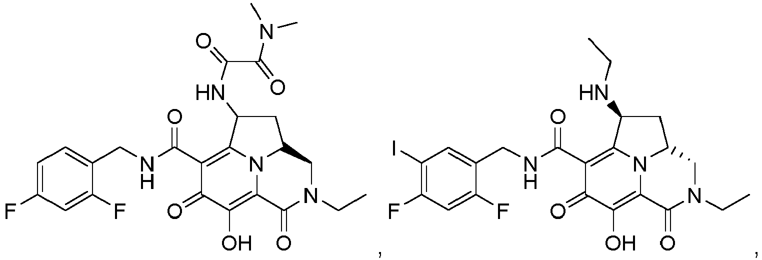 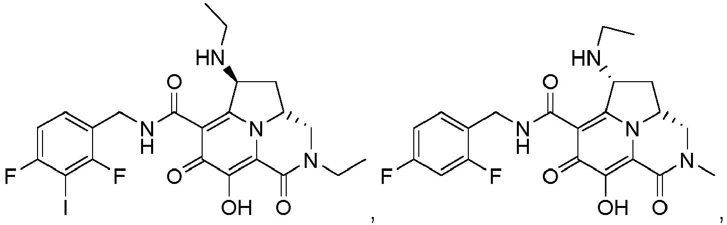 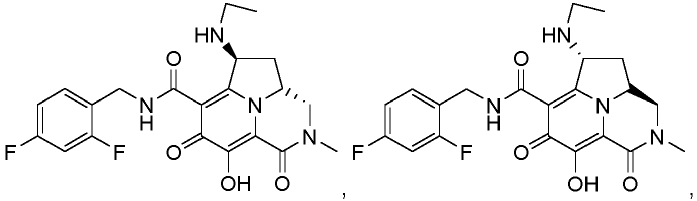 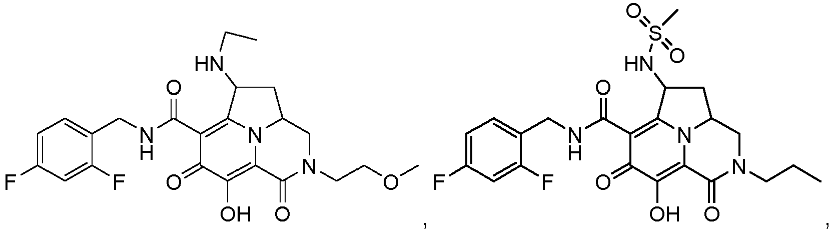 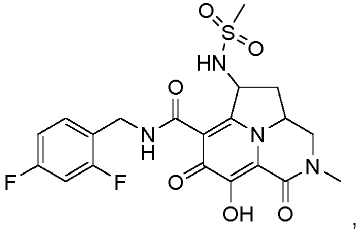 or a pharmaceutically acceptable salt or prodrug thereof.

11. A pharmaceutical composition comprising (i) an effective amount of a compound according to any one of claims 1 to 10, or a pharmaceutically acceptable salt thereof, and (ii) a pharmaceutically acceptable carrier.

12. A compound according to any one of claims 1 to 10, or a pharmaceutically acceptable salt thereof, for use in therapy.

13. A compound according to any one of claims 1 to 10, or a pharmaceutically acceptable salt thereof, for use in the inhibition of HIV integrase, for the treatment or prophylaxis of infection by HIV, or for the treatment, prophylaxis, or delay in the onset or progression of AIDS in a subject.

14. The pharmaceutical composition of claim 11, further comprising one or more additional therapeutic agents selected from raltegravir, lamivudine, abacavir, ritonavir, dolutegravir, arunavir, atazanavir, emtricitabine, tenofovir, elvitegravir, rilpivirine and lopinavir.

## Patentansprüche

1. Eine Verbindung der Formel: 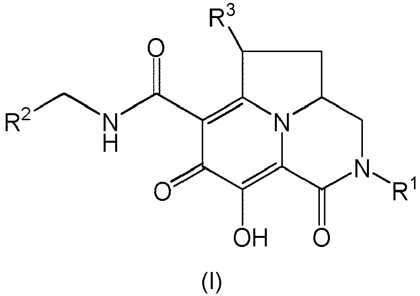 oder ein pharmazeutisch annehmbares Salz davon,
wobei:
R¹ C₁-C₆-Alkyl oder -(C₁-C₆-Alkenyl)-O-(C₁-C₆-alkyl) ist,
R² Phenyl ist, das gegebenenfalls substituiert ist mit bis zu 3 Ring-Substituentengruppen, jeweils unabhängig ausgewählt aus Halogen,
R³ -N(R⁴)₂ ist,
jedes Vorkommen von R⁴ unabhängig ausgewählt ist aus H, C₁-C₆-Alkyl, C₁-C₆-Halogenalkyl, C₃-C₇-Cycloalkyl, -CH₂-(C₃-C₇-Cycloalkyl), Phenyl, Benzyl, -C(O)-C(O)-N(R⁵)₂,-S(O)₂-C₁-C₆-Alkyl, -S(O)₂-Phenyl, -(C₁-C₆-Alkenyl)-O-(C₁-C₆-alkyl) und -(C(O)-C₁-C₆-Alkyl, wobei der Phenylrest einer -S(O)₂-Phenyl-Gruppe gegebenenfalls substituiert sein kann mit einer C₁-C₆-Alkylgruppe, oder beide R⁴-Gruppen und das gemeinsame Stickstoffatom, an das sie gebunden sind, verbunden sind unter Bildung einer Azetidinyl-, Piperidinyl- oder Pyrrolidinylgruppe, und
jedes Vorkommen von R⁵ unabhängig ausgewählt ist aus H und C₁-C₆-Alkyl.

2. Die Verbindung nach Anspruch 1 der Formel (la), (Ib), (Ic) und (Id):

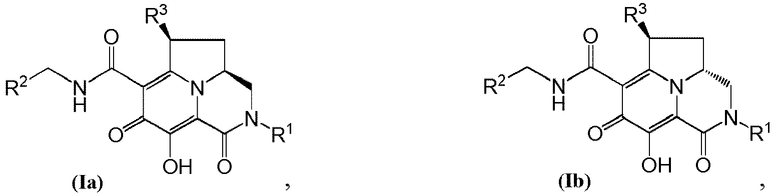

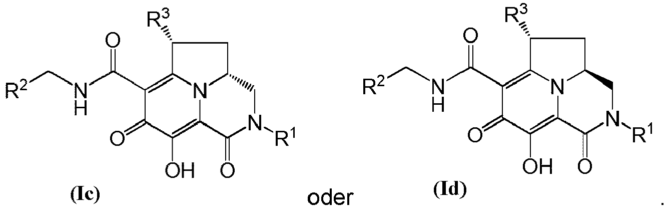

3. Die Verbindung nach Anspruch 1 oder 2, wobei R¹ C₁-C₆-Alkyl ist.

4. Die Verbindung nach Anspruch 3, wobei R¹ Ethyl ist.

5. Die Verbindung nach einem der Ansprüche 1 bis 4, wobei R² ausgewählt ist aus:

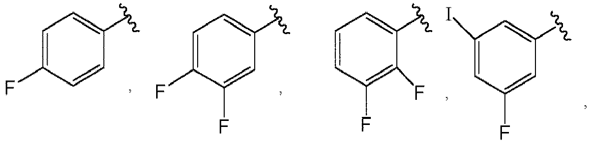

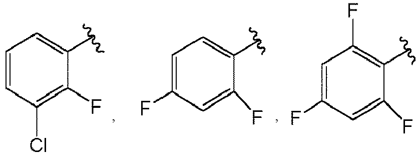

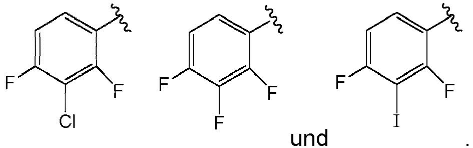

6. Die Verbindung nach Anspruch 5, wobei R² ist:

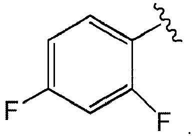

7. Die Verbindung nach einem der Ansprüche 1 bis 6, wobei R³ -N(R⁴)₂ ist, wobei jedes Vorkommen von R⁴ unabhängig ausgewählt ist aus H und C₁-C₆-Alkyl.

8. Die Verbindung nach Anspruch 7, wobei R³ -NHCH₂CH₃ ist.

9. Die Verbindung nach Anspruch 1 oder 2, wobei R¹ ausgewählt ist aus Methyl, Ethyl, n-Propyl und -CH₂CH₂OCH₃, R² ausgewählt ist aus: 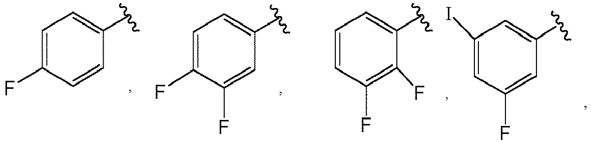 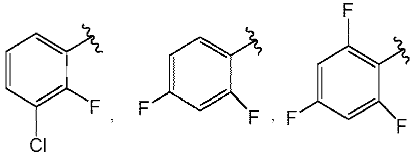 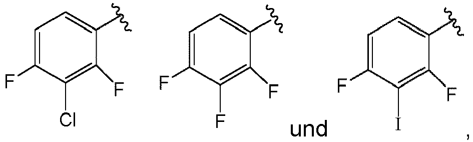 und
R³ ausgewählt ist aus -NH₂, -NHCH₃, -NHCH₂CH₃, -NHCH₂CH₂CH₃, -NHCH₂-Cyclopropyl, -NH-Benzyl, NHCH₂CH₂OCH₃, -NHCH₂CH₂CF₃, -NHCD₂CD₃, Pyrrolidinyl, -N(CH₃)₂, Azetidinyl, -NHCH₂CH₂CH₂Cl, -NHS(O)₂CH₃, -NHS(O)₂-(p-Toluol), -NHC(O)CH₃ und -NHC(O)C(O)N(CH₃)₂.

10. Eine Verbindung nach Anspruch 1, ausgewählt aus: 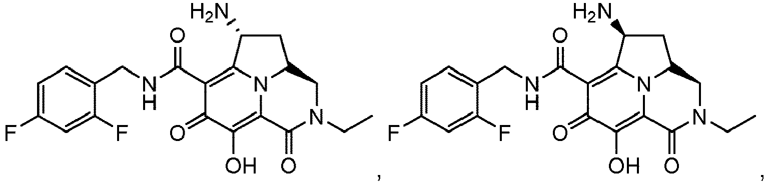 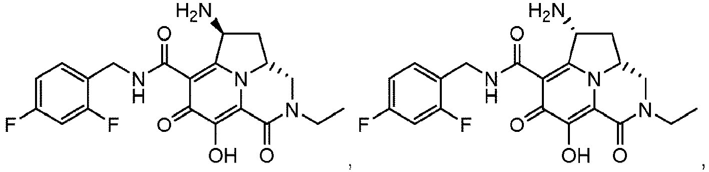 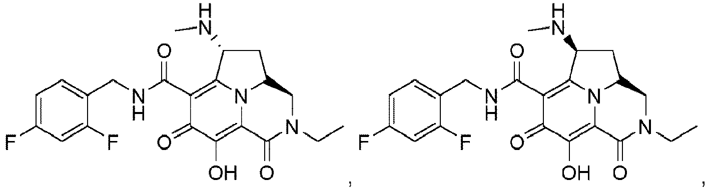 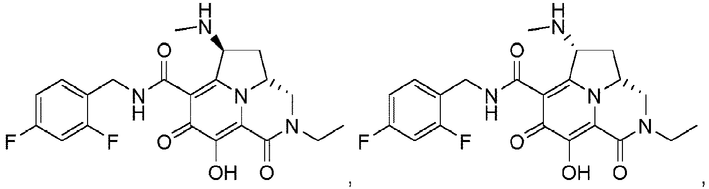 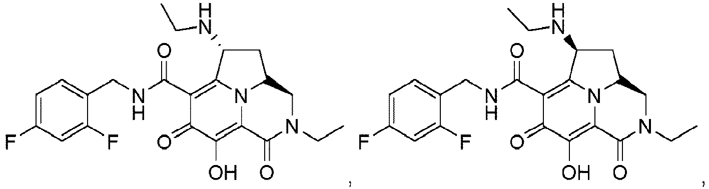 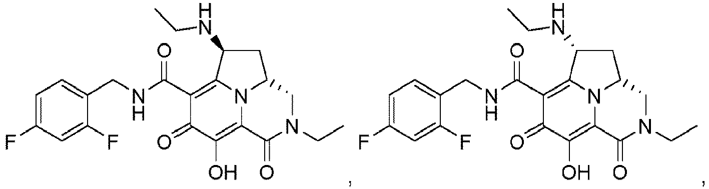 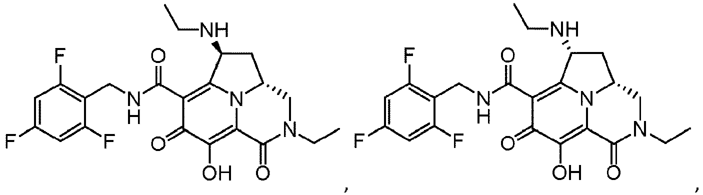 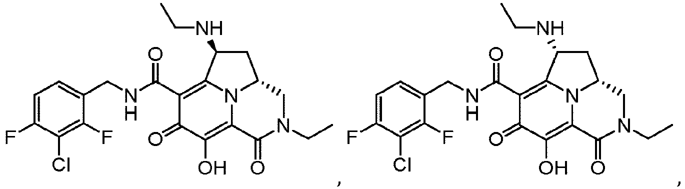 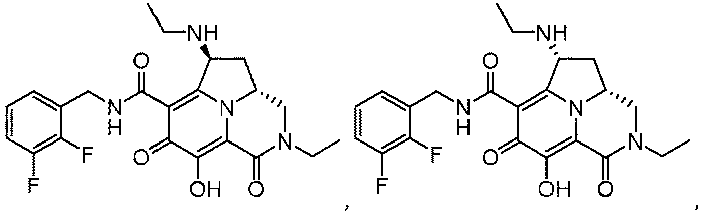 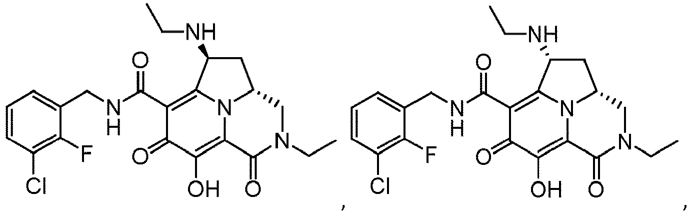 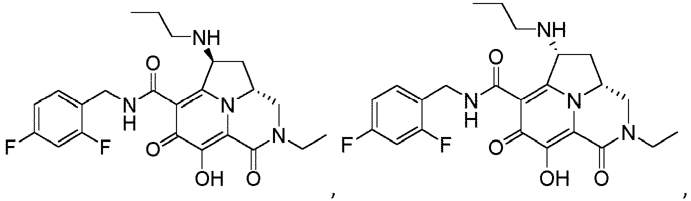 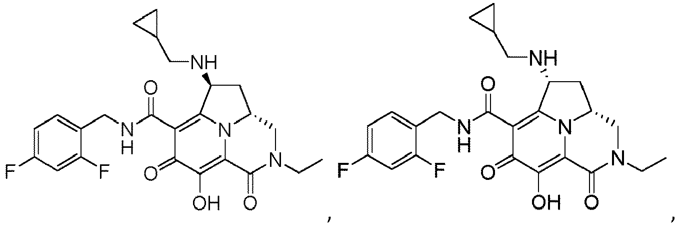 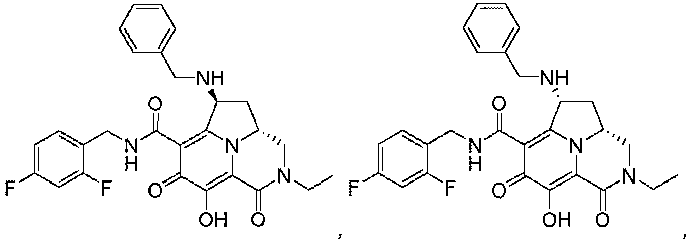 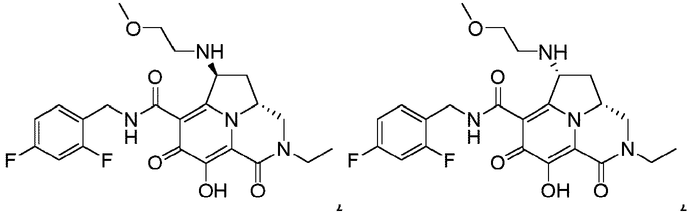 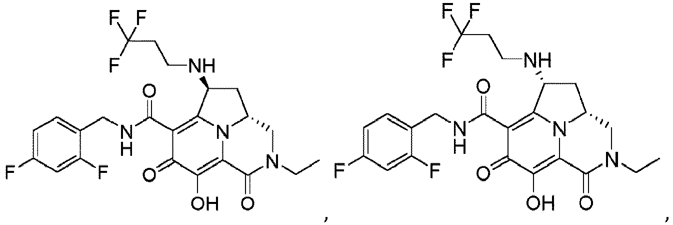 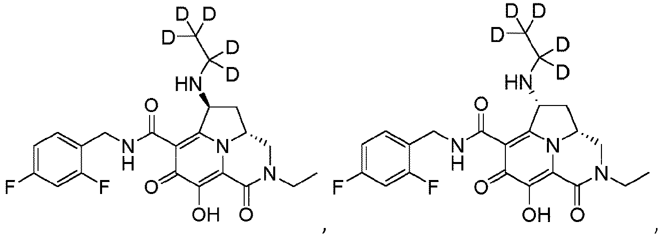 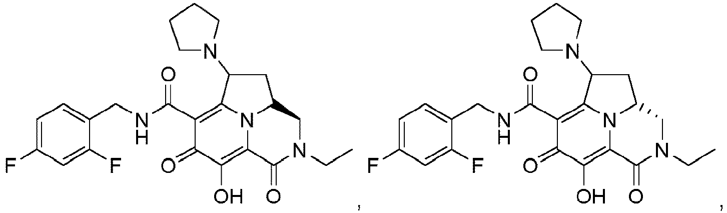 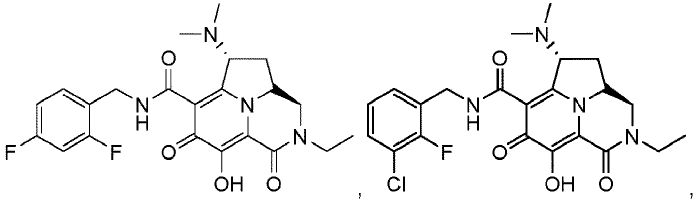 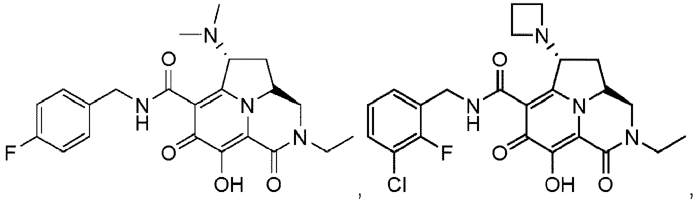 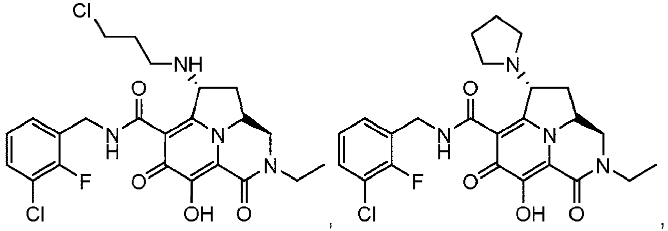 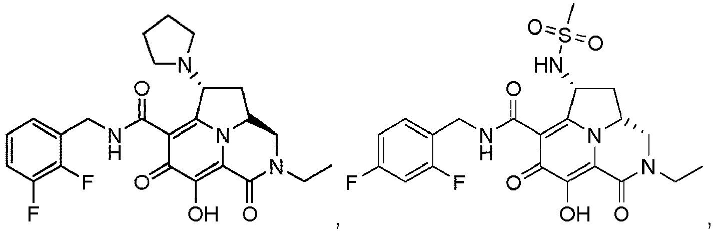 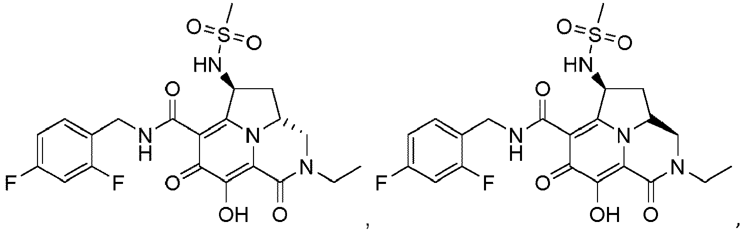 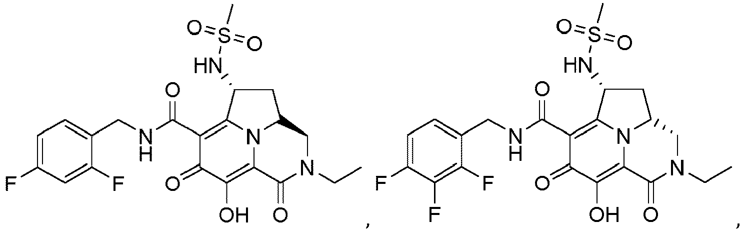 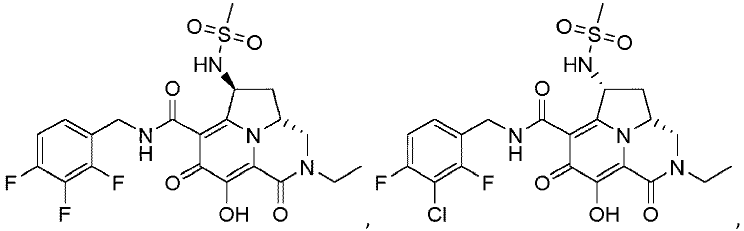 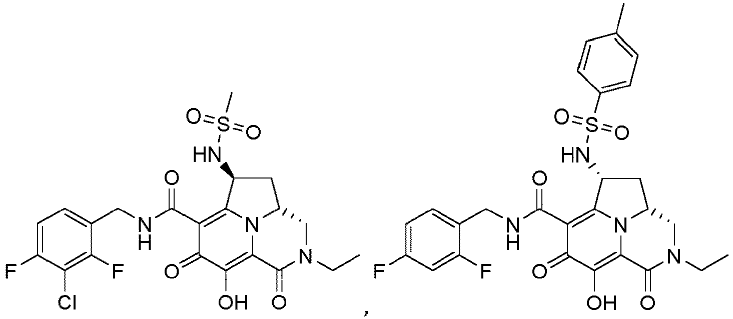 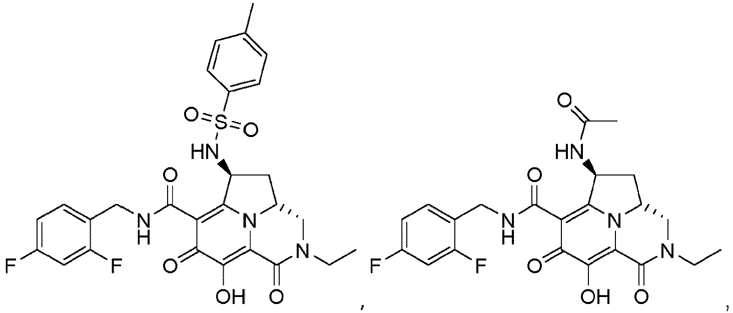 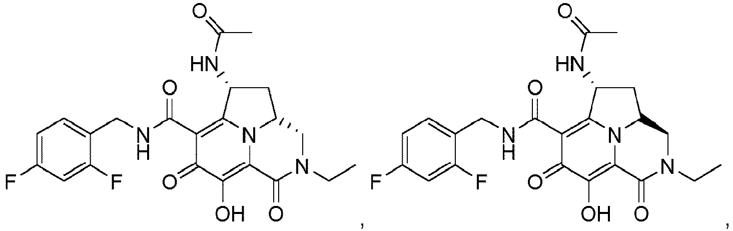 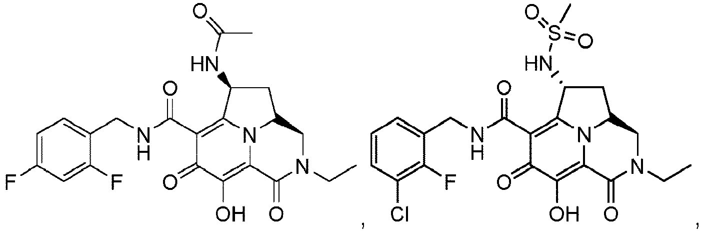 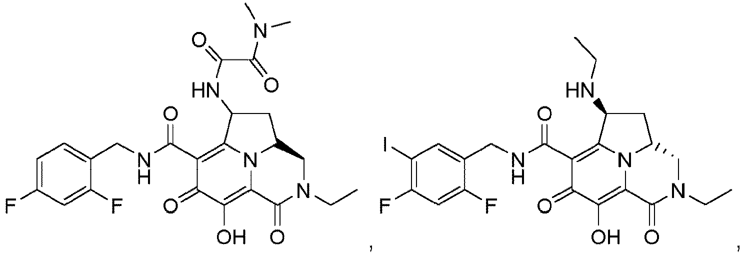 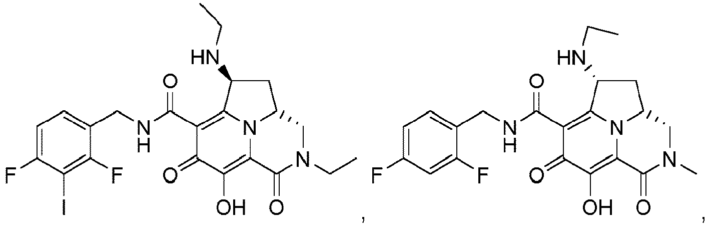 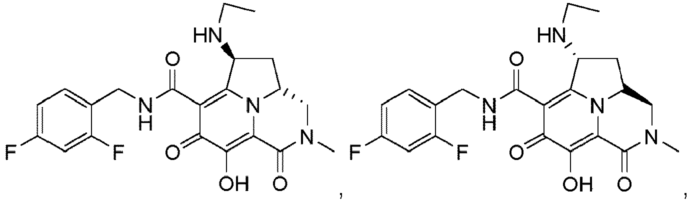 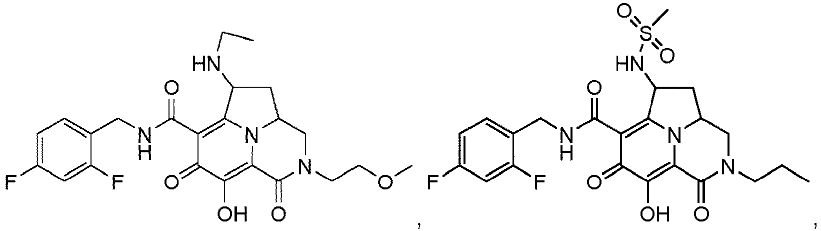 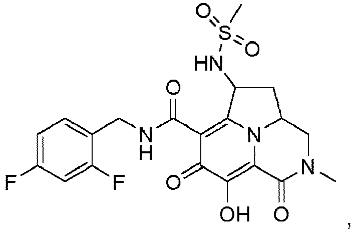 oder ein pharmazeutisch annehmbares Salz oder Prodrug davon.

11. Eine pharmazeutische Zusammensetzung, die (i) eine wirksame Menge einer Verbindung gemäß einem der Ansprüche 1 bis 10 oder eines pharmazeutisch annehmbaren Salzes davon und (ii) einen pharmazeutisch annehmbaren Träger umfasst.

12. Eine Verbindung gemäß einem der Ansprüche 1 bis 10 oder ein pharmazeutisch annehmbares Salz davon zur Verwendung in der Therapie.

13. Eine Verbindung gemäß einem der Ansprüche 1 bis 10 oder ein pharmazeutisch annehmbares Salz davon zur Verwendung bei der Inhibierung von HIV-Integrase, zur Behandlung oder Prophylaxe einer HIV-Infektion oder zur Behandlung, Prophylaxe oder Verzögerung des Ausbruchs oder Fortschreitens von AIDS bei einem Subjekt.

14. Die pharmazeutische Zusammensetzung nach Anspruch 11, die ferner ein oder mehrere zusätzliche therapeutische Mittel umfasst, ausgewählt aus Raltegravir, Lamivudin, Abacavir, Ritonavir, Dolutegravir, Arunavir, Atazanavir, Emtricitabin, Tenofovir, Elvitegravir, Rilpivirin und Lopinavir.

## Revendications

1. Composé présentant la Formule : 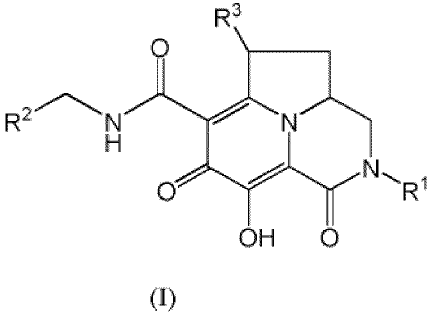 ou un sel pharmaceutiquement acceptable de celui-ci,
dans lequel :
R¹ est un C₁-C₆ alkyle ou un -(C₁-C₆ alcényle)-O-(C₁-C₆ alkyle) ;
R² est un phényle, qui est optionnellement substitué par jusqu'à 3 groupes substituants de cycle, chacun étant indépendamment choisi parmi un halo ;
R³ est -N(R⁴)₂ ;
chaque occurrence de R⁴ est indépendamment choisie parmi : H, C₁-C₆ alkyle, C₁-C₆ haloalkyle, C₃-C₇ cycloalkyle, -CH₂-(C₃-C₇ cycloalkyle), phényle, benzyle, -C(O)-C(O)-N(R⁵)₂,-S(O)₂-C₁-C₆ alkyle, -S(O)₂-phényle, -(C₁-C₆ alcényle)-O-(C₁-C₆ alkyle) et -C(O)-C₁-C₆ alkyle, dans lequel la fraction phényle d'un groupe -S(O)₂-phényle peut être optionnellement substituée par un groupe C₁-C₆ alkyle ; ou les deux groupes R⁴, et l'atome d'azote commun auquel ils sont attachés, se joignent pour former un groupe azétidinyle, pipéridinyle ou pyrrolidinyle ; et
chaque occurrence de R⁵ est indépendamment choisie parmi : H et C₁-C₆ alkyle.

2. Composé selon la revendication 1, présentant la formule (la), (Ib), (Ic) et (Id) :

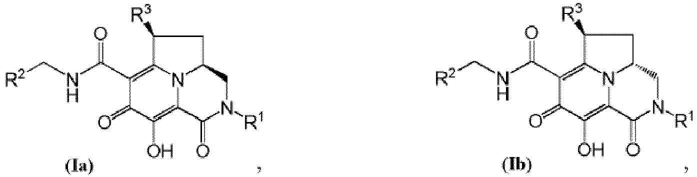

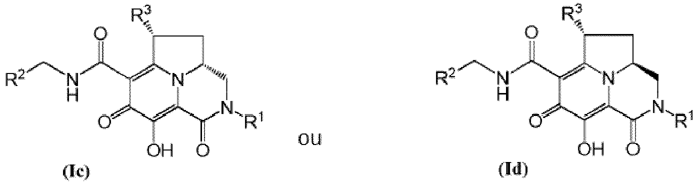

3. Composé selon la revendication 1 ou 2, dans lequel R¹ est un C₁-C₆ alkyle.

4. Composé selon la revendication 3, dans lequel R¹ est un éthyle.

5. Composé selon l'une quelconque des revendications 1 à 4, dans lequel R² est choisi parmi :

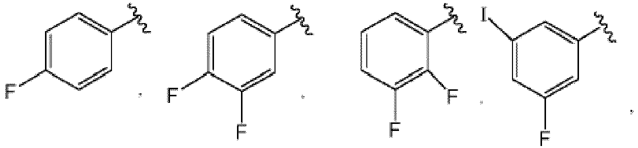

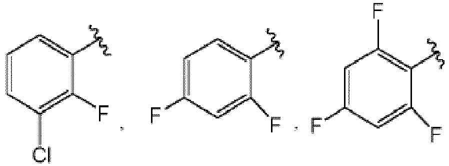

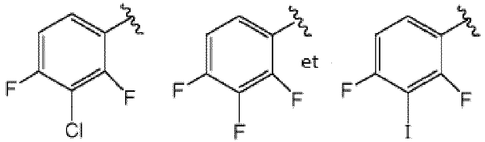

6. Composé selon la revendication 5, dans lequel R² est :

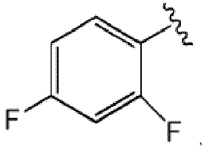

7. Composé selon l'une quelconque des revendications 1 à 6, dans lequel R³ est -N(R⁴)₂, dans lequel chaque occurrence de R⁴ est indépendamment choisie parmi : H et C₁-C₆ alkyle.

8. Composé selon la revendication 7, dans lequel R³ est -NHCH₂CH₃.

9. Composé selon la revendication 1 ou 2, dans lequel R¹ est choisi parmi : méthyle, éthyle, n-propyle et -CH₂CH₂OCH₃ ; R² est choisi parmi : 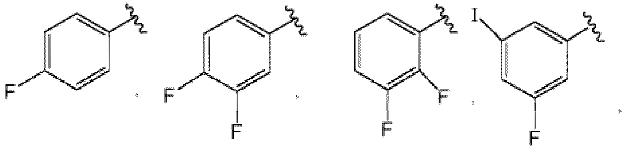 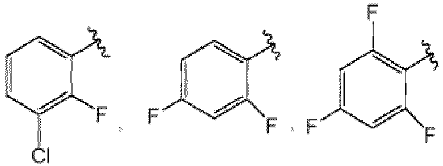 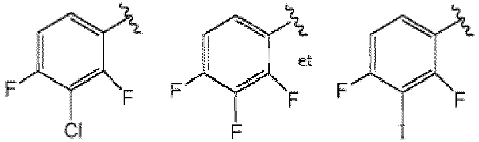 , et
R³ est choisi parmi : -NH₂, -NHCH₃, -NHCH₂CH₃, -NHCH₂CH₂CH₃, -NHCH₂-cyclopropyle, -NH-benzyle, -NHCH₂CH₂OCH₃, -NHCH₂CH₂CF₃, -NHCD₂CD₃, pyrrolidinyle,-N(CH₃)₂, azétidinyle, -NHCH₂CH₂CH₂Cl, -NHS(O)₂CH₃, -NHS(O)₂-(p-toluène), -NHC(O)CH₃ et -NHC(O)C(O)N(CH₃)₂.

10. Composé selon la revendication 1 choisi parmi : 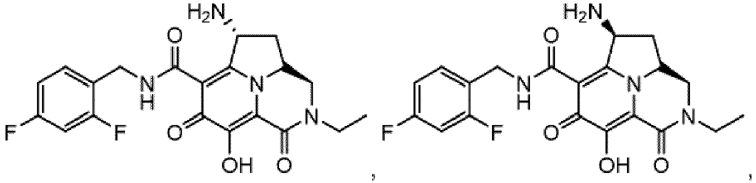 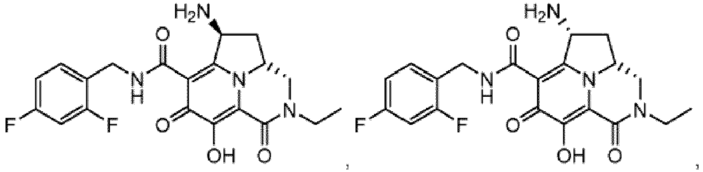 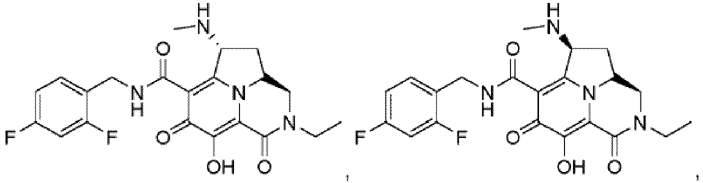 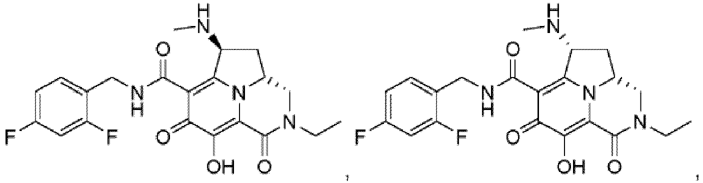 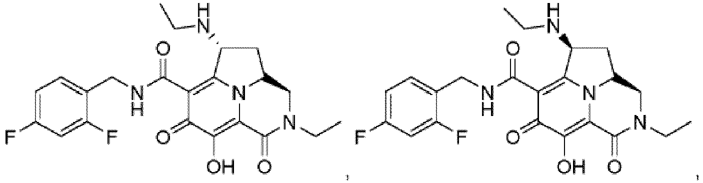 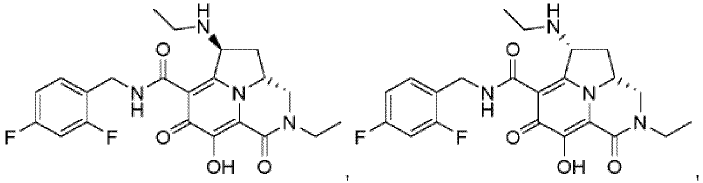 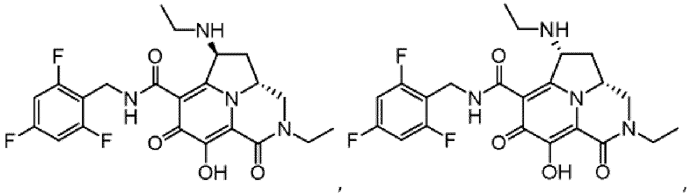 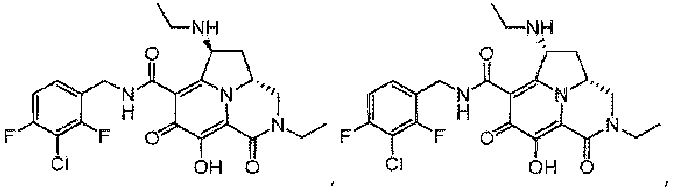 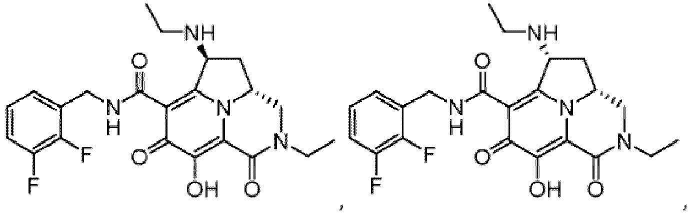 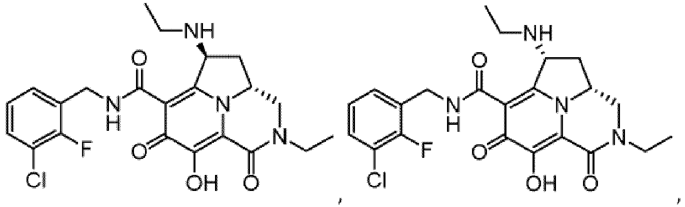 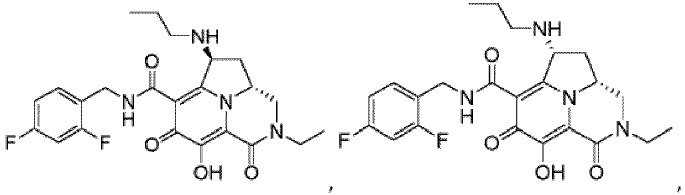 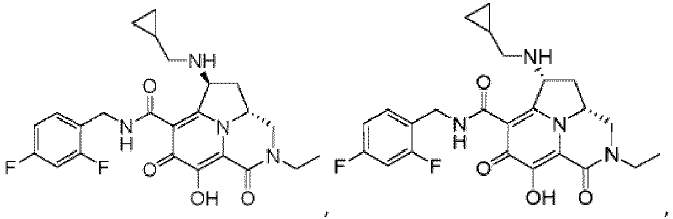 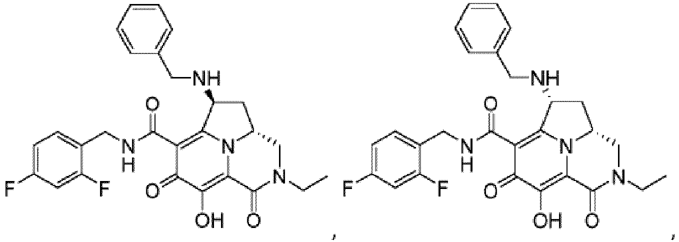 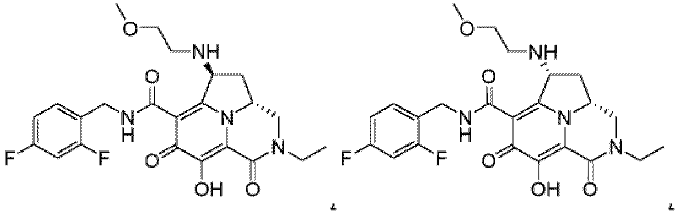 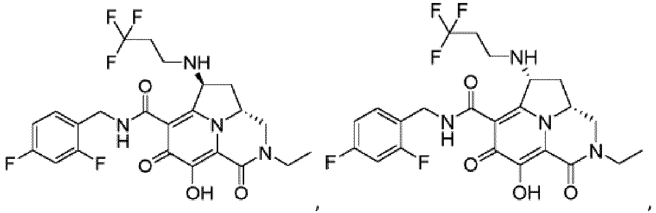 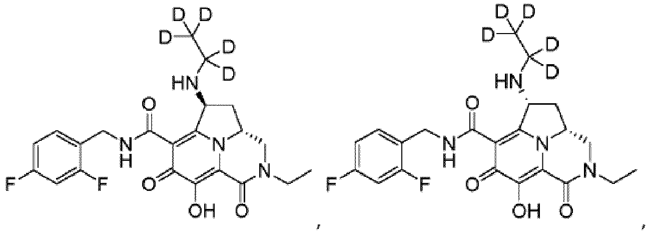 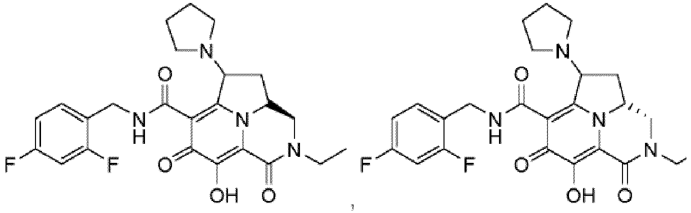 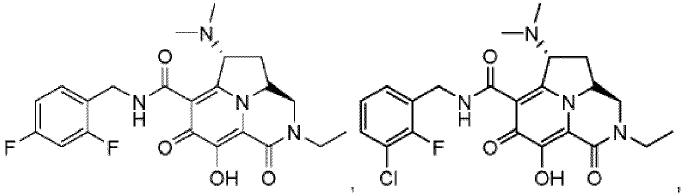 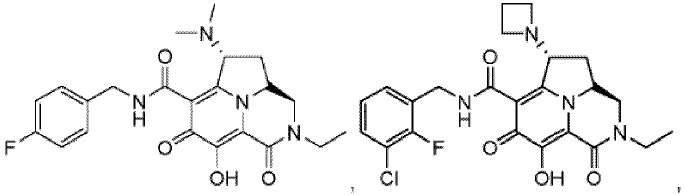 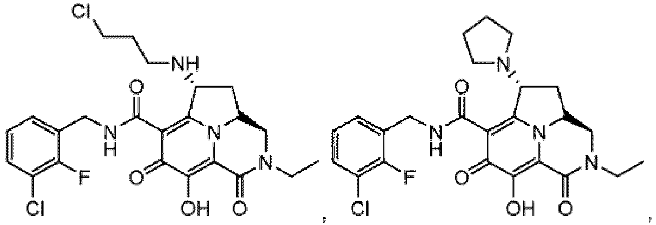 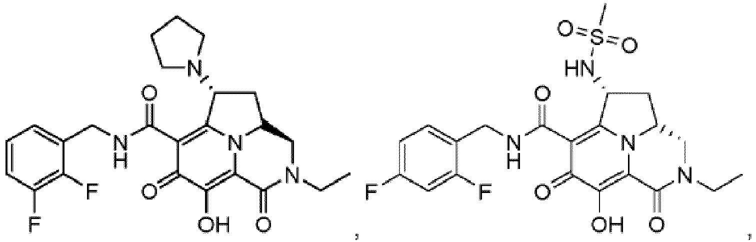 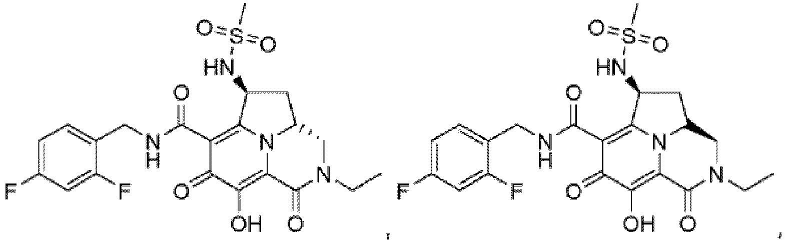 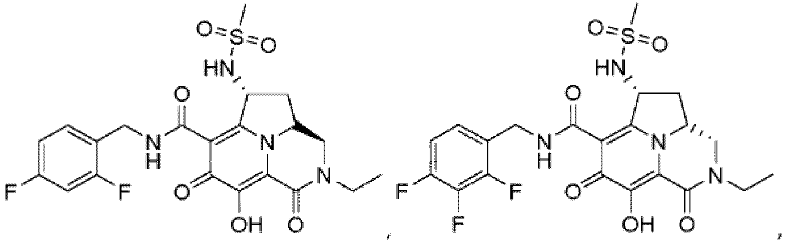 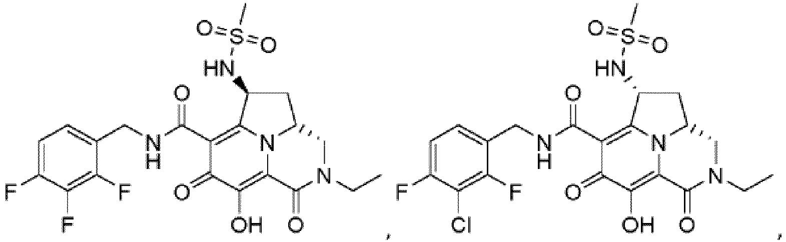 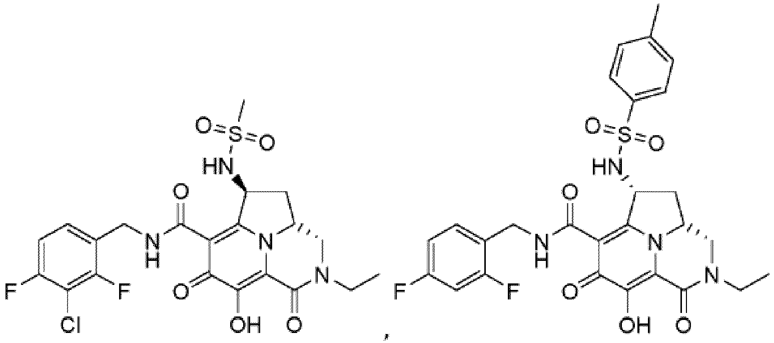 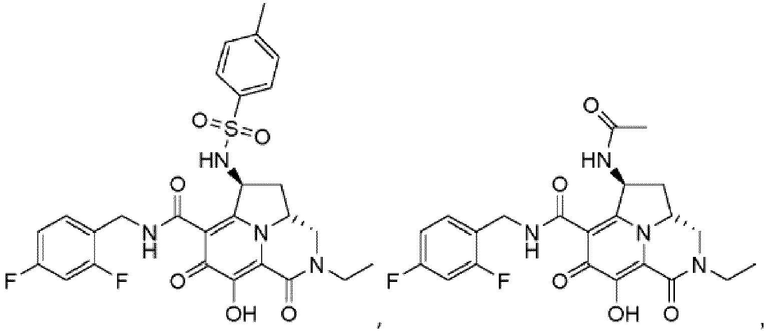 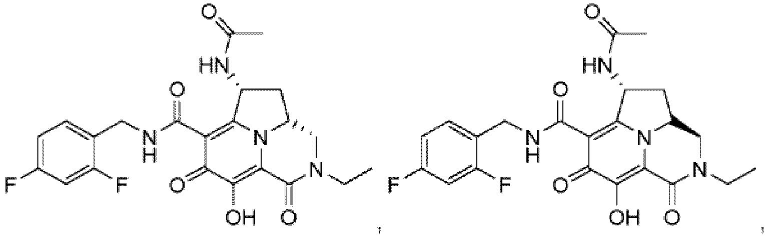 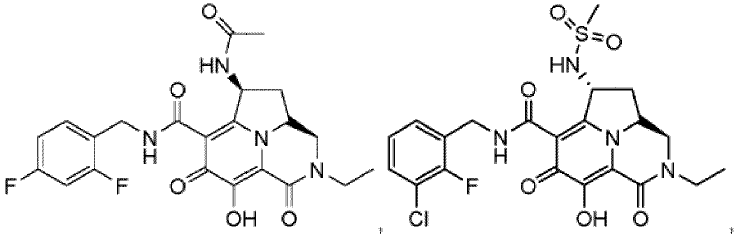 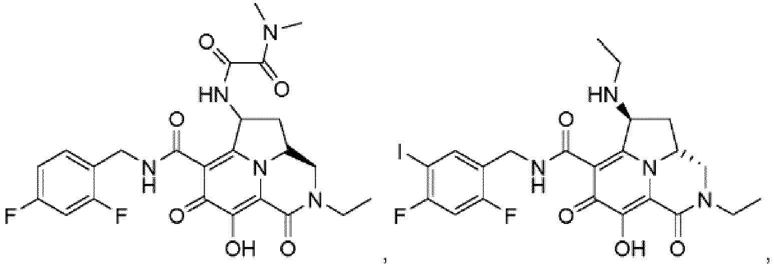 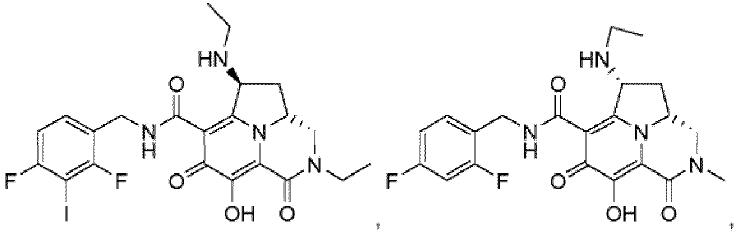 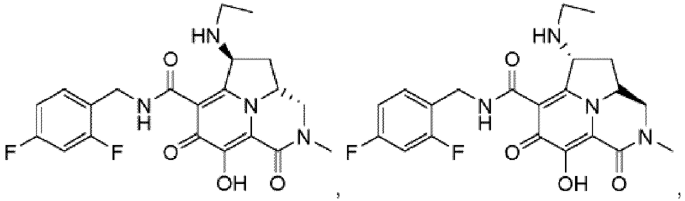 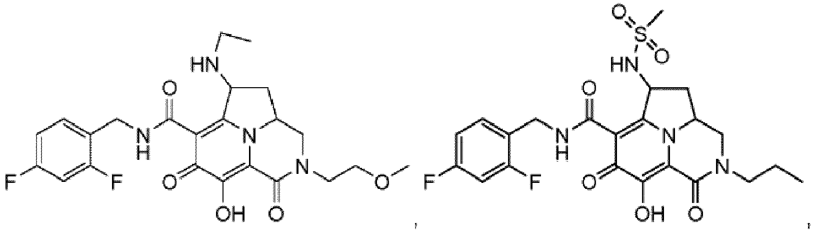 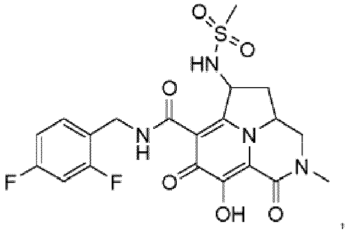 ou un sel pharmaceutiquement acceptable ou un promédicament de ceux-ci.

11. Composition pharmaceutique comprenant (i) une quantité efficace d'un composé selon l'une quelconque des revendications 1 à 10, ou d'un sel pharmaceutiquement acceptable de celui-ci, et (ii) un véhicule pharmaceutiquement acceptable.

12. Composé selon l'une quelconque des revendications 1 à 10, ou un sel pharmaceutiquement acceptable de celui-ci, pour utilisation en thérapie.

13. Composé selon l'une quelconque des revendications 1 à 10, ou un sel pharmaceutiquement acceptable de celui-ci, pour utilisation dans l'inhibition de l'intégrase du VIH, pour le traitement ou la prophylaxie d'une infection par le VIH ou pour le traitement, la prophylaxie ou le retardement dans l'apparition ou la progression du SIDA chez un sujet.

14. Composition pharmaceutique selon la revendication 11, comprenant en outre un ou plusieurs agents thérapeutiques supplémentaires choisis parmi : raltégravir, lamivudine, abacavir, ritonavir, dolutégravir, arunavir, atazanavir, emtricitabine, ténofovir, elvitégravir, rilpivirine et lopinavir.
